(19) Europäisches Patentamt
European Patent Office
Office européen des brevets

(11) EP 4 610 651 A2

(12) EUROPEAN PATENT APPLICATION

(43) Date of publication:
**03.09.2025 Bulletin 2025/36**

(21) Application number: **25189825.0**

(22) Date of filing: **30.08.2021**

(51) International Patent Classification (IPC):
***G01N 33/48** (2006.01)*

(52) Cooperative Patent Classification (CPC):
**G01N 15/1459; G01N 15/1429;** G01N 2015/1006; G01N 2015/1488; G16H 10/40

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.09.2020 JP 2020157930**

(62) Document number(s) of the earlier application(s) in accordance with Art. 76 EPC:
**21869154.1 / 4 215 901**

(71) Applicant: **SYSMEX CORPORATION**
**Kobe-shi**
**Hyogo 651-0073 (JP)**

(72) Inventors:
- **Asada, Shoichiro**
  **Kobe-shi, Hyogo, 651-0073 (JP)**
- **Kimura, Konobu**
  **Kobe-shi, Hyogo, 651-0073 (JP)**
- **Tanaka, Masamichi**
  **Kobe-shi, Hyogo, 651-0073 (JP)**
- **Suzuki, Kenichiro**
  **Kobe-shi, Hyogo, 651-0073 (JP)**
- **Nango, Kohei**
  **Kobe-shi, Hyogo, 651-0073 (JP)**

(74) Representative: **Hoffmann Eitle**
**Patent- und Rechtsanwälte PartmbB**
**Arabellastraße 30**
**81925 München (DE)**

Remarks:
This application was filed on 16.07.2025 as a divisional application to the application mentioned under INID code 62.

# (54) CELL ANALYSIS METHOD AND CELL ANALYZER

(57) Provided is a cell analysis method and a cell analyzer that, in a configuration in which data obtained from a plurality of cells contained in a specimen is analyzed, can process, at a requested throughput, data of cells having a significantly increased information amount. The cell analysis method includes: in a cell analyzer including a host processor and a parallel-processing processor, obtaining, on the basis of control by the host processor, data regarding each of a plurality of cells in a specimen; executing, by the parallel-processing processor, parallel processing regarding the data; and generating, on the basis of a result of the parallel processing, information regarding a cell type with respect to each of the plurality of cells.

FIG. 1

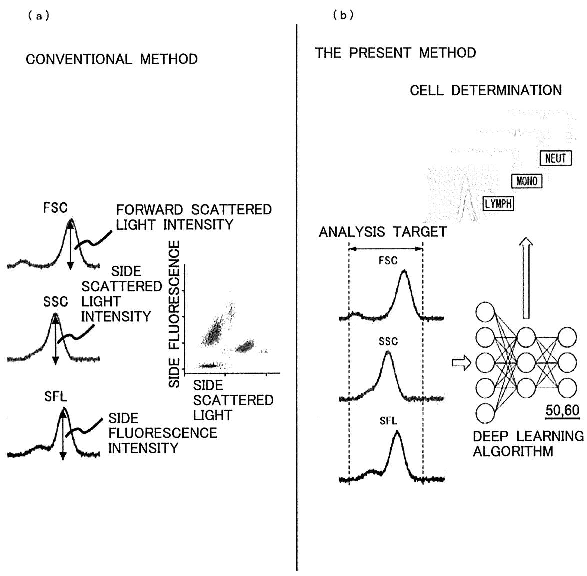


EP 4 610 651 A2

## Description

### TECHNICAL FIELD

The present invention relates to a cell analysis method and a cell analyzer.

### BACKGROUND ART

PATENT LITERATURE 1 describes a method in which: data obtained by measuring blood cells by a flow cytometer is analyzed in a data processing system having installed therein a processor; and the cells are classified according to the types. PATENT LITERATURE 1 indicates that information of the volume and the electric conductivity of the cells is further used when the cells cannot be classified according to optical information measured by the flow cytometer.

### CITATION LIST

### [PATENT LITERATURE]

[PTL 1] Japanese Laid-open Patent Publication No. 2012-519848 (translation of PCT International Application)

### SUMMARY OF THE INVENTION

### PROBLEMS TO BE SOLVED BY THE INVENTION

If the amount of information to be used for classification of cells is increased in order to improve the classification accuracy of cells, in a case of a specimen such as blood or urine containing a plurality of cells, the data volume per specimen becomes huge due to increase of the amount of information obtained from a single cell. For example, in order to classify individual cells by using a deep learning algorithm, the amount of information obtained from individual cells needs to be significantly increased in order to extract features of each cell. PATENT LITERATURE 1 does not disclose a system in which a significantly increased amount of information can be processed in the range of a requested throughput.

An aspect of the present invention is to provide a cell analysis method and a cell analyzer that, in a configuration in which data obtained from a plurality of cells contained in a specimen is analyzed, can satisfy a requested throughput with respect to data of cells having a significantly increased amount of information.

### SOLUTION TO THE PROBLEMS

In order to solve the above problem, a cell analysis method according to an aspect of the present invention using a cell analyzer (4000, 4000', 4000") that comprises a host processor (3001, 4831, 6001, 8111) and a parallel-processing processor (3002, 4833, 6002, 8112) includes: obtaining, on the basis of control by the host processor (3001, 4831, 6001, 8111), data regarding each of a plurality of cells in a specimen; executing, by the parallel-processing processor (3002, 4833, 6002, 8112), parallel processing regarding the data; and generating, on the basis of a result of the parallel processing, information regarding a cell type with respect to each of the plurality of cells.

In order to solve the above problem, a cell analysis method according to another aspect of the present invention includes: performing measurement of a cell by a measurement unit (400, 400a, 500, 500a, 700); executing, by a parallel-processing processor (3002, 4833, 6002, 8112) connected to the measurement unit (400, 400a, 500, 500a, 700) not via an Internet or an intranet, parallel processing of data regarding the cell based on the measurement; and generating, on the basis of a result of the parallel processing, information regarding a cell type of the cell.

In order to solve the above problem, a cell analysis method according to another aspect of the present invention includes: suctioning a specimen by a measurement unit (400, 400a, 500, 500a, 700) installed in a cell analyzer (4000, 4000', 4000"); generating data regarding a cell in the specimen having been suctioned; executing, by a parallel-processing processor (3002, 4833, 6002, 8112) installed in the cell analyzer (4000, 4000', 4000"), parallel processing regarding the data; and generating, on the basis of a result of the parallel processing executed by the parallel-processing processor (3002, 4833, 6002, 8112), information regarding a cell type of the cell.

In order to solve the above problem, a cell analyzer according to an aspect of the present invention includes: a measurement unit (400, 400a, 500, 500a, 700) configured to measure a plurality of cells contained in a specimen; a processor (3001, 4831, 6001, 8111) configured to perform information processing regarding analysis of the plurality of cells; and a parallel-processing processor (3002, 4833, 6002, 8112), wherein the measurement unit (400, 400a, 500, 500a, 700) obtains data regarding each of the plurality of cells, the parallel-processing processor (3002, 4833, 6002, 8112) executes parallel processing regarding the data, and the processor (3001, 4831, 6001, 8111) processes information, regarding a cell type of each of the plurality of cells, which has been generated on the basis of a result of the parallel processing.

ADVANTAGEOUS EFFECTS OF THE INVENTION

While a plurality of cells are analyzed on the basis of data having a large volume, a request for a throughput can be satisfied.

BRIEF DESCRIPTION OF THE DRAWINGS

[FIG. 1] FIG. 1(a) shows an example of white blood cell classification according to a conventional method. FIG. 1(b) shows an example of white blood cell classification according to the present method.

[FIG. 2] FIG. 2(a) shows an example of applying light to a cell flowing in a flow cell. FIG. 2(b) shows an example of performing sampling of a forward scattered light signal, a side scattered light signal, and a fluorescence signal. FIG. 2(c) shows an example of waveform data obtained by sampling.

[FIG. 3] FIG. 3 shows an example of a generation method for training data.

[FIG. 4] FIG. 4 shows an example of a label value.

[FIG. 5] FIG. 5 shows an example of an analysis method for analysis data.

[FIG. 6] FIG. 6 shows an example of the appearance of a cell analyzer.

[FIG. 7] FIG. 7 shows an example of a block diagram of a measurement unit.

[FIG. 8] FIG. 8 shows an example of a specimen suction part and a sample preparation part.

[FIG. 9] FIG. 9 shows a configuration example of an optical system of an FCM detector.

[FIG. 10] FIG. 10 shows a configuration example of a processing unit.

[FIG. 11] FIG. 11 shows a configuration example of a parallel-processing processor.

[FIG. 12] FIG. 12 shows an implementation example of the parallel-processing processor to the measurement unit.

[FIG. 13] FIG. 13 shows another implementation example of the parallel-processing processor to the measurement unit.

[FIG. 14] FIG. 14 shows another implementation example of the parallel-processing processor to the measurement unit.

[FIG. 15] FIG. 15 shows another implementation example of the parallel-processing processor to the measurement unit.

[FIG. 16] FIG. 16 shows an outline of operation in which a processor executes arithmetic processes of matrix data by using the parallel-processing processor.

[FIG. 17] FIG. 17(a) shows a calculation formula of the product of a matrix.

FIG. 17(b) shows an example of arithmetic processes executed in parallel in the parallel-processing processor.

[FIG. 18] FIG. 18 shows how arithmetic processes are executed in the parallel-processing processor.

[FIG. 19] FIG. 19(a) shows an example of waveform data of forward scattered light as waveform data inputted to a deep learning algorithm. FIG. 19(b) shows an outline of a matrix operation between waveform data and a filter.

[FIG. 20] FIG. 20 shows an example of a specimen analysis operation performed by the cell analyzer.

[FIG. 21] FIG. 21 shows an example of a cell analysis process.

[FIG. 22] FIG. 22 shows an example of parallel processing.

[FIG. 23] FIG. 23 shows another example of a block diagram of a measurement unit.

[FIG. 24] FIG. 24 shows an example of a block diagram of a processing unit.

[FIG. 25] FIG. 25 shows an outline of operation in which a processor executes arithmetic processes of matrix data by using a parallel-processing processor.

[FIG. 26] FIG. 26 shows another example of a block diagram of a measurement unit.

[FIG. 27] FIG. 27 shows another example of a block diagram of a processing unit.

[FIG. 28] FIG. 28 shows a configuration example of the measurement unit, the processing unit, and an analysis unit.

[FIG. 29] FIG. 29 shows another example of a block diagram of a measurement unit.

[FIG. 30] FIG. 30 shows an example of a block diagram of the analysis unit.

[FIG. 31] FIG. 31 shows an outline of operation in which a processor executes arithmetic processes of matrix data by using a parallel-processing processor.

[FIG. 32] FIG. 32 shows another example of a block diagram of a processing unit.

[FIG. 33] FIG. 33 shows another example of a block diagram of a measurement unit.

[FIG. 34] FIG. 34 shows another example of a block diagram of an analysis unit.

[FIG. 35] FIG. 35 shows an example of a block diagram of a measurement unit.

[FIG. 36] FIG. 36 shows a schematic example of an optical system of a flow cytometer.

[FIG. 37] FIG. 37 shows a schematic example of a sample preparation part of a measurement unit.

[FIG. 38] FIG. 38 shows a schematic example of a waveform data analysis system.

[FIG. 39] FIG. 39 shows an example of a block diagram of a vendor-side apparatus.

[FIG. 40] FIG. 40 shows an example of a block diagram of a measurement unit.

[FIG. 41] FIG. 41 shows another example of a block diagram of an analysis unit.

[FIG. 42] FIG. 42 shows an example of a function block diagram of a deep learning apparatus.

[FIG. 43] FIG. 43 shows an example of a flowchart of operation of a processing part for generating training data.

[FIG. 44] FIG. 44 shows a schematic diagram for describing a neural network.

FIG. 44(a) shows a schematic diagram showing an outline of the neural network. FIG. 44(b) shows a schematic diagram showing an arithmetic operation at each node. FIG. 44(c) shows a schematic diagram showing arithmetic operations between nodes.

[FIG. 45] FIG. 45 shows a mix matrix of a determination result according to a reference method and a determination result using the deep learning algorithm.

[FIG. 46] FIG. 46(a) shows an ROC curve of neutrophil. FIG. 46(b) shows an ROC curve of lymphocyte. FIG. 46(c) shows an ROC curve of monocyte.

[FIG. 47] FIG. 47(a) shows an ROC curve of eosinophil. FIG. 47(b) shows an ROC curve of basophil. FIG. 47(c) shows an ROC curve of control blood (CONT).

[FIG. 48] FIG. 48 shows a configuration example of a cell analyzer as an image analyzer.

[FIG. 49] FIG. 49 shows a configuration example of a processing part.

[FIG. 50] FIG. 50 shows an example of a generation method for training data.

[FIG. 51] FIG. 51 shows an example of a label value.

[FIG. 52] FIG. 52 shows an example of an image analysis method.

[FIG. 53] FIG. 53 shows an embodiment of an analysis result.

DESCRIPTION OF EMBODIMENTS

Hereinafter, outlines and embodiments of the present invention will be described in detail with reference to the attached drawings. In the following description and drawings, the same reference characters denote the same or similar components, and thus, description of the same or similar components is omitted.

[1. Cell analysis method]

The present embodiment discloses a cell analysis method that includes: in a cell analyzer including a host processor and a parallel-processing processor, obtaining, on the basis of control by the host processor, data regarding each of cells; executing, by the parallel-processing processor, parallel processing regarding the data; and generating, on the basis of the result of the parallel processing, information regarding the cell type with respect to each of the cells.

According to the present analysis method, even when data having a huge volume of several hundred megabytes to several gigabytes per specimen is analyzed, processing regarding data of cells can be executed in parallel by the parallel-processing processor provided separately from the host processor. Therefore, for example, even when cells are classified by a deep learning algorithm that uses data having a huge volume, processing of data is concluded only by the cell analyzer. For example, it is not necessary to transmit data of cells via the Internet or an intranet to an analysis server having a deep learning algorithm stored therein. Therefore, according to the present analysis method, it is not necessary to transmit a large volume of data from a cell analyzer to an analysis server, and to obtain an analysis result returning from the analysis server. Thus, the throughput of the cell analyzer can be maintained while the classification accuracy of cells is improved.

An example of an outline of the present embodiment will be described with reference to FIG. 1. FIG. 1(a) is a diagram schematically showing white blood cell classification according to a conventional method, and FIG. 1(b) is a diagram schematically showing white blood cell classification according to the present method. In FIG. 1(a) and FIG. 1(b), FSC represents an analog signal indicating the signal intensity of forward scattered light, SSC represents an analog signal of side scattered light, and SFL represents an analog signal indicating the signal intensity of side fluorescence. As shown in FIG. 1(a), in the conventional method, each individual cell contained in a specimen is measured by a flow cytometer, and the peak heights of the pulses of the analog signals of the respective forward scattered light, side scattered light, and side fluorescence are obtained as a forward scattered light intensity, a side scattered light intensity, and a side fluorescence intensity. Next, on the basis of the forward scattered light intensity, the side scattered light intensity, and the side fluorescence intensity, each cell is classified into a specific type. The result of the classifications of the cells is displayed as a scattergram as shown in FIG. 1(a). In the scattergram in FIG. 1(a), the horizontal axis represents the intensity of the side scattered light and the vertical axis represents the intensity of the side fluorescence.

As shown in FIG. 1(a), in the conventional white blood cell classification, the type of each blood cell is determined on the basis of only the information of the peak height of an analog waveform. In contrast to this, in the method according to the present embodiment, as data regarding cells in a specimen, as shown in FIG. 1(b), the entirety of the waveform of an analog signal obtained from a single cell by a flow cytometer is analyzed as analysis target data, whereby the cell is classified. In FIG. 1(b), a waveform obtained by drawing an analog signal obtained by a flow cytometer is shown. However, as described later, data regarding a cell in a specimen in the present embodiment means digital data (waveform data described later) which uses, as elements, the values indicating the signal intensity at a plurality of time points obtained by performing A/D conversion on this analog signal. This digital data group is matrix data, and in the present embodiment, is matrix data (i.e., one-dimensional array data) composed of one row $\times$ a plurality of columns, for example.

In the present embodiment, a deep learning algorithm 50 before being trained shown in FIG. 1(b) is caused to learn waveform data for each cell type. Then, waveform data of each cell of which the cell type is unknown and that is contained in the specimen is inputted to a trained deep learning algorithm 60, whereby a determination result of the cell type with respect to each cell is derived from the deep learning algorithm 60. The deep learning algorithm 50, 60 is one of artificial intelligence algorithms, and configured as a neural network that includes a middle layer composed of multiple layers. In the present embodiment, when processing regarding analysis of waveform data is to be executed according to the trained deep learning algorithm 60, a large number of matrix operations included in the deep learning algorithm 60 is executed by parallel processing, by using a parallel-processing processor installed in the cell analyzer. The cell analyzer includes: a parallel-processing processor capable of executing parallel processing; and an execution instruction processor (hereinafter, simply referred to as "processor") that causes the parallel-processing processor to execute parallel processing.

Hereinafter, each individual cell in a biological sample subject to analysis for the purpose of determining the cell type thereof will also be referred to as an "analysis target cell". **In** other words, a biological sample can contain a plurality of analysis target cells. A plurality of cells can include cells of a plurality of types to be analyzed.

An example of a biological sample is a biological sample collected from a subject. For example, the biological sample can include peripheral blood such as venous blood and arterial blood, urine, and a body fluid other than blood and urine. Examples of the body fluid other than blood and urine can include bone marrow aspirate, ascites, pleural effusion, cerebrospinal fluid, and the like. Hereinafter, the body fluid other than blood and urine may be simply referred to as a "body fluid". The blood sample may be any blood sample that is in a state where the number of cells can be counted and the cell types can be determined. Preferably, blood is peripheral blood. Examples of blood include peripheral blood collected using an anticoagulant agent such as ethylenediamine tetraacetate (sodium salt or potassium salt), heparin sodium, or the like. Peripheral blood may be collected from an artery or may be collected from a vein.

The cell types to be determined in the present embodiment are those according to the cell types based on morphological classification, and are different depending on the kind of the biological sample. When the biological sample is blood and the blood is collected from a healthy individual, the cell types to be determined in the present embodiment include, for example, red blood cell, nucleated cell such as white blood cell, platelet, and the like. Nucleated cells include, for example, neutrophils, lymphocytes, monocytes, eosinophils, and basophils. Neutrophils include, for example, segmented neutrophils and band neutrophils. Meanwhile, when blood is collected from an unhealthy individual, nucleated cells may include, for example, at least one type selected from the group consisting of immature granulocyte and abnormal cell. Such cells are also included in the cell types to be determined in the present embodiment. Immature granulocytes can include, for example, cells such as metamyelocytes, myelocytes, promyelocytes, and myeloblasts.

The nucleated cells may include, in addition to normal cells, abnormal cells that are not contained in peripheral blood of a healthy individual. Examples of abnormal cells are cells that appear when a person has a certain disease, and such abnormal cells are tumor cells, for example. **In** a case of the hematopoietic system, the certain disease can be a disease selected from the group consisting of, for example: myelodysplastic syndrome; leukemia such as acute myeloblastic leukemia, acute myeloblastic leukemia, acute promyelocytic leukemia, acute myelomonocytic leukemia, acute monocytic leukemia, erythroleukemia, acute megakaryoblastic leukemia, acute myeloid leukemia, acute lymphocytic leukemia, lymphoblastic leukemia, chronic myelogenous leukemia, or chronic lymphocytic leukemia; malignant lymphoma such as Hodgkin’s lymphoma or non-Hodgkin’s lymphoma; and multiple myeloma.

Further, abnormal cells can include, for example, cells that are not usually observed in peripheral blood of a healthy individual, such as: lymphoblasts; plasma cells; atypical lymphocytes; reactive lymphocytes; erythroblasts, which are nucleated erythrocytes, such as proerythroblasts, basophilic erythroblasts, polychromatic erythroblasts, orthochromatic erythroblasts, promegaloblasts, basophilic megaloblasts, polychromatic megaloblasts, and orthochromatic megaloblasts; megakaryocytes including micromegakaryocytes; and the like.

When the biological sample is urine, the cell types to be determined in the present embodiment can include, for example, red blood cell, white blood cell, epithelial cell such as that of transitional epithelium, squamous epithelium, and the like. Examples of abnormal cells include, for example, bacteria, fungi such as filamentous fungi and yeast, tumor cells, and the like.

When the biological sample is a body fluid that usually does not contain blood components, such as ascites, pleural effusion, or spinal fluid, the cell types can include, for example, red blood cell, white blood cell, and large cell. The "large cell" here means a cell that is separated from an inner membrane of a body cavity or a peritoneum of a viscus, and that is larger than white blood cells. For example, mesothelial cells, histiocytes, tumor cells, and the like correspond to the "large cell".

When the biological sample is bone marrow aspirate, the cell types to be determined in the present embodiment can include, as normal cells, mature blood cells and immature hematopoietic cells. Mature blood cells include, for example, red blood cells, nucleated cells such as white blood cells, platelets, and the like. Nucleated cells such as white blood cells include, for example, neutrophils, lymphocytes, plasma cells, monocytes, eosinophils, and basophils. Neutrophils include, for example, segmented neutrophils and band neutrophils. Immature hematopoietic cells include, for example, hematopoietic stem cells, immature granulocytic cells, immature lymphoid cells, immature monocytic cells, immature erythroid cells, megakaryocytic cells, mesenchymal cells, and the like. Immature granulocytes can include cells such as, for example, metamyelocytes, myelocytes, promyelocytes, myeloblasts, and the like. Immature lymphoid cells include, for example, lymphoblasts and the like. Immature monocytic cells include monoblasts and the like. Immature erythroid cells include, for example, nucleated erythrocytes such as proerythroblasts, basophilic erythroblasts, polychromatic erythroblasts, orthochromatic erythroblasts, promegaloblasts, basophilic megaloblasts, polychromatic megaloblasts, and orthochromatic megaloblasts. Megakaryocytic cells include, for example, megakaryoblasts and the like.

Examples of abnormal cells that can be included in bone marrow include, for example, hematopoietic tumor cells of a disease selected from the group consisting of: myelodysplastic syndrome; leukemia such as acute myeloblastic leukemia, acute myeloblastic leukemia, acute promyelocytic leukemia, acute myelomonocytic leukemia, acute monocytic leukemia, erythroleukemia, acute megakaryoblastic leukemia, acute myeloid leukemia, acute lymphocytic leukemia, lymphoblastic leukemia, chronic myelogenous leukemia, or chronic lymphocytic leukemia; malignant lymphoma such as Hodgkin’s lymphoma or non-Hodgkin’s lymphoma; and multiple myeloma, which have been described above, and metastasized tumor cells of a malignant tumor developed in an organ other than bone marrow.

FIG. 1 shows an example of using, as the signal obtained from each cell, a forward scattered light signal, a side scattered light signal, and a side fluorescence signal, which are optical signals obtained by applying light to the cell flowing in a flow cell. However, the signal is not limited in particular as long as the signal indicates a feature of each cell and allows classification of cells for each type.

The signal obtained from each cell may be any of a signal indicating a morphological feature of the cell, a signal indicating a chemical feature thereof, a signal indicating a physical feature thereof, and a signal indicating a genetic feature thereof, but, preferably, is a signal indicating a morphological feature of the cell. The signal indicating a morphological feature of the cell is, preferably, an optical signal obtained from the cell.

Preferably, the optical signal is a light signal obtained as an optical response as a result of application of light to the cell. The light signal can include at least one type selected from a signal based on light scattering, a signal based on light absorption, a signal based on transmitted light, and a signal based on fluorescence.

The signal based on light scattering can include a scattered light signal caused by light application and a light loss signal caused by light application. The scattered light signal serves as a parameter that indicates a feature of a cell and that is different in accordance with the light reception angle of scattered light with respect to the advancing direction of application light. The forward scattered light signal is used as a parameter that indicates the size of the cell. The side scattered light signal is used as a parameter that indicates complexity of the nucleus of the cell.

"Forward" of the forward scattered light means the advancing direction of light emitted from a light source. When the angle of application light is defined as 0 degrees, "forward" can include a forward low angle at which the light reception angle is about 0 to 5 degrees, and/or a forward high angle at which the light reception angle is about 5 to 20 degrees. "Side" is not limited as long as the "side" does not overlap "forward". When the angle of application light is defined as 0 degrees, "side" can include a light reception angle being about 25 degrees to 155 degrees, preferably about 45 degrees to 135 degrees, and more preferably about 90 degrees.

The signal based on light scattering may include polarized light or depolarized light as a component of the signal. For example, scattered light caused by application of light to a cell is received through a polarizing plate, whereby only scattered light polarized at a specific angle can be received. Meanwhile, when light is applied to a cell through a polarizing plate, and the resultant scattered light is received through a polarizing plate that allows passage therethrough of only polarized light having an angle different from that of the polarizing plate for light application, only depolarized scattered light can be received.

A light loss signal indicates the loss amount of received light based on decrease, of the received light amount at a light receiving part, which is caused by application of light to a cell and scattering of the light. Preferably, the light loss signal is obtained as a light loss (axial light loss) in the optical axis direction of the application light. The light loss signal can be expressed as a proportion of the received light amount at the time of flowing of a cell in the flow cell, when the received light amount at the light receiving part in a state where the cell is not flowing in the flow cell is defined a 100%. Similar to the forward scattered light signal, the axial light loss is used as a parameter that indicates the size of the cell, but the signal that is obtained differs depending on whether the cell has translucency or not.

The signal based on fluorescence may be fluorescence that is excited as a result of application of light to a cell labeled with a fluorescent substance, or may be an intrinsic fluorescence that occurs from a non-stained cell. The fluorescent substance may be a fluorescent dye that binds to nucleic acid or membrane protein, or may be a labeled antibody obtained by modifying, with a fluorescent dye, an antibody that binds to a specific protein of a cell.

The optical signal may be obtained in a form of image data obtained by applying light to a cell and capturing an image of the cell to which the light has been applied. The image data can be obtained by capturing, with an imaging element such as a TDI camera or a CCD camera, an image of each individual cell flowing in a flow path, by use of a so-called imaging flow cytometer. Alternatively, a specimen or a measurement sample containing cells is applied, sprayed, or spot-applied on a slide glass, and an image of the slide glass is captured by an imaging element, whereby image data of cells may be obtained.

The signal obtained from a cell is not limited to an optical signal, and may be an electrical signal obtained from the cell. As for the electrical signal, for example, DC current is applied to the flow cell, and change in impedance caused by a cell flowing in the flow cell may be used as the electrical signal. The thus obtained electrical signal serves as a parameter that reflects the volume of the cell. Alternatively, as for the electrical signal, change in impedance at the time of application of a radio frequency to a cell flowing in the flow cell may be used as the electrical signal. The thus obtained electrical signal serves as a parameter that reflects the electric conductivity of the cell.

The signal obtained from a cell may be a combination of at least two kinds of signals out of the above-described signals obtained from a cell. Through combination of a plurality of signals, the features of a cell can be pleiotropically analyzed, and thus, cell classification with a higher accuracy is enabled. As for the combination, for example, at least two out of a plurality of optical signals, e.g., a forward scattered light signal, a side scattered light signal, and a fluorescence signal, may be combined. Alternatively, scattered light signals having different angles, e.g., a low angle scattered light signal and a high angle scattered light signal, may be combined. Still alternatively, an optical signal and an electrical signal may be combined. The kind and number of signals to be combined are not limited in particular.

**In** the cell analysis method of the present embodiment, the cell type determination method is not limited to a method that uses a deep learning algorithm. From individual cells passing through a predetermined position in a flow path, a signal intensity is obtained, for each of the cells, at a plurality of time points in a time period while the cell is passing through the predetermined position, and on the basis of a result in which the obtained signal intensities at the plurality of time points regarding the each individual cell are recognized as a pattern, the cell type may be determined. The pattern may be recognized as a numerical pattern of signal intensities at a plurality of time points, or may be recognized as a shape pattern obtained when signal intensities at a plurality of time points are plotted on a graph. When the pattern is recognized as a numerical pattern, if a numerical pattern of an analysis target cell and a numerical pattern for which the cell type is already known are compared with each other, the cell type can be determined. For the comparison between the numerical pattern of an analysis target cell and a control numerical pattern, Spearman rank correlation, z-score, or the like can be used, for example. When the pattern of the graph shape of an analysis target cell and the pattern of a graph shape for which the cell type is already known are compared with each other, the cell type can be determined. For the comparison between the pattern of the graph shape of an analysis target cell and the pattern of the graph shape for which the cell type is already known, geometric shape pattern matching may be used, or a feature descriptor represented by SIFT Descriptor may be used, for example.

<Outline of cell analysis method>

Next, with reference to examples shown in FIG. 2, and FIG. 3 to FIG. 5, a generation method for training data 75 and an analysis method for waveform data will be described.

<Waveform data>

FIG. 2 is a schematic diagram for describing waveform data to be used in the present analysis method. As shown in FIG. 2(a), when a specimen containing a cell C is caused to flow in a flow cell FC, and light is applied to the cell C flowing in the flow cell FC, forward scattered light FSC is generated in a forward direction with respect to the advancing direction of light. Similarly, side scattered light SSC and side fluorescence SFL are generated to a side direction with respect to the advancing direction of light. The forward scattered light is received by a first light receiving part D1, and a signal corresponding to the received light amount is outputted. The side scattered light is received by a second light receiving part D2, and a signal corresponding to the received light amount is outputted. The side fluorescence is received by a third light receiving part D3, and a signal corresponding to the received light amount is outputted. Accordingly, an analog signal representing change in the signal associated with a lapse of time is outputted from each of the light receiving parts D1 to D3. An analog signal corresponding to the forward scattered light will be referred to as a "forward scattered light signal", an analog signal corresponding to the side scattered light will be referred to as a "side scattered light signal", and an analog signal corresponding to the side fluorescence will be referred to as a "fluorescence signal". One pulse of each analog signal corresponds to one cell.

The analog signals are inputted to an A/D converter, to be converted to digital signals. FIG. 2(b) schematically shows conversion to a digital signal performed by the A/D converter. Here, in order to simplify description, the analog signal is depicted to be directly inputted to the A/D converter. The analog signal may be directly converted, without changing the level thereof, to a digital signal. However, processing such as noise removal, baseline correction, and normalization may be performed as necessary. As shown in FIG. 2(b), from a start point, which is the time point when the level of the forward scattered light signal, among the analog signals inputted from the light receiving parts D1 to D3, has reached a level set as a predetermined threshold, the A/D converter samples the forward scattered light signal, the side scattered light signal, and the fluorescence signal. The A/D converter samples the respective analog signals at a predetermined sampling rate (e.g., sampling at 1024 points at a 10 nanosecond interval, sampling at 128 points at an 80 nanosecond interval, sampling at 64 points at a 160 nanosecond interval, or the like).

FIG. 2(c) schematically shows waveform data obtained through sampling. Through the sampling, as waveform data corresponding to one cell, matrix data that has, as elements, values digitally indicating the analog signal level at a plurality of time points is obtained. In this manner, the A/D converter generates a digital signal of forward scattered light, a digital signal of side scattered light, and a digital signal of side fluorescence that correspond to one cell. The A/D conversion is repeated until the number of cells in the digital signal reaches a predetermined number, or until a predetermined time period has elapsed from the start of causing the specimen to flow in the flow cell. Accordingly, as shown in FIG. 2(c), a digital signal obtained by combining waveform data of N cells contained in one specimen is obtained. A set of sampling data of each cell (in the example in FIG. 2, the set of 1024 digital values obtained every 10 nanoseconds from t=0ns to t=10240 ns) will be referred to as waveform data, and the set of waveform data obtained from one specimen will be referred to as a digital signal.

Each piece of waveform data generated by the A/D converter may be provided with an index for identifying the corresponding cell. As the indexes, for example, integers of 1 to N are provided in the sequential order of the generated pieces of waveform data, and the waveform data of forward scattered light, the waveform data of side scattered light, and the waveform data of side fluorescence that have been obtained from the same cell are each provided with the same index.

Since one piece of waveform data corresponds to one cell, the index corresponds to the cell that has been measured. Since an identical index is provided to the pieces of the waveform data that correspond to the same cell, a deep learning algorithm described later can analyze, as one set, the waveform data of forward scattered light, the waveform data of side scattered light, and the waveform data of fluorescence that correspond to an individual cell, and can classify the type of the cell.

<Generation of training data>

FIG. 3 is a schematic diagram showing an example of a generation method for training data to be used for training a deep learning algorithm for determining the type of a cell. The training data 75 is waveform data generated on the basis of an analog signal 70a of forward scattered light (FSC), an analog signal 70b of side scattered light (SSC) and an analog signal 70c of side fluorescence (SFL) which have been obtained with respect to a cell contained in a specimen through measurement of the specimen performed by a flow cytometer. The method for obtaining the waveform data has been described above.

As for the training data 75, for example, a specimen is measured by a flow cytometer, and waveform data of a cell determined, as a result of analyzing cells contained in the specimen on the basis of a scattergram according to a conventional method, to have a high possibility of being a specific cell type can be used. An example using a blood cell counter will be described. First, a blood specimen is measured by a flow cytometer, and waveform data of forward scattered light, side scattered light, and fluorescence of each individual cell contained in the specimen is accumulated. On the basis of the side scattered light intensity (the height of the pulse of the side scattered light signal) and the fluorescence intensity (the height of the pulse of the fluorescence signal), each cell is classified into a group of neutrophil, lymphocyte, monocyte, eosinophil, basophil, immature granulocyte, or abnormal cell. A label value corresponding to the classified cell type is provided to the waveform data of the cell, whereby training data is obtained. For example, the mode, the average value, or the median of the side scattered light intensity and the side fluorescence intensity of cells included in the neutrophil group is obtained, representative cells are identified on the basis of the value, and a label value "1" corresponding to neutrophil is provided to the waveform data of these cells, whereby training data can be obtained. The generation method for the training data is not limited thereto. For example, only specific cells are recovered by a cell sorter, each cell is measured by a flow cytometer, and a label value for the cell is provided to the obtained waveform data, whereby training data may be obtained.

The analog signals 70a, 70b, 70c respectively represent a forward scattered light signal, a side scattered light signal, and a side fluorescence signal at the time when a neutrophil has been measured by a flow cytometer. When these analog signals are subjected to A/D conversion as described above, waveform data 72a of the forward scattered light signal, waveform data 72b of the side scattered light signal, and waveform data 72c of the side fluorescence signal are obtained. Cells adjacent each other in each of the waveform data 72a, 72b, 72c each store a signal level at an interval corresponding to the sampling rate, e.g., a 10 nanosecond interval. The pieces of the waveform data 72a, 72b, 72c are each combined with a label value 77 indicating the type of the cell being the source of the data, and the three pieces of waveform data corresponding to the cell, in other words, the data of the three signal intensities (the signal intensity of forward scattered light, the signal intensity of side scattered light, and the signal intensity of side fluorescence), are inputted, so as to form a set, as the training data 75 to the deep learning algorithm 50. **In** the example in FIG. 3, since the cell being the source of the training data is a neutrophil, "1" is provided as the label value 77 indicating that the cell is a neutrophil, to the waveform data 72a, 72b, 72c, whereby the training data 75 is generated. FIG. 4 shows an example of the label value 77. Since the training data 75 is generated for each cell type, as for the label value, a label value 77 different in accordance with the cell type is provided.

<Outline of deep learning>

Using FIG. 3 as an example, an outline of training of a neural network will be described. Preferably, a neural network 50 is a convolutional neural network having a convolution layer. The number of nodes of an input layer 50a in the neural network 50 corresponds to the number of elements of the array included in the waveform data of the training data 75 to be inputted. The number of elements of the array is equal to the sum of the number of elements of the waveform data 72a, 72b, 72c of forward scattered light, side scattered light, and side fluorescence which correspond to one cell. In the example in FIG. 3, each of the waveform data 72a, 72b, 72c includes 1024 elements, and thus, the number of nodes of the input layer 50a is $1024\times3=3072$. The waveform data 72a, 72b, 72c is inputted to the input layer 50a of the neural network 50. The label value 77 of each piece of the waveform data of the training data 75 is inputted to an output layer 50b of the neural network, whereby the neural network 50 is trained. A reference character 50c in FIG. 3 represents the middle layer.

<Analysis method for waveform data>

FIG. 5 shows an example of a method for analyzing waveform data of a cell being an analysis target. In the analysis method for the waveform data, from an analog signal 80a of forward scattered light, an analog signal 80b of side scattered light, and an analog signal 80c of side fluorescence obtained from an analysis target cell by a flow cytometer, analysis data 85 composed of waveform data obtained by the above-described method is generated.

Preferably, the analysis data 85 and the training data 75 have the same obtaining condition at least. The obtaining condition includes conditions for measuring cells contained in a specimen by a flow cytometer, e.g., a preparation condition for a measurement sample, the flow speed at which the measurement sample is caused to flow in a flow cell, the intensity of light to be applied to the flow cell, the amplification factor at light receiving parts that receive scattered light and fluorescence, and the like. The obtaining condition further includes a sampling rate at the time of performing A/D conversion on an analog signal.

When the analysis target cell flows in a flow cell, the analog signal 80a of forward scattered light, the analog signal 80b of side scattered light, and the analog signal 80c of side fluorescence are obtained. When these analog signals 80a, 80b, 80c are subjected to A/D conversion as described above, the time points when the signal intensities have been obtained are synchronized for each cell, and waveform data 82a of the forward scattered light signal, waveform data 82b of the side scattered light signal, and waveform data 82c of the side fluorescence signal are obtained. The pieces of the waveform data 82a, 82b, 82c are combined such that the pieces of data of the three signal intensities (the signal intensity of forward scattered light, the signal intensity of side scattered light, and the signal intensity of side fluorescence) of each cell form a set, and the resultant set is inputted as the analysis data 85 to the deep learning algorithm 60.

When the analysis data 85 has been inputted to an input layer 60a of a neural network 60 forming the trained deep learning algorithm 60, an analysis result 83 is outputted from an output layer 60b, as classification information regarding the type of the cell and corresponding to the analysis data 85. A reference character 60c in FIG. 5 represents the middle layer. The classification information regarding the cell type is, for example, a probability at which the cell belongs to each of a plurality of cell types. Further, it may be determined that the analysis target cell for which the analysis data 85 has been obtained belongs to the classification that has the highest value among the probabilities, and the analysis result 83 may include a label value 82 or the like being an identifier representing the cell type thereof. The analysis result 83 may be the label value itself, or may be data obtained by replacing the label value with information (e.g., character string) that indicates the cell type. In the example in FIG. 5, on the basis of the analysis data 85, the deep learning algorithm 60 outputs a label value "1", which has the highest probability that the analysis target cell for which the analysis data 85 has been obtained belongs to the classification. Further, character data "neutrophil" corresponding to this label value is outputted as the analysis result 83. The output of the label value may be performed by the deep learning algorithm 60, but another computer program may output a most preferable label value on the basis of the probability calculated by the deep learning algorithm 60.

[2. Cell analyzer and measurement of biological sample in the cell analyzer]

The waveform data of a cell according to the present embodiment, and the analog signal of the cell being the source of the waveform data can be obtained in a first cell analyzer 4000 or a second cell analyzer 4000'. (a) of FIG. 6 shows an example of the appearance of a cell analyzer 4000. (b) of FIG. 6 shows an example of a cell analyzer 4000'. In (a) of FIG. 6, the cell analyzer 4000 includes: a measurement unit 400; and a processing unit 300 for controlling setting of the measurement condition for a sample and measurement thereof in the measurement unit 400, and for analyzing an analysis result of data of each cell by the deep learning algorithm 60. In (b) in FIG. 6, the cell analyzer 4000' includes: a measurement unit 500; and a processing unit 300 for controlling setting of the measurement condition for a sample and measurement thereof in the measurement unit 500, and for analyzing an analysis result of data of each cell by the deep learning algorithm 60. The measurement unit 400, 500 and the processing unit 300 can be communicably connected to each other in a wired or wireless manner. Hereinafter, a configuration example of the measurement unit 400, 500 will be shown below, but the present embodiment should not be construed to be limited to the example below. The processing unit 300 may be used in common by a vendor-side apparatus 100 described later.

<First cell analyzer and preparation of measurement sample>

(Configuration of measurement unit and processing unit)

A configuration example in which the measurement unit 400 is a blood analyzer including a FCM detector being a flow cytometer for detecting each cell in a blood sample, more specifically, the measurement unit 400 is a blood cell counter, will be described.

(Configuration example 1)

With reference to FIG. 7 to FIG. 19, configuration examples of the measurement unit 400 and the processing unit 300 will be described. FIG. 7 shows an example of a block diagram of the measurement unit 400. As shown in FIG. 7, the measurement unit 400 includes: a FCM detector 410 for detecting blood cells; an analog processing part 420 for processing an analog signal outputted from the FCM detector 410; a measurement unit controller 480; a sample preparation part 440; an apparatus mechanism part 430; and a specimen suction part 450.

FIG. 8 is a schematic diagram for describing the specimen suction part 450 and the sample preparation part 440. The specimen suction part 450 includes: a nozzle 451 for suctioning a blood specimen (whole blood) from a blood collection tube T; and a pump 452 for providing a negative pressure/positive pressure to the nozzle. The nozzle 451 is moved upwardly and downwardly by the apparatus mechanism part 430, to be inserted into the blood collection tube T. When the pump 452 provides a negative pressure in a state where the nozzle 451 is inserted in the blood collection tube T, the blood specimen is suctioned via the nozzle 451. The apparatus mechanism part 430 may include a hand member for inverting and stirring the blood collection tube T before suctioning the blood from the blood collection tube T.

The sample preparation part 440 includes five reaction chambers 440a to 440e. The reaction chambers 440a to 440e are used in measurement channels of DIFF, RET, WPC, PLT-F, and WNR, respectively. Each reaction chamber has connected thereto, via flow paths, a hemolytic agent container containing a hemolytic agent and a staining liquid container containing a staining liquid, which serve as reagents for the corresponding measurement channel. One reaction chamber and reagents (a hemolytic agent and a staining liquid) connected thereto form a measurement channel. For example, the DIFF measurement channel is composed of: a DIFF hemolytic agent and a DIFF staining liquid which serve as DIFF measurement reagents; and the DIFF reaction chamber 440a. The other measurement channels are configured in similar manners. Here, an example of a configuration in which one measurement channel includes one hemolytic agent and one staining liquid is shown. However, one measurement channel need not necessarily include both of a hemolytic agent and a staining liquid, and a plurality of measurement channels may share one reagent.

Through horizontal and up-down movement by the apparatus mechanism part 430, the nozzle 451 having suctioned a blood specimen accesses, from above, a reaction chamber, among the reaction chambers 440a to 440e, that corresponds to a measurement channel that corresponds to an order, and the nozzle 451 discharges the suctioned blood specimen. The sample preparation part 440 supplies a corresponding hemolytic agent and a corresponding staining liquid to the reaction chamber having the blood specimen discharged therein, to mix the blood specimen, the hemolytic agent, and the staining liquid in the reaction chamber, thereby preparing a measurement sample. The prepared measurement sample is supplied from the reaction chamber to the FCM detector 410 via a flow path, to be subjected to measurement of cells by flow cytometry.

FIG. 9 shows a configuration example of an optical system of the FCM detector 410. As shown in FIG. 9, in measurement by a flow cytometer, when each cell contained in a measurement sample passes through a flow cell (sheath flow cell) 4113 provided in the flow cytometer, a light source 4111 applies light to the flow cell 4113, and scattered light and fluorescence emitted from the cell in the flow cell 4113 due to this light are detected.

In FIG. 9, light emitted from a laser diode being the light source 4111 is applied via a light application lens system 4112 to each cell passing through the flow cell 4113.

In the present embodiment, the light source 4111 of the flow cytometer is not limited in particular, and a light source 4111 that has a wavelength suitable for excitation of the fluorescent dye is selected. As such a light source 4111, a semiconductor laser light source including a red semiconductor laser light source and/or a blue semiconductor laser light source, a gas laser light source such as an argon laser light source or a helium-neon laser, a mercury arc lamp, or the like is used, for example. In particular, a semiconductor laser light source is suitable because the semiconductor laser light source is very inexpensive when compared with a gas laser light source.

As shown in FIG. 9, forward scattered light emitted from a particle passing through the flow cell 4113 is received by a forward scattered light receiving element 4116 via a condenser lens 4114 and a pin hole part 4115. The forward scattered light receiving element 4116 is a photodiode. Side scattered light is received by a side scattered light receiving element 4121 via a condenser lens 4117, a dichroic mirror 4118, a bandpass filter 4119, and a pin hole part 4120. The side scattered light receiving element 4121 is a photodiode. Side fluorescence is received by a side fluorescence receiving element 4122 via the condenser lens 4117 and the dichroic mirror 4118. The side fluorescence receiving element 4122 is an avalanche photodiode. As the forward scattered light receiving element 4116, the side scattered light receiving element 4121, and the side fluorescence receiving element 4122, a photomultiplier may be used.

Reception light signals outputted from the respective light receiving elements 4116, 4121, 4122 are inputted to the analog processing part 420 via amplifiers 4151, 4152, 4153, respectively.

With reference back to FIG. 7, the analog processing part 420 performs processes such as noise removal and smoothing onto analog signals inputted from the FCM detector 410, and outputs the processed analog signals to the measurement unit controller 480.

The measurement unit controller 480 includes an A/D converter 482, a processor 4831, a RAM 4834, a storage 4835, a bus controller 4850, a parallel-processing processor 4833, and an interface part 489 connected to the processing unit 300. Further, the measurement unit controller 480 includes an interface part 484 connected to the A/D converter 482, and an interface part 488 connected to the apparatus mechanism part 430. As shown in FIG. 7, in the present configuration example, the parallel-processing processor 4833 is installed, in the cell analyzer 4000, in the form of being incorporated in the measurement unit 400.

The processor 4831 is connected to the interface part 489, the interface part 488, the interface part 484, the RAM 4834, and the storage 4835 via a bus 485. The processor 4831 is connected to the parallel-processing processor 4833 via the bus 485. The processing unit 300 is connected to components of the measurement unit 400 via the interface part 489 and the bus 485. The bus 485 is a transmission line having a data transfer rate of not less than several hundred MB/s, for example. The bus 485 may be a transmission line having a data transfer rate of not less than 1 GB/s, for example. The bus 485 performs data transfer on the basis of the PCI-Express or PCI-X standard, for example.

The A/D converter 482 converts an analog signal outputted from the analog processing part 420 into a digital signal. The A/D converter 482 converts the analog signal that is from measurement start of the specimen to measurement end, into a digital signal. When a plurality of kinds of analog signals (e.g., analog signals respectively corresponding to forward scattered light intensity, side scattered light intensity, and fluorescence intensity) are generated through measurement in a certain measurement channel, the A/D converter 482 converts each analog signal that is from measurement start to measurement end, into a digital signal. As described with reference to FIG. 9, for example, three kinds of analog signals (i.e., forward scattered light signal, side scattered light signal, and fluorescence signal) are inputted via a plurality of corresponding signal transmission paths 421 to the A/D converter 482. The A/D converter 482 converts each of the analog signals inputted from the plurality of signal transmission paths 421, into a digital signal. Each signal transmission path 421 is configured to transmit an analog signal as a differential signal, for example.

As described with reference to FIG. 2, the A/D converter 482 samples the analog signals at a predetermined sampling rate (e.g., sampling at 1024 points at a 10 nanosecond interval, sampling at 128 points at an 80 nanosecond interval, sampling at 64 points at a 160 nanosecond interval, or the like). By executing sampling processes on the three kinds of analog signals corresponding to each cell, the A/D converter 482 generates waveform data of the forward scattered light signal, waveform data of the side scattered light signal, and waveform data of the fluorescence signal for each cell. The A/D converter 482 provides an index to each piece of the generated waveform data. As shown in FIG. 2(c), the pieces of the generated waveform data form a digital signal such that pieces of waveform data of N cells contained in one specimen are contiguous with each other. Accordingly, three digital signals corresponding to three kinds of analog signals (forward scattered light signal, side scattered light signal, and fluorescence signal) obtained from N cells are generated.

In addition to generating waveform data from each analog signal, the A/D converter 482 may calculate a peak value from the pulse of the analog signal.

The A/D converter 482 inputs the generated digital signals to the bus 485. The bus controller 4850 transmits the digital signals outputted from the A/D converter 482, to the RAM 4834, through DMA (Direct Memory Access) transfer, for example. The RAM 4834 stores the digital signals.

Using the parallel-processing processor 4833, the processor 4831 executes an analysis process on waveform data included in the generated digital signal, in accordance with the deep learning algorithm 60. That is, the processor 4831 is programed to execute an analysis process on waveform data included in the digital signal, in accordance with the deep learning algorithm 60. Analysis software 4832 for analyzing data of cells on the basis of the deep learning algorithm 60 may be stored in the storage 4835. In this case, the processor 4831 executes the analysis software 4832 stored in the storage 4835, thereby executing a data analysis process based on the deep learning algorithm 60. The processor 4831 is a CPU (Central Processing Unit), for example. For example, Core i9, Core i7, or Core i5 manufactured by Intel Corporation, Ryzen 9, Ryzen 7, Ryzen 5, or Ryzen 3 manufactured by AMD, or the like may be used as the processor 4831.

The processor 4831 controls the parallel-processing processor 4833. The parallel-processing processor 4833 executes parallel processing regarding, for example, matrix operation in accordance with control by the processor 4831. That is, the processor 4831 is a master processor of the parallel-processing processor 4833, and the parallel-processing processor 4833 is a slave processor of the processor 4831. The processor 4831 is also referred to as a host processor or a main processor.

The parallel-processing processor 4833 executes in parallel a plurality of arithmetic processes being at least a part of processing regarding analysis of waveform data. The parallel-processing processor 4833 is a GPU (Graphics Processing Unit), an FPGA (Field Programmable Gate Array), or an ASIC (Application Specific Integrated Circuit), for example. When the parallel-processing processor 4833 is an FPGA, the parallel-processing processor 4833 may have programed therein in advance an arithmetic process regarding the trained deep learning algorithm 60, for example. When the parallel-processing processor 4833 is an ASIC, the parallel-processing processor 4833 may have incorporated therein in advance a circuit for executing the arithmetic process regarding the trained deep learning algorithm 60, or may have a programmable module built therein in addition to such an incorporated circuit, for example. As the parallel-processing processor 4833, GeForce, Quadro, TITAN, Jetson, or the like manufactured by NVIDIA Corporation may be used, for example. In the case of the Jetson series, Jetson Nano, Jetson Tx2, Jetson Xavier, or Jetson AGX Xavier is used, for example.

The processor 4831 executes a calculation process regarding control of the measurement unit 400, for example. The processor 4831 executes a calculation process regarding control signals transmitted/received between the apparatus mechanism part 430, the sample preparation part 440, and the specimen suction part 450, for example. The processor 4831 executes a calculation process regarding transmission/reception of information with respect to the processing unit 300, for example. The processor 4831 executes processes regarding reading out of program data from the storage 4835, developing of a program onto the RAM 4834, and transmission/reception of data with respect to the RAM 4834, for example. The above-described processes executed by the processor 4831 are required to be executed in a predetermined sequential order, for example. For example, when processes needed for control of the apparatus mechanism part 430, the sample preparation part 440, and the specimen suction part 450 are assumed to be A, B, and C, the processes are required to be executed in the sequential order of B, A, and C, in some cases. Since the processor 4831 often executes such continuous processes that depend on a sequential order, even when the number of arithmetic units (each may be referred to as a "processor core", a "core", or the like) is increased, the processing speed is not always increased.

Meanwhile, the parallel-processing processor 4833 executes a large number of regular calculation processes such as arithmetic operations on matrix data including a large number of elements, for example. In the present embodiment, the parallel-processing processor 4833 executes parallel processing in which at least a part of processes of analyzing waveform data in accordance with the deep learning algorithm 60 are parallelized. The deep learning algorithm 60 includes a large number of matrix operations, for example. For example, the deep learning algorithm 60 may include at least 100 matrix operations, or may include at least 1000 matrix operations. The parallel-processing processor 4833 has a plurality of arithmetic units, and the respective arithmetic units can simultaneously execute matrix operations. That is, the parallel-processing processor 4833 can execute, in parallel, matrix operations by a plurality of respective arithmetic units, as parallel processing. For example, a matrix operation included in the deep learning algorithm 60 can be divided into a plurality of arithmetic processes that are not dependent on a sequential order with each other. The thus divided arithmetic processes can be executed in parallel by a plurality of arithmetic units, respectively. These arithmetic units may be each referred to as a "processor core", a "core", or the like.

As a result of execution of such parallel processing, speed up of arithmetic processing in the entirety of the measurement unit 400 can be realized. A process such as a matrix operation included in the deep learning algorithm 60 may be referred to as "Single Instruction Multiple Data (SIMD) processing", for example. The parallel-processing processor 4833 is suitable for such an SIMD operation, for example. Such a parallel-processing processor 4833 may be referred to as a vector processor.

As described above, the processor 4831 is suitable for executing diverse and complicated processes. Meanwhile, the parallel-processing processor 4833 is suitable for executing in parallel a large number of regular processes. Through parallel execution of a large number of regular processes, the TAT (Turn Around Time) required for a calculation process is shortened.

The parallel processing to be executed by the parallel-processing processor 4833 is not limited to matrix operations. For example, when the parallel-processing processor 4833 executes a learning process in accordance with the deep learning algorithm 50, differential operations or the like regarding the learning process can be the target of the parallel processing.

As for the number of arithmetic units of the processor 4831, a dual core (the number of cores: 2), a quad core (the number of cores: 4), or an octa core (the number of cores: 8) is adopted, for example. Meanwhile, the parallel-processing processor 4833 has at least ten arithmetic units (the number of cores: 10), and can execute ten matrix operations in parallel, for example. The parallel-processing processor 4833 that has, for example, several-ten arithmetic units also exists. The parallel-processing processor 4833 that has, for example, at least 100 arithmetic units (the number of cores: 100) and that can execute 100 matrix operations in parallel also exists. The parallel-processing processor 4833 that has, for example, several hundred arithmetic units also exists. The parallel-processing processor 4833 that has, for example, at least 1000 arithmetic units (the number of cores: 1000) and that can execute 1000 matrix operations in parallel also exists. The parallel-processing processor 4833 that has, for example, several thousand arithmetic units also exists.

FIG. 11 shows a configuration example of the parallel-processing processor 4833. The parallel-processing processor 4833 includes a plurality of arithmetic units 4836 and a RAM 4837. The respective arithmetic units 4836 execute arithmetic processes on matrix data in parallel. The RAM 4837 stores data regarding the arithmetic processes executed by the arithmetic units 4836. The RAM 4837 is a memory that has a capacity of at least 1 gigabyte. The RAM 4837 may be a memory that has a capacity of 2 gigabytes, 4 gigabytes, 6 gigabytes, 8 gigabytes, 10 gigabytes, or more. Each arithmetic unit 4836 obtains data from the RAM 4837 and executes an arithmetic process. The arithmetic unit 4836 may be referred to as a "processor core", a "core", or the like.

FIG. 12 to FIG. 14 each show an installation example of the parallel-processing processor 4833 to the measurement unit 400. FIG. 12 to FIG. 14 each show an example in which the parallel-processing processor 4833 is installed, in the cell analyzer 4000, in the form of being incorporated in the measurement unit 400. FIG. 12 and FIG. 13 each show an installation example in which the processor 4831 and the parallel-processing processor 4833 are provided as separate bodies. As shown in FIG. 12, the processor 4831 is installed on a substrate 4838, for example. The parallel-processing processor 4833 is installed on a graphic board 4830, and the graphic board 4830 is connected to the substrate 4838 via a connector 4839, for example. The processor 4831 is connected to the parallel-processing processor 4833 via the bus 485. As shown in FIG. 13, the parallel-processing processor 4833 may be directly installed on the substrate 4838, and connected to the processor 4831 via the bus 485, for example. FIG. 14 shows an installation example in which the processor 4831 and the parallel-processing processor 4833 are integrally provided. As shown in FIG. 14, the parallel-processing processor 4833 may be built in the processor 4831 installed on the substrate 4838, for example.

FIG. 15 shows another installation example of the parallel-processing processor 4833 to the measurement unit 400. FIG. 15 shows an example in which the parallel-processing processor 4833 is installed to the measurement unit 400 by means of an external apparatus 4800 connected to the measurement unit 400. For example, the parallel-processing processor 4833 is mounted on the external apparatus 4800 being a USB (Universal Serial Bus) device, and this USB device is connected to the bus 485 via an interface part 487, whereby the parallel-processing processor 4833 is installed to the cell analyzer 4000. The USB device may be a small device such as a USB dongle, for example. The interface part 487 is a USB interface having a transfer rate of several hundred Mbps, for example, and more preferably, is a USB interface having a transfer rate of several Gbps to several ten Gbps or higher. As the external apparatus 4800 having the parallel-processing processor 4833 mounted thereon, Neural Compute Stick 2 manufactured by Intel Corporation may be used, for example.

A plurality of USB devices each having the parallel-processing processor 4833 mounted thereon may be connected to the interface part 487, whereby a plurality of the parallel-processing processors 4833 may be installed to the cell analyzer 4000. The parallel-processing processor 4833 mounted on one USB device has a smaller number of arithmetic units 4836 than a GPU or the like in some cases. Therefore, if a plurality of USB devices are connected to the measurement unit 400, scale-up of the number of cores can be realized.

FIG. 16, FIG. 17, and FIG. 18 each show an outline of arithmetic processes executed by the parallel-processing processor 4833 on the basis of control of the analysis software 4832 which operates on the processor 4831. FIG. 16 shows a configuration example of the parallel-processing processor 4833 which executes arithmetic processes. The parallel-processing processor 4833 includes a plurality of the arithmetic units 4836 and the RAM 4837. The processor 4831, which executes the analysis software 4832, can issue an order to the parallel-processing processor 4833 to cause the parallel-processing processor 4833 to execute at least a part of arithmetic processes necessary for analysis of waveform data according to the deep learning algorithm 60. The processor 4831 orders the parallel-processing processor 4833 to execute arithmetic processes regarding waveform data analysis based on the deep learning algorithm. All or at least a part of waveform data corresponding to the signals detected by the FCM detector 410 is stored in the RAM 4834. The data stored in the RAM 4834 is transferred to the RAM 4837 of the parallel-processing processor 4833. The data stored in the RAM 4834 is transferred to the RAM 4837 by a DMA (Direct Memory Access) method, for example. The plurality of arithmetic units 4836 of the parallel-processing processor 4833 respectively execute in parallel arithmetic processes with respect to the data stored in the RAM 4837. Each of the plurality of arithmetic units 4836 obtains necessary data from the RAM 4837, to execute an arithmetic process. Data corresponding to the arithmetic result is stored into the RAM 4837 of the parallel-processing processor 4833. The data corresponding to the arithmetic result is transferred from the RAM 4837 to the RAM 4834 by a DMA method, for example.

FIG. 17 shows an outline of a matrix operation executed by the parallel-processing processor 4833. Prior to analyzing waveform data in accordance with the deep learning algorithm 60, calculation of the product of a matrix (matrix operation) is executed. The parallel-processing processor 4833 executes in parallel a plurality of arithmetic processes regarding the matrix operation, for example. (a) of FIG. 17 shows a calculation formula of the product of a matrix. In the calculation formula shown in (a), a matrix c is obtained by a product of a matrix a of n rows × n columns and a matrix b of n rows × n columns. As shown as an example in FIG. 17, the calculation formula is described in a hierarchical loop syntax. (b) of FIG. 17 shows an example of arithmetic processes executed in parallel in the parallel-processing processor 4833. The calculation formula shown as an example in FIG. 17(a) can be divided into n × n arithmetic processes, n × n being the number of combinations of a loop variable i for the first hierarchical level and a loop variable j for the second hierarchical level, for example. Such divided arithmetic processes are arithmetic processes that are not dependent on each other, and thus can be executed in parallel.

FIG. 18 is a conceptual diagram showing that a plurality of arithmetic processes shown as an example in (b) of FIG. 17 are executed in parallel in the parallel-processing processor 4833. As shown in FIG. 18, each of the plurality of arithmetic processes is assigned to one of the plurality of arithmetic units 4836 of the parallel-processing processor 4833. The respective arithmetic units 4836 execute in parallel the assigned arithmetic processes. That is, the respective arithmetic units 4836 simultaneously execute the divided arithmetic processes.

Through the arithmetic operations shown as an example in FIG. 17 and FIG. 18 performed by the parallel-processing processor 4833, information regarding the probability at which a cell corresponding to the waveform data belongs to each of a plurality of cell types is obtained, for example. On the basis of the results of the arithmetic operations, the processor 4831, which executes the analysis software 4832, performs analysis regarding the cell type of the cell that corresponds to the waveform data. The arithmetic results are stored in the RAM 4837 of the parallel-processing processor 4833, to be transferred from the RAM 4837 to the RAM 4834. The processor 4831 transmits a result of analysis performed on the basis of the arithmetic results stored in the RAM 4834, to the processing unit 300 via the bus 485 and the interface part 489.

The arithmetic operations of the probability at which a cell belongs to each of a plurality of cell types may be performed by a processor different from the parallel-processing processor 4833. For example, the arithmetic results by the parallel-processing processor 4833 may be transferred from the RAM 4837 to the RAM 4834, and on the basis of the arithmetic results read out from the RAM 4834, the processor 4831 may perform arithmetic operations of the information regarding the probability at which the cell corresponding to each piece of waveform data belongs to each of a plurality of cell types. Alternatively, the arithmetic results by the parallel-processing processor 4833 may be transferred from the RAM 4837 to the processing unit 300, and a processor installed in the processing unit 300 may perform arithmetic operations of the information regarding the probability at which the cell corresponding to each piece of waveform data belongs to each of a plurality of cell types.

In the present embodiment, the processes shown in FIG. 17 and FIG. 18 are applied to an arithmetic process (also referred to as a filtering process) regarding a convolution layer in the deep learning algorithm 60, for example.

FIG. 19 shows an outline of an arithmetic process regarding a convolution layer. (a) of FIG. 19 shows an example of waveform data of forward scattered light (FSC), as waveform data to be inputted to the deep learning algorithm 60. As shown in FIG. 2, the waveform data of the present embodiment is one-dimensional matrix data. To put it more simply, the waveform data is an array in which elements are arranged in a line. Here, for convenience of description, the number of elements of the waveform data is assumed to be n (n is an integer of 1 or greater). (a) of FIG. 19 shows a plurality of filters. Each filter is generated through a learning process of the deep learning algorithm 50. Each of the plurality of filters is one-dimensional matrix data indicating features of the waveform data. Although each filter shown in (a) of FIG. 19 is matrix data of 1 row × 3 columns, the number of columns is not limited to three. A matrix operation is performed on each filter and the waveform data that is inputted to the deep learning algorithm 60, whereby features corresponding to the cell type of the waveform data are calculated. (b) of FIG. 19 shows an outline of a matrix operation between waveform data and a filter. As shown in (b) of FIG. 19, a matrix operation is executed while each filter is shifted with respect to the elements of the waveform data, one by one. Calculation of the matrix operation is executed according to Formula 1 below.

[Math 1]

$$\sum_{P=0}^{L-1} \sum_{q=0}^{M-1} h_{pq} x_{i+p,j+q} \qquad \text{(Formula 1)}$$

In Formula 1, the suffixes of x are variables that indicate the row number and the column number of the waveform data. The suffixes of h are variables that indicate the row number and the column number of the filter. In the example shown in FIG. 19, the waveform data is one-dimensional matrix data, and the filter is matrix data of 1 row × 3 columns, and thus, L=1, M=3, p=0, q=0, 1, 2, i=0, and j=0, 1,..., n-1.

The parallel-processing processor 4833 executes in parallel the matrix operation represented by Formula 1, by means of the plurality of respective arithmetic units 4836. On the basis of the arithmetic processes executed by the parallel-processing processor 4833, classification information regarding the cell type of each cell is generated. The generated classification information is transmitted to the processing unit 300 which is used in generation and display of a test result of the specimen based on the classification information.

The measurement unit 400 can process the waveform data and identification information so as to be associated with each other. Specifically, the measurement unit 400 can generate an analysis result (i.e., classification information regarding the cell type of each cell) of the waveform data and identification information so as to be associated with each other. The measurement unit 400 transmits, to the processing unit 300, the classification information regarding the cell type of each cell and identification information in association with each other, for example. Examples of the identification information include: (1) identification information of a biological sample corresponding to the waveform data; (2) identification information of a cell corresponding to the waveform data (e.g., an index described above); (3) identification information of a patient corresponding to the waveform data; (4) identification information of a test corresponding to the waveform data; (5) identification information of a cell analyzer by which the waveform data has been measured; and (6) identification information of a facility (hereinafter, referred to as a "test-related facility") such as a hospital or a test facility where the waveform data has been measured. It should be noted that (1) identification information of a biological sample corresponding to the waveform data can include information for determining the priority of parallel processing such as: information regarding the time at which a measurement order for the biological sample has been registered; information regarding the time at which the analyzer has identified the biological sample; information regarding the time at which the analyzer has started measurement of the biological sample; information for identifying whether the biological sample is an urgent specimen or a routine specimen; and information for identifying whether measurement of the biological sample is re-measurement or new measurement. When the measurement unit 400 receives a test order from, for example, an LIS (Laboratory Information System) or the processing unit 300, the measurement unit 400 can obtain at least one of the above identification information (1) to (6) or a combination thereof from the LIS or the processing unit 300. For example, at least one of (1) to (6) shown as examples is transmitted to the processing unit 300 in association with the classification information, and is provided as a test result to a user via the processing unit 300. A plurality of combinations of (1) to (6) shown as examples may be transmitted to the processing unit 300 in association with the classification information. The measurement unit 400 itself can also generate at least one of the above identification information (1) to (6) or a combination thereof, for example.

Association between the waveform data and the above identification information (at least one of the above (1) to (6) or a combination thereof) may be performed by the processing unit 300. When the processing unit 300 has received a test order from, for example, the LIS, the processing unit 300 obtains identification information of the biological sample or identification information of the patient. The processing unit 300 instructs the measurement unit 400 to perform measurement corresponding to the above test order. When the processing unit 300 has obtained, from the measurement unit 400, a result (i.e., waveform data) corresponding to the measurement instruction, the processing unit 300 associates the result (i.e., the waveform data) with the above identification information.

With reference back to FIG. 6, the processing unit 300 is connected to the processor 4831 via the interface part 489 and the bus 485, and can receive analysis results of the processor 4831 and the parallel-processing processor 4833. The interface part 489 is a USB interface, for example.

FIG. 10 shows a configuration of the processing unit 300. The processing unit 300 includes a processor 3001, a bus 3003, a storage 3004, an interface part 3006, a display part 3015, and an operation part 3016. The processing unit 300 as hardware is implemented by a general personal computer, and functions as a processing unit of the cell analyzer 4000, by executing a dedicated program stored in the storage 3004.

The processor 3001 is a CPU, and can execute a program stored in the storage 3004.

The storage 3004 includes a hard disk device. The storage 3004 stores at least a program 60 for processing the classification information of each cell transmitted from the measurement unit 400 and for generating a test result of the specimen. The test result of a specimen means, as described later, a result of counting blood cells contained in the specimen, on the basis of the analysis result 83 and classification information 82 of each individual cell obtained by the measurement unit 400.

The display part 3015 includes a computer screen. The display part 3015 is connected to the processor 3001 via the interface part 3006 and the bus 3003. The display part 3015 can receive an image signal inputted from the processor 3001, and can display the analysis result 83 received from the measurement unit 400 and a test result obtained by the processor 3001 analyzing the analysis result 83.

The operation part 3016 includes a pointing device including a keyboard, a mouse, or a touch panel. A user such as a doctor or a laboratory technician operates the operation part 3016 to input a measurement order to the cell analyzer 4000, thereby being able to input a measurement instruction in accordance with the measurement order. The operation part 3016 can also receive an instruction of displaying a test result from the user. By operating the operation part 3016, the user can view various types of information regarding the test result, such as a graph, a chart, or flag information provided to the specimen.

The measurement unit 400 described above is connected to the processing unit 300 via the interface part 3006. The processor 4831 of the measurement unit 400 can transmit, to the processor 3001 of the processing unit 300, classification information of each individual cell generated by the deep learning algorithm 60 in association with identification information of the specimen. The processor 3001 stores, into the storage 3004, the analysis result 83 of each cell received from the measurement unit 400, in association with identification information of the specimen.

<Operation of cell analyzer>

With reference to FIG. 20 to FIG. 22, a specimen analysis operation performed by the cell analyzer 4000 will be described.

When the processor 3001 of the processing unit 300 has received a measurement order and a measurement instruction from the user via the operation part 3016, the processor 3001 transmits a measurement command to the measurement unit 400 (step S1).

Upon receiving the measurement command, the processor 4831 of the measurement unit 400 starts measurement of a specimen. The processor 4831 causes the specimen suction part 450 to suction the specimen from a blood collection tube T (step S10). Next, the processor 4831 causes the specimen suction part 450 to dispense the suctioned specimen into one of the reaction chambers 440a to 440e of the sample preparation part 440. The measurement command transmitted from the processing unit 300 in step S1 includes information of a measurement channel for which measurement is requested by the measurement order. On the basis of the information of the measurement channel included in the measurement command, the processor 4831 controls the specimen suction part 450 so as to discharge the specimen into the reaction chamber of the corresponding measurement channel.

The processor 4831 causes the sample preparation part 440 to prepare a measurement sample (step S11). Specifically, upon receiving an order from the processor 4831, the sample preparation part 440 supplies the reagents (hemolytic agent and staining liquid) into the reaction chamber having the specimen discharged therein, to mix the specimen with the reagents. Accordingly, a measurement sample in which red blood cells are hemolyzed by the hemolytic agent and in which cells, such as white blood cells or reticulocytes, serving as the target of the measurement channel are stained by the staining liquid, is prepared in the reaction chamber.

The processor 4831 causes the FCM detector 410 to measure the prepared measurement sample (step S12). Specifically, the processor 4831 controls the apparatus mechanism part 430 to send the measurement sample in the reaction chamber of the sample preparation part 440, to the FCM detector 410. The reaction chamber and the FCM detector 410 are connected to each other by a flow path, and the measurement sample sent from the reaction chamber flows in the flow cell 4113, and is irradiated with laser light by the light source 4111 (see FIG. 9). When a cell contained in the measurement sample passes through the flow cell 4113, light is applied to the cell. Forward scattered light, side scattered light, and side fluorescence generated from the cell are detected by the light receiving elements 4116, 4121, 4122, respectively, and analog signals corresponding to the intensities of the received lights are outputted. The analog signals are outputted to the A/D converter 482 via the analog processing part 420.

The A/D converter 482 samples each analog signal at a predetermined rate, to generate a digital signal including waveform data of each individual cell (step S13). The generation methods for the waveform data and the digital signal have been described above. The processor 4831 takes each digital signal generated by the A/D converter 482, into the RAM 4834. For example, the processor 4831 controls the bus controller 4850 to cause the digital signal generated by the A/D converter 482 to be taken into the RAM 4834 through DMA transfer. Through DMA transfer, the digital signal is directly transferred to the RAM 4834, not via the processor 4831. Specifically, the processor 4831 takes, into the RAM 4834, the digital signal of the forward scattered light signal, the digital signal of the side scattered light, and the digital signal of the fluorescence signal, which have been obtained from each cell contained in the test target specimen. Each digital signal is stored in the RAM 4834.

The processor 4831 executes cell classification based on the waveform data included in each generated digital signal, by using the deep learning algorithm 60 (step S14). The process of S14 will be described later.

The processor 4831 transmits an analysis result 83 including classification information 82 of each individual cell obtained as a result of S14, to the processing unit 300 in association with identification information of the specimen (step S15). As for the analysis results 83, for example, an analysis result 83 of each of a plurality of cells contained in one specimen is sent, in association with identification information of the specimen, to the processing unit 300.

Upon receiving the analysis results 83 from the measurement unit (step S2), the processor 3001 of the processing unit 300 analyzes the analysis results 83 by using a program stored in the storage 3004, and generates a test result of the specimen (step S3). In the process of S3, for example, on the basis of the label values included in the analysis results 83 of the individual cells, the number of cells is counted for each cell type. For example, with respect to one specimen, when there are N pieces of classification information provided with a label value "1" which indicates neutrophil, a counting result that the number of neutrophils = N, is obtained as a test result of the specimen.

The processor 3001 obtains a counting result regarding a measurement item corresponding to the measurement channel on the basis of the analysis results 83, and stores the counting result, together with the identification information of the specimen, into the storage 3004. The measurement item corresponding to the measurement channel is an item of which the counting result is requested by the measurement order. For example, a measurement item corresponding to the DIFF channel is the numbers of five classifications of white blood cells, i.e., monocytes, neutrophils, lymphocytes, eosinophils, and basophils. A measurement item corresponding to the RET channel is the number of reticulocytes. A measurement item corresponding to PLT-F is the number of platelets. A measurement item corresponding to WPC is the number of hematopoietic progenitor cells. A measurement item corresponding to WNR is the numbers of white blood cells and nucleated erythrocytes. The counting result is not limited to that of an item (also referred to as "reportable item") for which measurement as listed above is requested, and can include a counting result of another cell of which measurement can be performed in the same measurement channel. For example, when the measurement channel is DIFF, as shown in FIG. 4, immature granulocytes (IG) and abnormal cells are also included in the counting result in addition to the five classifications of white blood cells. Further, the processor 3001 analyzes the obtained counting result to generate a test result of the specimen, and stores the result into the storage 3004. The analysis of the counting result includes performing determination as to, for example, whether the counting result is in a normal value range, whether any abnormal cell has been detected, whether difference from the previous test result is in an allowable range, and the like.

The processor 3001 displays the generated test result on the display part 3015 (step S4).

Next, with reference to FIG. 21, a process of cell classification in step S14 will be described. The process of cell classification in step S14 is a process performed by the processor 4831 in accordance with operation of the analysis software 4832. The processor 4831 causes each digital signal taken into the RAM 4834 in step S13, to be transferred to the parallel-processing processor 4833 (S101). As shown in FIG. 16, the processor 4831 causes the digital signal to be DMA-transferred from the RAM 4834 to the RAM 4837. For example, the processor 4831 controls the bus controller 4850 to DMA-transfer the digital signal from the RAM 4834 to the RAM 4837.

The processor 4831 instructs the parallel-processing processor 4833 to execute parallel processing onto the waveform data included in the digital signal (S102). The processor 4831 instructs the execution of parallel processing by calling a kernel function of the parallel-processing processor 4833, for example. The process executed by the parallel-processing processor 4833 will be described later with reference to a flowchart shown as an example in FIG. 22. The processor 4831 instructs the parallel-processing processor 4833 to execute a matrix operation regarding the deep learning algorithm 60, for example. The digital signal is decomposed into a plurality of pieces of waveform data, to be sequentially inputted to the deep learning algorithm 60. An index corresponding to each cell and included in the digital signal is not inputted to the deep learning algorithm 60. The waveform data inputted to the deep learning algorithm 60 is subjected to arithmetic operations performed by the parallel-processing processor 4833.

The processor 4831 receives results of arithmetic operations executed by the parallel-processing processor 4833 (S103). The arithmetic results are DMA-transferred from the RAM 4837 to the RAM 4834 as shown in FIG. 16, for example.

On the basis of the arithmetic results by the parallel-processing processor 4833, the processor 4831 generates an analysis result of the cell type of each measured cell (S104).

FIG. 22 shows an operation example of the arithmetic processes of the parallel-processing processor 4833 executed on the basis of an instruction from the processor 4831 according to operation of the analysis software 4832.

The processor 4831, which executes the analysis software 4832, causes the parallel-processing processor 4833 to execute assignment of arithmetic processes to the arithmetic units 4836 (S110). For example, the processor 4831 causes the parallel-processing processor 4833 to execute assignment of arithmetic processes to the arithmetic units 4836 by calling a kernel function of the parallel-processing processor 4833. As shown in FIG. 18, for example, a matrix operation regarding the deep learning algorithm 60 is divided into a plurality of arithmetic processes, and the respective divided arithmetic processes are assigned to the arithmetic units 4836. A plurality of pieces of waveform data are sequentially inputted to the deep learning algorithm 60. A matrix operation corresponding to the waveform data is divided into a plurality of arithmetic processes, to be assigned to the arithmetic units 4836.

The arithmetic processes are processed in parallel by a plurality of arithmetic units 4836 (S111). The arithmetic processes are executed on the plurality of pieces of waveform data.

Arithmetic results generated through the parallel processing by the plurality of arithmetic units 4836 are transferred from the RAM 4837 to the RAM 4834 (S112). For example, the arithmetic results are DMA-transferred from the RAM 4837 to the RAM 4834.

(Configuration example 2)

With reference to FIG. 23 and FIG. 24, another configuration example of the cell analyzer 4000 composed of the measurement unit 400 and the processing unit 300 will be described. In the present configuration example, the parallel-processing processor is provided in the processing unit 300.

FIG. 23 shows another example of a block diagram of the measurement unit 400. Except that the measurement unit 400 shown in FIG. 23 is not provided with the A/D converter 482, the processor 4831, the RAM 4834, the storage 4835, and the parallel-processing processor 4833, and is provided with a connection port 4201, the measurement unit 400 shown in FIG. 23 has configurations and functions similar to those of the measurement unit 400 described with reference to FIG. 6 and FIG. 7 and in related description thereof. A connection cable 4202 is connected to the connection port 4201.

FIG. 24 shows an example of a block diagram of the processing unit 300. As shown in FIG. 24, the processing unit 300 includes the processor 3001, a parallel-processing processor 3002, the storage 3004, a RAM 3005, the interface part 3006, an A/D converter 3008, the bus controller 4850, an interface part 3009, and these are connected to the bus 3003. That is, in the example in FIG. 24, the parallel-processing processor 3002 is installed, in the cell analyzer 4000, in the form of being incorporated in the processing unit 300. The bus 3003 is a transmission line having a data transfer rate of not less than several hundred MB/s, for example. The bus 3003 may be a transmission line having a data transfer rate of not less than 1 GB/s.

The bus 3003 performs data transfer on the basis of the PCI-Express or PCI-X standard, for example. Configurations of the processor 3001, the parallel-processing processor 3002, the storage 3004, and the RAM 3005, and processes executed by these are similar to the configurations and processes of the processor 4831, the parallel-processing processor 4833, the storage 4835, and the RAM 4834 described with reference to FIG. 11 to FIG. 19 above. The A/D converter 3008 samples each analog signal outputted from the measurement unit 400 as described above, and generates a digital signal including waveform data of cells. The generation method for the digital signal has been described above. In the example in FIG. 23 and FIG. 24, the connection cable 4202 is provided with transmission paths the number of which corresponds to the kinds of analog signals transmitted from the measurement unit 400 to the processing unit 300, for example. For example, the connection cable 4202 is implemented by twisted-pair cables, and has pairs of wires, the number of the pairs corresponding to the kinds of analog signals transmitted to the processing unit 300. The transmission path from a connection port 3007 to the A/D converter 3008 may also have wires of which the number corresponds to the kinds of analog signals transmitted to the processing unit 300. In the transmission path from the connection port 3007 to the A/D converter 3008, the analog signals are transmitted as differential signals, for example.

As shown in FIG. 25, the processor 3001 and the parallel-processing processor 3002 have configurations and functions similar to those of the processor 4831 and the parallel-processing processor 4833 described above. The parallel-processing processor 3002 includes a plurality of arithmetic units 3200 and a RAM 3201. Analysis software 3100 for analyzing the cell type of each measured cell is executed on the processor 3001. The parallel-processing processor 3002 need not necessarily be directly connected to the bus 3003. For example, the parallel-processing processor 3002 may be mounted to a USB device, and this USB device may be connected to the bus 3003 via an interface part (not shown), whereby the parallel-processing processor 3002 may be installed as a part of the processing unit 300 to the cell analyzer 4000. This USB device may be a small device such as a USB dongle, for example.

As shown in FIG. 23 and FIG. 24, the processing unit 300 is connected to the interface part 489 of the measurement unit 400 via the interface part 3006. The processing unit 300 transmits control signals of the apparatus mechanism part 430 and the sample preparation part 440, to the measurement unit 400 via the interface part 3006. The interface part 3006 is a USB interface, for example.

In the example shown in FIG. 23 and FIG. 24, the processing unit 300 is connected to the connection port 4201 of the measurement unit 400 via the connection port 3007 connected to the A/D converter 3008 and the connection cable 4202 connected to the connection port 3007, in addition to the interface part 3006. The connection port 4201 is connected to the analog processing part 420. Each analog signal outputted from the connection port 4201 to the processing unit 300 is a signal obtained as a result of the output of the FCM detector 410 of the measurement unit 400 being processed by the analog processing part 420, as described above. The analog processing part 420 performs a process including noise removal on the analog signal inputted from the FCM detector 410. The analog signal having been processed by the analog processing part 420 is transmitted to the processing unit 300 via the connection port 4201 and the connection cable 4202. The connection cable 4202 is formed so as to have a length of, for example, 1 meter or less in order to reduce noise during signal transmission. The analog signal is transmitted as, for example, a differential signal to the processing unit 300 via the connection cable 4202. The processing unit 300 may include a plurality of the connection ports 3007.

The processing unit 300 may obtain analog signals from a plurality of the measurement units 400 via a plurality of the connection ports 3007.

Each analog signal transmitted from the measurement unit 400 via the connection cable 4202 is converted into a digital signal by the A/D converter 3008 of the processing unit 300. As described with reference to FIG. 2, for example, the A/D converter 3008 samples the analog signal transmitted at a predetermined sampling rate (e.g., sampling at 1024 points at a 10 nanosecond interval, sampling at 128 points at an 80 nanosecond interval, sampling at 64 points at a 160 nanosecond interval, or the like), and generates waveform data for each cell. The waveform data is stored into the storage 3004 or the RAM 3005 via the bus 3003. The waveform data is DMA-transferred to the RAM 3005, for example. The processor 3001 and the parallel-processing processor 3002 execute arithmetic processes on the waveform data stored in the storage 3004 or the RAM 3005.

The analysis software 3100, which operates on the processor 3001, has functions similar to those of the analysis software 4832 shown in FIG. 16. The processor 3001 executes the analysis software 3100, thereby generating classification information regarding the cell type of each measured cell, through operations similar to those described with reference to FIG. 16, FIG. 17, FIG. 18, FIG. 21, and FIG. 22 and in related description thereof.

In the case of the cell analyzer 4000 of configuration example 2 shown in FIG. 23 and FIG. 24, in the flowchart shown in FIG. 20, step S13 (digital signal generation) and step S14 (cell classification) are performed in the processing unit 300. Step S15 (transmission of classification information) is omitted. The processes in FIG. 21 and FIG. 22 are performed by the processor 3001 and the parallel-processing processor 3002 of the processing unit 300.

The processor 3001 can process the waveform data and identification information so as to be associated with each other. Specifically, The processor 3001 can associate an analysis result (i.e., classification information regarding the cell type of each cell) of the waveform data and identification information with each other. Examples of the identification information include: (1) identification information of a biological sample corresponding to the waveform data; (2) identification information of a cell corresponding to the waveform data; (3) identification information of a patient corresponding to the waveform data; (4) identification information of a test corresponding to the waveform data; (5) identification information of a cell analyzer by which the waveform data has been measured; and (6) identification information of a test-related facility where the waveform data has been measured. When the processor 3001 receives a test order from, for example, an LIS, the processor 3001 can obtain at least one of the above identification information (1) to (6) or a combination thereof from the LIS. For example, at least one of (1) to (6) shown as examples is provided to the user as a test result in association with the classification information. A plurality of combinations of (1) to (6) shown as examples may be provided to the user as a test result in association with the classification information.

(Configuration example 3)

With reference to FIG. 26 and FIG. 27, another configuration example of the cell analyzer 4000 composed of the measurement unit 400 and the processing unit 300 will be described. In the present configuration example as well, the parallel-processing processor 3002 is installed, in the cell analyzer 4000, in the form of being incorporated in the processing unit 300.

FIG. 26 shows another example of a block diagram of the measurement unit 400. Except that the measurement unit 400 shown in FIG. 26 is not provided with the processor 4831, the RAM 4834, the storage 4835, and the parallel-processing processor 4833, and is provided with an interface part 4851 and a transmission line 4852 for transmitting the digital signals generated in the A/D converter 482 to the processing unit 300, the measurement unit 400 shown in FIG. 26 has configurations and functions similar to those of the measurement unit 400 described with reference to FIG. 6 and FIG. 7 and in related description thereof. The interface part 4851 is an interface serving as a dedicated line having a communication band of not less than 1 gigabit/second, for example. For example, the interface part 4851 is an interface according to Gigabit Ethernet, USB 3.0, or Thunderbolt 3. When the interface part 4851 is of Gigabit Ethernet, the transmission line 4852 is a LAN cable, for example. When the interface part 4851 is of USB 3.0, the transmission line 4852 is a USB cable according to USB 3.0. The transmission line 4852 is a dedicated transmission line for transmitting the digital signals between the measurement unit 400 and the processing unit 300, for example.

FIG. 27 shows another example of a block diagram of the processing unit 300. Except that the processing unit 300 shown in FIG. 27 is not provided with the A/D converter 3008 and the connection port 3007, and is provided with an interface part 3010, the processing unit 300 shown in FIG. 27 has configurations and functions similar to those of the processing unit 300 described with reference to FIG. 24 and in related description thereof. The processing unit 300 may be connected to a plurality of the measurement units 400 via a plurality of the interface parts 3010 and a plurality of the interface parts 3006.

The processor 3001 and the parallel-processing processor 3002 have configurations and functions similar to those of the processor 3001 and the parallel-processing processor 3002 described with reference to FIG. 25 and in related description thereof. In FIG. 27, the parallel-processing processor 3002 need not necessarily be directly connected to the bus 3003. For example, the parallel-processing processor 3002 may be mounted to a USB device, and this USB device may be connected to the bus 3003 via an interface part (not shown). This USB device may be a small device such as a USB dongle, for example.

The analysis software 3100, which operates on the processor 3001, has functions similar to those of the analysis software 4832 shown in FIG. 16. The analysis software 3100 analyzes the cell type of each measured cell, through operations similar to those described with reference to FIG. 16, FIG. 17, FIG. 18, FIG. 21, and FIG. 22 and in related description thereof.

In the case of the cell analyzer 4000 of configuration example 3 shown in FIG. 26 and FIG. 27, in the flowchart shown in FIG. 20, step S14 (cell classification) is performed in the processing unit 300. Step S15 (transmission of classification information) is omitted. Processes in FIG. 21 and FIG. 22 are performed by the processor 3001 and the parallel-processing processor 3002 of the processing unit 300.

In the configuration in FIG. 26 and FIG. 27, analog signals (forward scattered light signal, side scattered light signal, side fluorescence signal) of each cell generated in the FCM detector 410 are converted into digital signals in the A/D converter 482 in the measurement unit 400. The digital signals are sent to the processing unit 300 via the interface part 484, the bus 485, the interface part 4851, and the transmission line 4852. The transmission line 4852 is a dedicated transmission line for transmitting the digital signals between the measurement unit 400 and the processing unit 300 as described above. For example, the measurement unit 400 and the processing unit 300 are connected in a one-to-one relationship via the transmission line 4852. In other words, the transmission line 4852 is a transmission line that provides no transmission of data related to an apparatus other than components (e.g., the measurement unit 400 and the processing unit 300) forming the cell analyzer 4000, for example. The transmission line 4852 is a transmission line different from an intranet or the Internet, for example. Accordingly, even when digital signals generated through A/D conversion in the measurement unit 400 are transmitted to the processing unit 300, bottleneck in the communication speed of transmission of the digital signals can be avoided.

(Configuration example 4)

With reference to FIG. 28, FIG. 29, FIG. 30, FIG. 31, and FIG. 32, another configuration example of the cell analyzer 4000 will be described.

In the present configuration example, as shown as an example in FIG. 28, an analysis unit 600 is provided between the measurement unit 400 and the processing unit 300. That is, in the configuration in FIG. 28, FIG. 29, FIG. 30, FIG. 31, and FIG. 32, the cell analyzer 4000 is composed of the measurement unit 400, the processing unit 300, and the analysis unit 600. The analysis unit 600 analyzes the cell type of each measured cell. As described later, in the present configuration example, a parallel-processing processor 6002 is installed, in the cell analyzer 4000, in the form of being incorporated in the analysis unit 600.

The measurement unit 400 shown as an example in FIG. 29 has configurations and functions similar to those of the measurement unit 400 described with reference to FIG. 26 and in related description thereof. The analysis unit 600 is provided between the measurement unit 400 and the processing unit 300. The analysis unit 600 may be connected to a plurality of the measurement units 400. The analysis unit 600 may be connected to a plurality of the processing units 300. The interface part 4851 is an interface having a communication band of not less than 1 gigabit/second, for example. For example, the interface part 4851 is an interface according to Gigabit Ethernet, USB 3.0, or Thunderbolt 3. When the interface part 4851 is of Gigabit Ethernet, the transmission line 4852 is a LAN cable, for example. When the interface part 4851 is of USB 3.0, the transmission line 4852 is a USB cable according to USB 3.0. The transmission line 4852 is a dedicated transmission line for transmitting the digital signals between the measurement unit 400 and the processing unit 300 as described above. For example, the measurement unit 400 and the processing unit 300 are connected in a one-to-one relationship via the transmission line 4852.

FIG. 30 shows a configuration example of the analysis unit 600. The analysis unit 600 includes a processor 6001, the parallel-processing processor 6002, a bus 6003, a storage 6004, a RAM 6005, an interface part 6006, and an interface part 6007, for example, and these are connected to the bus 6003. The bus 6003 is a transmission line having a data transfer rate of not less than several hundred MB/s, for example. The bus 3003 may be a transmission line having a data transfer rate of not less than 1 GB/s. The bus 3003 performs data transfer on the basis of the PCI-Express or PCI-X standard, for example. The analysis unit 600 may be connected to a plurality of the measurement units 400 via a plurality of the interface parts 6006. When a plurality of the measurement units 400 are provided, an analysis unit 600 may be connected to each of the measurement units 400 (e.g., a plurality of the measurement units 400 and a plurality of the analysis units 600 are connected in a one-to-one relationship, respectively).

As shown in FIG. 31, the processor 6001 and the parallel-processing processor 6002 have configurations and functions similar to those of the processor 4831 and the parallel-processing processor 4833 described above. The parallel-processing processor 6002 includes a plurality of arithmetic units 6200 and a RAM 6201. Analysis software 6100, which analyzes the cell type of each measured cell, operates on the processor 6001. The analysis software 6100, which operates on the processor 6001, has functions similar to those of the analysis software 4832 shown in FIG. 16. The analysis software 6100 analyzes the cell type of each measured cell through operations similar to those described with reference to FIG. 16, FIG. 17, FIG. 18, FIG. 21, and FIG. 22 and in related description thereof. The analysis software 6100 transmits classification information of each measured cell to the processing unit 300 via the interface part 6007. The interface part 6007 is of Ethernet (registered trademark) or USB, for example. The interface part 6007 may be an interface capable of performing wireless communication (e.g., WiFi (registered trademark) or Bluetooth (registered trademark)).

FIG. 32 shows a configuration example of the processing unit 300. The processing unit 300 shown in FIG. 32 need not necessarily include the parallel-processing processor 3002, unlike the processing unit 300 shown in FIG. 24 and FIG. 27. In addition, the analysis software 3100 shown in FIG. 24 and FIG. 27 need not necessarily operate on the processor 3001 shown in FIG. 32. The processing unit 300 receives analysis results from the analysis unit 600 via the interface part 3006. The interface part 3006 is of Ethernet or USB, for example. The interface part 3006 may be an interface capable of performing wireless communication (e.g., WiFi or Bluetooth).

In the configuration in FIG. 29, FIG. 30, FIG. 31, and FIG. 32, analog signals (forward scattered light signal, side scattered light signal, side fluorescence signal) of each cell generated in the FCM detector 410 are converted into digital signals by the A/D converter 482 in the measurement unit 400. Waveform data is sent to the analysis unit 600 via the interface part 484, the bus 485, the interface part 4851, and the transmission line 4852. The interface part 4851 is a dedicated interface that connects the measurement unit 400 and the analysis unit 600 to each other as described above, and the interface part 4851 connects the measurement unit 400 and the analysis unit 600 in a one-to-one relationship. In other words, the transmission line 4852 is a transmission line that provides no transmission of data related to an apparatus other than components (e.g., the measurement unit 400 and the processing unit 300) forming the cell analyzer 4000, for example. The transmission line 4852 is a transmission line different from an intranet or the Internet, for example. Accordingly, even when digital signals generated through A/D conversion in the measurement unit 400 are transmitted to the processing unit 300, bottleneck in the communication speed of transmission of the digital signals can be avoided.

In the case of the cell analyzer 4000 of configuration example 4 shown in FIG. 29, FIG. 30, FIG. 31, and FIG. 32, in the flowchart shown in FIG. 20, step S14 (cell classification) and step S15 (transmission of classification information) are performed in the analysis unit 600. That is, the digital signals generated in step S13 are transmitted from the measurement unit 400 to the analysis unit 600, and classification information is transmitted to the processing unit 300 in step S15. The processes in FIG. 21 and FIG. 22 are performed by the processor 6001 and the parallel-processing processor 6002 of the analysis unit 600.

(Configuration example 5)

With reference to FIG. 28, FIG. 33, and FIG. 34, another configuration example of the cell analyzer 4000 will be described. Similar to configuration example 4 described above, the cell analyzer of this configuration example 5 also includes the measurement unit 400, the processing unit 300, and the analysis unit 600.

The measurement unit 400 shown in FIG. 33 has functions and configurations similar to those of the measurement unit 400 described with reference to FIG. 23 and in related description thereof. The measurement unit 400 shown in FIG. 33 is connected to the analysis unit 600 via the connection cable 4202. For example, the connection cable 4202 is implemented by twisted-pair cables, and has pairs of wires, the number of the pairs corresponding to the kinds of analog signals transmitted to the processing unit 300. The connection cable 4202 is formed so as to have a length of, for example, 1 meter or less in order to reduce noise during signal transmission. The measurement unit 400 transmits the analog signals to the analysis unit 600 via the connection cable 4202.

The analysis unit 600 shown in FIG. 34 has functions and configurations similar to those of the analysis unit 600 described with reference to FIG. 30 and in related description thereof. That is, in the example in FIG. 34, the parallel-processing processor 6002 is installed, in the cell analyzer 4000, in the form of being incorporated in the analysis unit 600. The analysis unit 600 shown in FIG. 34 further includes a connection port 6008 and an A/D converter 6009. The analog signals transmitted from the analysis unit 600 via the connection cable 4202 are inputted to the A/D converter 6009 via the connection port 6008. The A/D converter 6009 converts the analog signals into digital signals through a process similar to that performed by the A/D converter 482.

The analysis unit 600 may be connected to a plurality of the measurement units 400 via a plurality of the connection ports 6008. When a plurality of the measurement units 400 are provided, an analysis unit 600 may be connected to each of the measurement units 400 (e.g., a plurality of the measurement units 400 and a plurality of the analysis units 600 are connected in a one-to-one relationship, respectively).

As shown in FIG. 31, the processor 6001 and the parallel-processing processor 6002 have configurations and functions similar to those of the processor 4831 and the parallel-processing processor 4833 described above. The analysis software 6100, which analyzes the cell type of each measured cell, operates on the processor 6001. The analysis software 6100, which operates on the processor 6001, has functions similar to those of the analysis software 4832 shown in FIG. 16. The analysis software 6100 analyzes the cell type of each measured cell through operations similar to those described with reference to FIG. 16, FIG. 17, FIG. 18, FIG. 21, and FIG. 22 and in related description thereof. The analysis software 6100 transmits an analysis result of the cell type of each measured cell to the processing unit 300 via the interface part 6007. The interface part 6007 is of Ethernet or USB, for example. The interface part 6007 may be an interface capable of performing wireless communication (e.g., WiFi or Bluetooth).

In the case of the cell analyzer 4000 of configuration example 5 shown in FIG. 33 and FIG. 34, in the flowchart shown in FIG. 20, step S13 (generation of digital signal), step S14 (cell classification), and step S15 (transmission of classification information) are performed in the analysis unit 600. That is, generation of digital signals (step S13) is performed in the A/D converter 6009 of the analysis unit 600, cell classification (step S14) based on the digital signals is performed by the processor 6001 and the parallel-processing processor 6002, and classification information is transmitted from the analysis unit 600 to the processing unit 300 (step S15). The processes in FIG. 21 and FIG. 22 are performed by the processor 6001 and the parallel-processing processor 6002 of the analysis unit 600.

(Data size of waveform data and digital signal)

Next, the data sizes of the waveform data and the digital signal will be described. In the present embodiment, for example, with respect to each of an analog signal of forward scattered light (FSC), an analog signal of side scattered light (SSC), and an analog signal of side fluorescence (SFL), sampling is performed for each cell at a plurality of time points at a certain interval. Examples of the sampling rate include sampling at 1024 points at a 10 nanosecond interval, sampling at 128 points at an 80 nanosecond interval, sampling at 64 points at a 160 nanosecond interval, and the like. The data amount is 2 bytes per sampling, for example. With respect to each of FSC, SSC, and SFL, data (in the case of the rate of 1024 points, 2 bytes$\times$1024=2048 bytes) of an amount corresponding to the sampling rate is obtained. This data amount is the data amount per cell. In a single measurement, FSC, SSC, and SFL are measured with respect to at least 100 cells, for example. Alternatively, in a single measurement, FSC, SSC, and SFL may be measured with respect to at least 1000 cells, for example. Alternatively, in a single measurement, FSC, SSC, SFL may be measured with respect to about 10000 to about 140000 cells, for example. Therefore, for example, when the number of cells measured in a single measurement is 100000 and the sampling rate is 1024, the data amount of the digital signal of each of FSC, SSC, and SFL is 2 bytes$\times$1024$\times$100000=204,800,000 bytes, and the data amount in total of FSC, SSC, and SFL is 614,400,000 bytes.

Further, FSC, SSC, and SFL are measured for each measurement channel. Therefore, for example, when the number of cells measured in a single measurement is 100000, the sampling rate is 1024, and the number of measurement channels is 5, the data amount of each of FSC, SSC, and SFL is 2 bytes$\times$1024$\times$ 100000$\times$5=1,024,000,000 bytes, and the data amount in total of FSC, SSC, and SFL is 3,072,000,000 bytes.

Thus, the volume of each digital signal is several hundred megabytes to several gigabytes per specimen, for example, and is at least 1 gigabyte depending on the number of cells, the sampling rate, and the number of measurement channels.

According to the present embodiment, when the digital signal having a huge volume of several hundred megabytes to several gigabytes per specimen is analyzed, the analysis process using the deep learning algorithm 60 is concluded inside the cell analyzer 4000 or 4000' as described above, and the digital signal is not transmitted to an analysis server provided outside the cell analyzer 4000 or 4000', via the Internet or an intranet. Therefore, decrease in the throughput associated with increase in communication load caused by transmission of the digital signal from the cell analyzer 4000 or 4000' to an analysis server can be avoided.

<Second cell analyzer and measurement of biological sample in the second cell analyzer>

As a configuration example of the second cell analyzer 4000', an example of a block diagram of a urine particle analyzer or a body fluid analyzer in which the measurement unit 500 includes a flow cytometer for measuring a urine sample or a body fluid sample is shown.

FIG. 35 is an example of a block diagram of the measurement unit 500. In FIG. 35, The measurement unit 500 includes: a specimen distribution part 501; a sample preparation part 502; an optical detector 505; an amplification circuit 550 which amplifies an output signal (an output signal amplified by a preamplifier) of the optical detector 505; a filter circuit 506 which performs a filtering process on the output signal from the amplification circuit 550, an A/D converter 507 which converts an output signal (analog signal) of the filter circuit 506 into a digital signal; the processor 4831; the parallel-processing processor 4833; the RAM 4834; the bus controller 4850; a storage device 511a; and a LAN (Local Area Network) adaptor 512. The processor 4831, the parallel-processing processor 4833, the RAM 4834, the bus controller 4850, the storage device 511a, and the LAN adaptor 512 are connected to a bus 508. The deep learning algorithm 60 and analysis software based on the deep learning algorithm 60 are stored in the storage device 511a.

As described on the basis of the example of the first cell analyzer 4000, the processor 4831 performs predetermined arithmetic processes on the digital signal outputted from the A/D converter 507. The processor 4831 analyzes waveform data by using the parallel-processing processor 4833. The configuration examples of the processor 4831 and the parallel-processing processor 4833 and the operation of the parallel-processing processor 4833 are similar to those described above with reference to FIG. 11 to FIG. 19 and in related description thereof.

The processing unit 300 is connected to the measurement unit 500 by a LAN cable via the LAN adaptor 512, for example, and by this processing unit 300, generation of a test result based on measurement data obtained by the measurement unit 500 is performed. The optical detector 505, the amplification circuit 550, the filter circuit 506, the A/D converter 507, the processor 4831, the parallel-processing processor 4833, and the storage device 511a form an optical measurement part 510 which measures a measurement sample and generates measurement data.

FIG. 36 shows a configuration of the optical detector 505 of the measurement unit 500. In FIG. 36, a condenser lens 552 condenses, to a flow cell 551, laser light emitted from a semiconductor laser light source 553 serving as a light source, and a condenser lens 554 condenses, to a forward scattered light receiving part 555, forward scattered light emitted from a particle in a measurement sample. Another condenser lens 556 condenses, to a dichroic mirror 557, side scattered light and fluorescence emitted from the particle. The dichroic mirror 557 reflects the side scattered light to a side scattered light receiving part 558, and allows the fluorescence to pass therethrough toward a fluorescence receiving part 559. These light signals reflect characteristics of the particle in the measurement sample. The forward scattered light receiving part 555, the side scattered light receiving part 558, and the fluorescence receiving part 559 convert the light signals into electric signals, and output a forward scattered light signal, a side scattered light signal, and a fluorescence signal, respectively. These outputs are amplified by a preamplifier, and then subjected to the subsequent processing. As for each of the forward scattered light receiving part 555, the side scattered light receiving part 558, and the fluorescence receiving part 559, a low sensitivity output and a high sensitivity output can be switched through switching of the drive voltage. Switching of these sensitivities is performed by the processor 4831 described later. In the present embodiment, a photodiode may be used as the forward scattered light receiving part 555, photomultiplier tubes may be used as the side scattered light receiving part 558 and fluorescence receiving part 55, or photodiodes may be used as the side scattered light receiving part 558 and the fluorescence receiving part 559. The fluorescence signal outputted from the fluorescence receiving part 559 is amplified by a preamplifier, and then provided to branched two signal channels. The two signal channels are connected to the amplification circuit 550 described above with reference to FIG. 35. The fluorescence inputted to one of the signal channels is amplified by the amplification circuit 550 so as to have a high sensitivity.

(Preparation of measurement sample)

FIG. 37 is a schematic diagram showing a function configuration of the sample preparation part 502 and the optical detector 505 shown in FIG. 35. The specimen distribution part 501 shown in FIG. 35 and FIG. 37 includes a suction tube 517 and a syringe pump. The specimen distribution part 501 suctions a specimen (urine or body fluid) 00b via the suction tube 517, and dispenses the specimen into the sample preparation part 502. The sample preparation part 502 includes a reaction chamber 512u and a reaction chamber 512b. The specimen distribution part 501 distributes a quantified measurement sample to each of the reaction chamber 512u and the reaction chamber 512b.

In the reaction chamber 512u, the distributed biological sample is mixed with a first reagent 519u as a diluent and a third reagent 518u that contains a dye. Due to the dye contained in the third reagent 518u, particles in the specimen are stained. When the biological sample is urine, the sample prepared in the reaction chamber 512u is used as a first measurement sample for analyzing urine particles that are relatively large, such as red blood cells, white blood cells, epithelial cells, or tumor cells. When the biological sample is body fluid, the sample prepared in the reaction chamber 512u is used as a third measurement sample for analyzing red blood cells in the body fluid.

Meanwhile, in the reaction chamber 512b, the distributed biological sample is mixed with a second reagent 519b as a diluent and a fourth reagent 518b that contains a dye. As described later, the second reagent 519b has a hemolytic action. Due to the dye contained in the fourth reagent 518b, particles in the specimen are stained. When the biological sample is urine, the sample prepared in the reaction chamber 512b serves as a second measurement sample for analyzing bacteria in the urine. When the biological sample is body fluid, the sample prepared in the reaction chamber 512b serves as a fourth measurement sample for analyzing nucleated cells (white blood cells and large cells) and bacteria in the body fluid.

A tube extends from the reaction chamber 512u to the flow cell 551 of the optical detector 505, whereby the measurement sample prepared in the reaction chamber 512u can be supplied to the flow cell 551. A solenoid valve 521u is provided at the outlet of the reaction chamber 512u. A tube also extends from the reaction chamber 512b, and this tube is connected to a portion of the tube extending from the reaction chamber 2u. Accordingly, the measurement sample prepared in the reaction chamber 512b can be supplied to the flow cell 551. A solenoid valve 521b is provided at the outlet of the reaction chamber 512u.

The tube extended from the reaction chambers 512u, 512b to the flow cell 551 is branched before the flow cell 551, and a branched tube is connected to a syringe pump 520a. A solenoid valve 521c is provided between the syringe pump 520a and the branch point.

Between the connection point of the tubes extending from the respective reaction chambers 512u, 512b and the branch point, the tube is further branched, and a branched tube is connected to a syringe pump 520b. Between the branch point of the tube extending to the syringe pump 520b and the connection point, a solenoid valve 521d is provided.

The sample preparation part 502 has connected thereto a sheath liquid storing part 522 which stores a sheath liquid, and the sheath liquid storing part 522 is connected to the flow cell 551 by a tube. The sheath liquid storing part 522 has connected thereto a compressor 522a, and when the compressor 522a is driven, compressed air is supplied to the sheath liquid storing part 522, and the sheath liquid is supplied from the sheath liquid storing part 522 to the flow cell 551.

As for the two kinds of suspensions (measurement samples) prepared in the respective reaction chambers 512u, 512b, the suspension (the first measurement sample when the biological sample is urine; the third measurement sample when the biological sample is body fluid) in the reaction chamber 512u is led to the optical detector 505, to form a thin flow enveloped by the sheath liquid in the flow cell 551, and laser light is applied to the thin flow. Then, in a similar manner, the suspension (the second measurement sample when the biological sample is urine; the fourth measurement sample when the biological sample is body fluid) in the reaction chamber 512b is led to the optical detector 505, to form a thin flow in the flow cell 551, and laser light is applied to the thin flow. Such operations are automatically performed by causing the solenoid valves 521a, 521b, 521c, 521d, a drive part 503, and the like to operate by control of the processor 4831 (controller).

The first reagent to the fourth reagent will be described in detail. The first reagent 519u is a reagent having a buffer as a main component, contains an osmotic pressure compensation agent so as to allow obtainment of a stable fluorescence signal without hemolyzing red blood cells, and is adjusted to have 100 to 600 mOsm/kg so as to realize an osmotic pressure suitable for classification measurement. Preferably, the first reagent 519u does not have a hemolytic action on red blood cells in urine.

Different from the first reagent 519u, the second reagent 519b has a hemolytic action. This is for facilitating passage of the later-described fourth reagent 518b through cell membranes of bacteria so as to promote staining. Further, this is also for contracting contaminants such as mucus fibers and red blood cell fragments. The second reagent 519b contains a surfactant in order to acquire a hemolytic action. As the surfactant, a variety of anionic, nonionic, and cationic surfactants can be used, but a cationic surfactant is particularly suitable. Since the surfactant can damage the cell membranes of bacteria, nucleic acids of bacteria can be efficiently stained by the dye contained in the fourth reagent 518b. As a result, bacteria measurement can be performed through a short-time staining process.

As still another embodiment, the second reagent 519b may acquire a hemolytic action not by a surfactant but by being adjusted to be acidic or to have a low pH. Having a low pH means that the pH is lower than that of the first reagent 19u. When the first reagent 519u is in a range of neutral or weakly acidic to weakly alkaline, the second reagent 19b is acidic or strongly acidic. When the pH of the first reagent 519u is 6.0 to 8.0, the pH of the second reagent 519b is lower than that, and is preferably 2.0 to 6.0.

The second reagent 519b may contain a surfactant and be adjusted to have a low pH.

As still another embodiment, the second reagent 519b may acquire a hemolytic action by having a lower osmotic pressure than the first reagent 19u.

Meanwhile, the first reagent 519u does not contain any surfactant. In another embodiment, the first reagent 519u may contain a surfactant, but the kind and concentration thereof need to be adjusted so as not to hemolyze red blood cells. Therefore, preferably, the first reagent 519u does not contain the same surfactant as that of the second reagent 519b, or even if the first reagent 519u contains the same surfactant as that of the second reagent 519b, the concentration of the surfactant in the first reagent 519u is lower than that in the second reagent 519b.

The third reagent 518u is a staining reagent for use in measurement of urine particles (red blood cells, white blood cells, epithelial cells, casts, or the like). As the dye contained in the third reagent 518u, a dye that stains membranes is selected in order to also stain particles that do not have nucleic acids. Preferably, the third reagent 518u contains an osmotic pressure compensation agent for the purpose of preventing hemolysis of red blood cells and for the purpose of obtaining a stable fluorescence intensity, and is adjusted to have 100 to 600 mOsm/kg so as to realize an osmotic pressure suitable for classification measurement. The cell membrane and nucleus (membrane) of urine particles are stained by the third reagent 18u. As the staining reagent containing a dye that stains membranes, a condensed benzene derivative is used, and a cyanine-based dye can be used, for example. The third reagent 18u stains not only cell membranes but also nuclear membranes. When the third reagent 518u is used in nucleated cells such as white blood cells and epithelial cells, the staining intensity in the cytoplasm (cell membrane) and the staining intensity in the nucleus (nuclear membrane) are combined, whereby the staining intensity becomes higher than in the urine particles that do not have nucleic acids. Accordingly, nucleated cells such as white blood cells and epithelial cells can be discriminated from urine particles that do not have nucleic acids such as red blood cells. As the third reagent, the reagents described in US Patent Publication No. 5891733 can be used. US Patent Publication No. 5891733 is incorporated herein by reference. The third reagent 518u is mixed with urine or body fluid, together with the first reagent 519u.

The fourth reagent 518b is a staining reagent that can accurately measure bacteria even when the specimen contains contaminants having sizes equivalent to those of bacteria and fungi. The fourth reagent 518b is described in detail in EP Patent Application Publication No. 1136563. As the dye contained in the fourth reagent 518b, a dye that stains nucleic acids is suitably used. As the staining reagent containing a dye that stains nuclei, the cyanine-based dye of US Patent No. 7309581 can be used, for example. The fourth reagent 518b is mixed with urine or a specimen, together with the second reagent 519b. EP Patent Application Publication No. 1136563 and US Patent No. 7309581 are incorporated herein by reference.

Therefore, preferably, the third reagent 518u contains a dye that stains cell membranes, whereas the fourth reagent 518b contains a dye that stains nucleic acids. Urine particles may include those that do not have a nucleus, such as red blood cells. Therefore, by the third reagent 518u containing a dye that stains cell membranes, urine particles including those that do not have a nucleus can be detected. Since the second reagent can damage cell membranes of bacteria, nucleic acids of bacteria and fungi can be efficiently stained by the dye contained in the fourth reagent 18b. As a result, bacteria measurement can be performed through a short-time staining process.

[3. First waveform data analysis system]

<Configuration of first waveform data analysis system>

Another embodiment relates to a waveform data analysis system. With reference to FIG. 38, the first waveform data analysis system includes a deep learning apparatus 100A. The vendor-side apparatus 100 operates as the deep learning apparatus 100A. The deep learning apparatus 100A causes the neural network 50 to learn by using training data, and provides the user with the deep learning algorithm 60 trained by the training data. The deep learning algorithm 60 configured as a learned neural network is provided from the deep learning apparatus 100A to the measurement unit 400 through a storage medium 98 or a communication network 99. The measurement unit 400 performs analysis of waveform data of an analysis target cell by using the deep learning algorithm 60 configured as a learned neural network.

The deep learning apparatus 100A is implemented as a general-purpose computer, for example, and performs a deep learning process on the basis of a flowchart described later. The measurement unit 400 performs a waveform data analysis process on the basis of a flowchart described later. The storage medium 98 is a computer-readable non-transitory tangible storage medium such as a DVD-ROM or a USB memory, for example.

The deep learning apparatus 100A is connected to a measurement unit 400a or a measurement unit 500a. The configuration of the measurement unit 400a or the measurement unit 500a is similar to that of the measurement unit 400 or the measurement unit 500 described above. The deep learning apparatus 100A obtains training waveform data obtained by the measurement unit 400a or the measurement unit 500a. The generation method for the training waveform data is as described above.

As shown in FIG. 38, the measurement unit 400a or the measurement unit 500a includes the flow cell 4113, 551. In the measurement unit 400a or the measurement unit 500a, a biological sample is sent to the flow cell 4113, 551. The biological sample supplied to the flow cell 4113, 551 is irradiated with light from the light source 4112, 553, and forward scattered light, side scattered light, and side fluorescence emitted from each cell in the biological sample are detected by the light detectors (4116, 4121, 4122, 555, 558, 559). The measurement unit 400a or the measurement unit 500a generates waveform data from a forward scattered light signal, a side scattered light signal, and a side fluorescence signal obtained through detection of light by the light detectors (4116, 4121, 4122, 555, 558, 559), and transmits the waveform data to the vendor-side apparatus 100.

<Hardware configuration of deep learning apparatus>

FIG. 39 shows an example of a block diagram of the vendor-side apparatus 100 (the deep learning apparatus 100A). The vendor-side apparatus 100 includes a processing part 10 (10A), an input part 16, and an output part 17.

The processing part 10 includes for example: a CPU 11 which performs data processing described later, a memory 12 to be used as a work area for data processing; a storage 13 which stores a program and processing data described later; a bus 14 which transmits data between parts; an interface part (I/F part) 15 which performs input/output of data with respect to an external apparatus; and a GPU 19. The input part 16 and the output part 17 are connected to the processing part 10 via the interface part 15. For example, the input part 16 is an input device such as a keyboard or a mouse, and the output part 17 is a display device such as a liquid crystal display. The GPU 19 functions as an accelerator that assists arithmetic processes (e.g., parallel arithmetic processes) performed by the CPU 11. That is, in the description below, the processes performed by the CPU 11 also include processes performed by the CPU 11 using the GPU 19 as an accelerator. The GPU 19 has a function equivalent to that of the parallel-processing processor 4833 described above. Here, instead of the GPU 19, a chip that is suitable for calculation in a neural network may be installed. Examples of such a chip include FPGA, ASIC, and Myriad X (Intel).

In order to perform the process of each step to be described below with reference to FIG. 43, the processing part 10 has previously stored, in the storage 13, a program and the deep learning algorithm 50 configured as a neural network before being trained according to the present embodiment, in an executable form, for example. The executable form is a form generated through conversion of a programming language by a compiler, for example. The processing part 10 uses the program stored in the storage 13, to perform training processes on the neural network 50 before being trained.

In the description below, unless otherwise specified, the processes performed by the processing part 10 mean processes performed by the CPU 11 on the basis of the program and the neural network 50 stored in the storage 13 or the memory 12. The CPU 11 temporarily stores necessary data (intermediate data being processed, etc.) using the memory 12 as a work area, and stores, as appropriate in the storage 13, data to be saved for a long time such as arithmetic results.

<Hardware configuration of analyzer>

The configuration of the measurement unit 400 or 500 which processes waveform data on the basis of the algorithm provided from the vendor-side apparatus 100 is equivalent to the configuration described above. The measurement unit 400 or 500 may also have the function of the vendor-side apparatus 100 (the deep learning apparatus 100A) to cause the neural network 50 to learn by using training data. In this case, the vendor-side apparatus 100 (the deep learning apparatus 100A) is not necessary.

In order to perform the process of each step in the waveform data analysis process described below, the measurement unit controller 480 has previously stored, in the storage 4835, a program and the deep learning algorithm 60 configured as a trained neural network according to the present embodiment, in an executable form, for example. The executable form is a form generated through conversion of a programming language by a compiler, for example. The measurement unit controller 480 uses the program and the deep learning algorithm 60 stored in the storage 4835, to perform processes.

As shown in FIG. 40, the measurement unit controller 480 may update the program and the deep learning algorithm 60 stored in the storage 4835, though a communication network via a LAN adaptor 481, for example.

As shown in FIG. 41, the analysis unit 600 described above may transmit digital signals and analysis results received from the measurement unit 400 to the deep learning apparatus 100A via an interface part 6011. For example, the analysis unit 600 transmits waveform data and classification information corresponding to the waveform data, to the deep learning apparatus 100A via the Internet. The deep learning apparatus 100A performs a learning process on the basis of the waveform data and the classification information corresponding to the waveform data transmitted from the analysis unit 600, to update the deep learning algorithm 60. The deep learning apparatus 100A transmits the updated deep learning algorithm 60 to the analysis unit 600. The analysis unit 600 updates the algorithm stored in the storage 6004 by the deep learning algorithm 60 transmitted from the deep learning apparatus 100A. For example, while the processor 6001 and the parallel-processing processor 6002 are processing waveform data transmitted from the measurement unit 400, the analysis unit 600 transmits, in parallel therewith, the waveform data to the deep learning apparatus 100A. Upon completion of analysis of the waveform data, i.e., upon completion of generation of classification information, the analysis unit 600 transmits the classification information to the deep learning apparatus 100A.

In the description below, unless otherwise specified, processes performed by the measurement unit controller 480 mean, in actuality, processes performed by the processor 4831 and the parallel-processing processor 4833 on the basis of the program and the deep learning algorithm 60 stored in the storage 4835 or the RAM 4834. The processor 4831 temporarily stores necessary data (such as intermediate data being processed, measurement results, analysis results, etc.) using the RAM 4834 as a work area, for example, and stores, as appropriate in the storage 4835, data to be saved for a long time such as arithmetic results.

<Function block and processing procedure>

(Deep learning process)

FIG. 42 shows an example of a function block of the deep learning apparatus 100A which performs deep learning. With reference to FIG. 42, a processing part 10A of the deep learning apparatus 100A according to the present embodiment includes a training data generation part 101, a training data input part 102, and an algorithm update part 103. These function blocks are realized when: a program for causing a computer to execute the deep learning process is installed in the storage 13 or the memory 12 of the processing part 10A shown in FIG. 39; and the program is executed by the CPU 11 and the GPU 19. A training data database (DB) 104 and an algorithm database (DB) 105 are stored in the storage 13 or the memory 12 of the processing part 10A.

Training waveform data 76a, 76b, 76c is obtained in advance by the measurement unit 400, 500, for example, and is stored in advance in the storage 13 or the memory 12 of the processing part 10A. The deep learning algorithm 50 is stored in the algorithm database 105 in advance.

The processing part 10A of the deep learning apparatus 100A performs the processes shown in FIG. 43. With reference to the function blocks shown in FIG. 42, the processes of steps S211, S214, and S216 shown in FIG. 43 are performed by the training data generation part 101. The process of step S212 is performed by the training data input part 102. The processes of steps S213 and S215 are performed by the algorithm update part 103.

With reference to FIG. 43, an example of the deep learning process performed by the processing part 10A will be described. First, the processing part 10A obtains the training waveform data 72a, 72b, 72c. The training waveform data 72a is waveform data of forward scattered light, the training waveform data 72b is waveform data of side scattered light, and the training waveform data 72c is waveform data of side fluorescence. The training waveform data 72a, 72b, 72c is obtained, for example, through operation by an operator, from the measurement unit 400, 500, from the storage medium 98, or via the I/F part 15 through a communication network. When the training waveform data 72a, 72b, 72c is obtained, information regarding which cell type the training waveform data 72a, 72b, 72c indicates is also obtained. The information regarding which cell type is indicated may be associated with the training waveform data 72a, 72b, 72c, or may be inputted by the operator through the input part 16.

In step S211, the processing part 10A generates the training data 75 from the waveform data 72a, 72b, 72c and the label value 77 having been obtained.

In step S212, the processing part 10A inputs the training data 75 to the neural network 50, to obtain a trial result. The trial result is accumulated every time each of a plurality of pieces of the training data 75 is inputted to the neural network 50.

In the cell type analysis method according to the present embodiment, a convolutional neural network is used, and a stochastic gradient descent method is used. Therefore, in step S213, the processing part 10A determines whether or not trial results of a previously set predetermined number of times of trials have been accumulated. When the trial results of the predetermined number of times of trials have been accumulated (YES), the processing part 10A advances to the process of step S214. When the trial results of the predetermined number of times of trials have not been accumulated (NO), the processing part 10A advances to the process step S215.

Next, when the trial results of the predetermined number of times of trials have been accumulated, the processing part 10A updates, in step S214, connection weight w of the neural network 50, by using the training results accumulated in step S212. In the cell type analysis method according to the present embodiment, since the stochastic gradient descent method is used, the connection weight w of the neural network 50 is updated at the stage where the trial results of the predetermined number of times of trials have been accumulated. Specifically, the process of updating the connection weight w is a process of performing calculation according to the gradient descent method, represented by Formula 12 and Formula 13 described later.

In step S215, the processing part 10A determines whether or not the neural network 50 has been trained using a prescribed number of pieces of training data 75. When the training has been performed using the prescribed number of pieces of training data 75 (YES), the deep learning process ends.

When the neural network 50 has not been trained using the prescribed number of pieces of training data 75 (NO), the processing part 10A advances from step S215 to step S216, and performs the processes from step S211 to step S215 with respect to the next training waveform data.

In accordance with the processes described above, the neural network 50 is trained, whereby the deep learning algorithm 60 is obtained.

(Structure of neural network)

As described above, a convolutional neural network is used in the present embodiment. (a) of FIG. 44 shows an example of the structure of the neural network 50. The neural network 50 includes the input layer 50a, the output layer 50b, and the middle layer 50c between the input layer 50a and the output layer 50b. The middle layer 50c is composed of a plurality of layers. The number of layers forming the middle layer 50c can be, for example, 5 or greater, preferably 50 or greater, and more preferably 100 or greater.

In the neural network 50, a plurality of nodes 89 arranged in a layered manner are connected between the layers. Accordingly, information is propagated only in one direction indicated by an arrow D in the drawing, from the input-side layer 50a to the output-side layer 50b.

(Arithmetic operation at each node)

(b) of FIG. 44 is a schematic diagram showing arithmetic operations performed at each node. Each node 89 receives a plurality of inputs, and calculates one output (z). In the case of the example shown in (b) of FIG. 44, the node 89 receives four inputs. The total input (u) received by the node 89 is represented by Formula 2 below as an example. In the present embodiment, one-dimensional matrix data is used as the training data 75 and the analysis data 85. Therefore, when variables of the arithmetic expression correspond to two-dimensional matrix data, a process of converting the variables so as to correspond to one-dimensional matrix data is performed.

[Math 2]

$$u = w_1 x_1 + w_2 x_2 + w_3 x_3 + w_4 x_4 + b \qquad \text{(Formula 2)}$$

Each input is multiplied by a different weight. In Formula 2, b is a value referred to as bias. The output (z) of the node serves as an output of a predetermined function f with respect to the total input (u) represented by Formula 2, and is represented by Formula 3 below. The function f is referred to as an activation function.

[Math 3]

$$z = f(u) \qquad \text{(Formula 3)}$$

(c) of FIG. 44 is a schematic diagram showing arithmetic operations between nodes. In the neural network 50, nodes that each output a result (z) represented by Formula 3, with respect to the total input (u) to the node 89 represented by Formula 2, are arranged in a layered manner. The outputs of the nodes of the previous layer serve as inputs to nodes of the next layer. In the example shown in (c) of FIG. 44, the outputs of node 89a in the left layer serve as inputs to nodes 89b in the right layer. Each node 89b receives outputs from the nodes 89a. The connection between each node 89a and each node 89b is multiplied by a different weight. When the respective outputs from the plurality of nodes 89a are defined as x1 to x4, the inputs to the respective three nodes 89b are represented by Formula 4-1 to Formula 4-3 below.

[Math 4]

$$u_1 = w_{11}x_1 + w_{12}x_2 + w_{13}x_3 + w_{14}x_4 + b_1 \quad \text{(Formula 4-1)}$$

$$u_2 = w_{21}x_1 + w_{22}x_2 + w_{23}x_3 + w_{24}x_4 + b_2 \quad \text{(Formula 4-2)}$$

$$u_3 = w_{31}x_1 + w_{32}x_2 + w_{33}x_3 + w_{34}x_4 + b_3 \quad \text{(Formula 4-3)}$$

When Formula 4-1 to Formula 4-3 are generalized, Formula 4-4 is obtained. Here, i=1, ..., I, j=1, ..., J (I is the total number of inputs, and J is the total number of outputs).
[Math 5]

$$u_j = \sum_{i=1}^{I} w_{ji} x_i + b_j \quad \text{(Formula 4-4)}$$

When Formula 4-4 is applied to the activation function, an output is obtained. The output is represented by Formula 5 below.
[Math 6]

$$z_j = f(u_j) \quad (j = 1, 2, 3) \quad \text{(Formula 5)}$$

(Activation function)

In the cell type analysis method according to the embodiment, a rectified linear unit function is used as the activation function. The rectified linear unit function is represented by Formula 6 below.
[Math 7]

$$f(u) = \max(u, 0) \quad \text{(Formula 6)}$$

Formula 6 is a function obtained by setting u=0 to the part u<0 in the linear function with z=u. In the example shown in (c) of FIG. 44, using Formula 6, the output from the node of j=1 is represented by the formula below.

[Math 8]

$$z_1 = \max((w_{11}x_1 + w_{12}x_2 + w_{13}x_3 + w_{14}x_4 + b_1), 0)$$

(Neural network learning)

If the function expressed by use of a neural network is defined as y(x:w), the function y(x:w) varies when a parameter w of the neural network is varied. Adjusting the function y(x:w) such that the neural network selects a more suitable parameter w with respect to the input x is referred to as neural network training/learning. It is assumed that a plurality of pairs of the input and an output of the function expressed by use of the neural network have been provided. If a desirable output for an input x is defined as d, the pairs of the input/output are given as { (x1,d1), (x2,d2), ..., (xn,dn)}. The set of pairs each expressed as (x,d) is referred to as training data. Specifically, the set of pieces of waveform data (forward scattered light waveform data, side scattered light waveform data, fluorescence waveform data) shown in FIG. 3 is the training data shown in in FIG. 3.

The neural network learning means adjusting the weight w such that, with respect to any input/output pair (xn,dn), the output y(xn:w) of the neural network when given the input xn becomes close to the output dn as much as possible. An error function is a measure for closeness

[Math 9]

$$y(x_n : w) \approx d_n$$

between the training data and the function expressed by use of the neural network. The error function is also referred to as a loss function. An error function E(w) used in the cell type analysis method according to the embodiment is represented by Formula 7 below. Formula 7 is referred to as cross entropy.

[Math 10]

$$E(w) = -\sum_{n=1}^{N} \sum_{k=1}^{K} d_{nk} \log y_k(x_n; w) \qquad \text{(Formula 7)}$$

A method for calculating the cross entropy of Formula 7 will be described. In the output layer 50b of the neural network 50 used in the cell type analysis method according to the embodiment, i.e., in the last layer of the neural network, an activation function for classifying the input x into a finite number of classes in accordance with the contents, is used. The activation function is referred to as a softmax function, and represented by Formula 8 below. It is assumed that, in the output layer 50b, the nodes are arranged by the same number as the number of classes k. It is assumed that the total input u of each node k (k=1, ..., K) of an output layer L is given as $uk^{(L)}$ from the outputs of the previous layer L-1. Accordingly, the output of the k-th node in the output layer is represented by Formula 8 below.

[Math 11]

$$y_k \equiv z_k^{(L)} = \frac{\exp\left(u_k^{(L)}\right)}{\sum_{j=1}^{K} \exp\left(u_j^{(L)}\right)} \qquad \text{(Formula 8)}$$

Formula 8 is the softmax function. The sum of output y1, ..., yK determined by Formula 8 is always 1.

When each class is expressed as C1, ..., CK, output yK of node k in the output layer L (i.e., $uk^{(L)}$) represents the probability at which the given input x belongs to class CK. See Formula 9 below. The input x is classified into a class in which the probability represented by Formula 9 becomes highest.

[Math 12]

$$p(C_k | x) = y_k = z_k^{(L)} \qquad \text{(Formula 9)}$$

In the neural network learning, a function expressed by the neural network is considered as a model of the posterior probability of each class, and the likelihood of the weight w with respect to the training data is evaluated under such a probability model, and a weight w that maximizes the likelihood is selected.

It is assumed that the target output dn by the softmax function of Formula 8 is 1 only when the output is a correct class, and otherwise, the target output dn is 0. When the target output is expressed in a vector form dn=[dn1, ..., dnK], if, for example, the correct class of input xn is C3, only target output dn3 becomes 1, and the other target outputs become 0. When coding is performed in this manner, the posterior distribution is represented by Formula 10 below.

[Math 13]

$$p(d|x) = \prod_{k=1}^{K} p(C_k | x)^{d_k} \qquad \text{(Formula 10)}$$

Likelihood L(w) of the weight w with respect to the training data {(xn, dn)}(n=1,..., N) is represented by Formula 11 below. When the logarithm of the likelihood L(w) is taken and the sign is inverted, the error function of Formula 7 is derived.
[Math 14]

$$L(w) = \prod_{n=1}^{N} p(d_n|x_n; w) = \prod_{n=1}^{N} \prod_{k=1}^{K} p(C_k|x_n)^{d_{nk}} = \prod_{n=1}^{N} \prod_{k=1}^{K} (y_k(x; w))^{d_{nk}} \quad \text{(Formula 11)}$$

Learning means minimizing the error function E(w) calculated on the basis of the training data, with respect to the parameter w of the neural network. In the cell type analysis method according to the embodiment, the error function E(w) is represented by Formula 7.

Minimizing the error function E(w) with respect to the parameter w has the same meaning as finding a local minimum point of the function E(w). The parameter w is a weight of connection between nodes. The local minimum point of the weight w is obtained by iterative calculation of repeatedly updating the parameter w from an arbitrary initial value used as a starting point. An example of such calculation is the gradient descent method.

In the gradient descent method, a vector represented by Formula 12 below is used.
[Math 15]

$$\nabla E = \frac{\partial E}{\partial \mathbf{w}} = \left[\frac{\partial E}{\partial w_1}, \ldots, \frac{\partial E}{\partial w_M}\right]^T \quad \text{(Formula 12)}$$

In the gradient descent method, a process of moving the value of the current parameter w in the negative gradient direction (i.e., -VE) is repeated many times. When the current weight is $w^{(t)}$ and the weight after the moving is $w^{(t+1)}$, the arithmetic operation according to the gradient descent method is represented by Formula 13 below. The value t means the number of times the parameter w is moved.
[Math 16]

$$\mathbf{w}^{(t+1)} = \mathbf{w}^{(t)} - \epsilon \nabla E \quad \text{(Formula 13)}$$

The symbol

[Math 17]

$$\epsilon$$

is a constant that determines the magnitude of the update amount of the parameter w, and is referred to as a learning coefficient. As a result of repetition of the arithmetic operation represented by Formula 13, an error function $E(w^{(t)})$ decreases in association with increase of the value t, and the parameter w reaches a local minimum point.

It should be noted that the arithmetic operation according to Formula 13 may be performed on all of the training data (n=1, ..., N) or may be performed on only a part of the training data. The gradient descent method performed on only a part the training data is referred to as a stochastic gradient descent method. In the cell type analysis method according to the embodiment, the stochastic gradient descent method is used.

[4. Construction of deep learning model]

Using Sysmex XN-1000, blood collected from a healthy individual was measured as a healthy blood sample, and XN CHECK Lv2 (control blood from Streck (having been subjected to processing such as fixation)) was measured as an unhealthy blood sample. As a fluorescence staining reagent, Fluorocell WDF manufactured by Sysmex Corporation was used. As a hemolytic agent, Lysercell WDF manufactured by Sysmex Corporation was used. For each cell contained in each biological sample, waveform data of forward scattered light, side scattered light, and side fluorescence was obtained at 1024 points at a 10 nanosecond interval from the measurement start of forward scattered light. With respect to the healthy blood sample, waveform data of cells in bloods collected from 8 healthy individuals was pooled in the form of digital signals. With respect to the waveform data of each cell, classification of neutrophil (NEUT), lymphocyte (LYMPH), monocyte (MONO), eosinophil (EO), basophil (BASO), and immature granulocyte (IG) was manually performed, and each piece of waveform data was provided with annotation (labelling) of cell type. The time point at which the signal intensity of forward scattered light exceeded a threshold was defined as the measurement start time point, and the time points of obtainment of pieces of waveform data of forward scattered light, side scattered light, and side fluorescence were synchronized to each other, to generate training data. In addition, the control blood was provided with annotation "control blood-derived cell (CONT)". The training data was inputted to the deep learning algorithm so as to be learned.

With respect to blood cells of another healthy individual different from the healthy individual from whom the learned cell data was obtained, analysis waveform data was obtained by Sysmex XN-1000 in a manner similar to that for training data. Waveform data derived from the control blood was mixed, to create analysis data. This analysis data was inputted to the constructed deep learning algorithm and data of individual cell types was obtained.

FIG. 45 shows the result as a mix matrix. The horizontal axis represents the determination result by the constructed deep learning algorithm, and the vertical axis represents the determination result manually (reference method) obtained by a human. With respect to the determination result by the constructed deep learning algorithm, although slight confusion was observed between basophil and lymphocyte and between basophil and ghost, the determination result by the constructed deep learning algorithm exhibited a matching rate of 98.8% with the determination result by the reference method.

Next, with respect to each cell type, ROC analysis was performed, and sensitivity and specificity were evaluated. (a) of FIG. 46 shows an ROC curve of neutrophil, (b) of FIG. 46 shows an ROC curve of lymphocyte, (c) of FIG. 46 shows an ROC curve of monocyte, (a) of FIG. 47 shows an ROC curve of neutrophil, (b) of FIG. 47 shows an ROC curve of basophil, and (c) of FIG. 47 shows an ROC curve of control blood (CONT). Sensitivity and specificity were, respectively, 99.5% and 99.6% for neutrophil, 99.4% and 99.5% for lymphocyte, 98.5% and 99.9% for monocyte, 97.9% and 99.8% for eosinophil, 71.0% and 81.4% for basophil, and 99.8% and 99.6% for control blood (CONT). These were good results.

From the results above, it has been clarified that cell types can be determined with high classification accuracy by using the deep learning algorithm on the basis of signals obtained from cells contained in a biological sample and on the basis of waveform data.

Further, in some cases where unhealthy blood cells such as of a control blood are mixed with healthy blood cells, it has been difficult for a conventional scattergram to determine the type of each individual cell. However, it has been shown that, when the deep learning algorithm of the present embodiment is used, even when unhealthy blood cells are mixed with healthy blood cells, each individual cell can be determined.

[5. Analysis system using image analyzer]

An embodiment in which an image analyzer is used as a cell analyzer will be described. A third cell analyzer 4000" being an image analyzer analyzes captured image data, thereby estimating the cell type of each cell of which an image has been captured.

FIG. 48 shows a configuration example of the cell analyzer 4000". The cell analyzer 4000" shown in FIG. 48 includes a measurement unit 700 and a processing unit 800, measures a sample 901 prepared through pretreatment by a pretreatment apparatus 900, and performs analysis.

The measurement unit 700 includes a flow cell 710, light sources 720 to 723, condenser lenses 730 to 733, dichroic mirrors 740 to 741, a condenser lens 750, an optical unit 751, a condenser lens 752, and an imaging part 760. A sample 701 is caused to flow in a flow path 711 of the flow cell 710.

The light sources 720 to 723 each apply light to the sample 701 flowing in the flow cell 710. The light sources 720 to 723 are each implemented by a semiconductor laser light source, for example. Lights having wavelengths $\lambda$11 to $\lambda$14 are emitted from the light sources 720 to 723, respectively. The condenser lens 730 to 733 condense lights having the wavelengths $\lambda$11 to $\lambda$14 emitted from the light sources 720 to 723, respectively. The dichroic mirror 740 allows light having the wavelength $\lambda$11 to pass therethrough, and refracts light having the wavelength $\lambda$12. The dichroic mirror 741 allows lights having the wavelength $\lambda$11 and $\lambda$12 to pass therethrough, and refracts light having the wavelength $\lambda$13. In this manner, lights having the wavelengths $\lambda$11 to $\lambda$14 are applied to the sample 701 flowing in the flow path 711 of the flow cell 710. The number of semiconductor laser light sources of the measurement unit 700 is not particularly limited as long as the number is 1 or greater. The number of semiconductor laser light sources can be selected from 1, 2, 3, 4, 5, or 6, for example.

In a case where the sample 701 flowing in the flow cell 710 has been stained by a fluorescent dye, when lights having the wavelengths $\lambda$11 to $\lambda$13 are applied to the sample 701, fluorescence is generated from the fluorescent dye staining each cell. For example, fluorescences having wavelengths $\lambda$21, $\lambda$22, $\lambda$23 respectively corresponding to the wavelengths $\lambda$11, $\lambda$12, $\lambda$13 are generated. When light having the wavelength $\lambda$14 is applied to the sample 701 flowing in the flow cell 710, this light passes through each cell. The transmitted light having the wavelength $\lambda$14 and having passed through the cell is used in generation of a bright field image.

The condenser lens 750 condenses the fluorescences generated from the sample 701 flowing in the flow path 711 of the flow cell 710, and the transmitted light having passed through the sample 701 flowing in the flow path 711 of the flow cell 710. The optical unit 751 has a configuration in which four dichroic mirrors are combined. The four dichroic mirrors of the optical unit 751 reflect the fluorescences and the transmitted light at angles slightly different from each other, to be separated on the light receiving surface of the imaging part 760. The condenser lens 752 condenses the fluorescences and the transmitted light.

The imaging part 760 is implemented by a TDI (Time Delay Integration) camera. The imaging part 760 can capture images of the fluorescences and the transmitted light and output, to the processing unit 800, a fluorescence image corresponding to the fluorescences and a bright field image corresponding to the transmitted light, as imaging signals.

The processing unit 800 includes a processing part 811, a storage 812, an interface part 816, and a bus 815, as a hardware configuration. The processing part 811, the storage 812, and the interface part 816 are connected to the bus 815. Image data (e.g., fluorescence image, bright field image) formed by imaging signals captured by the imaging part 760 of the measurement unit 700 is stored in the storage 812 via the interface part 816. The processing part 811 reads out image data from the storage 812 and analyzes the image data.

FIG. 49 shows a configuration example of the processing part 811. The processing part 811 includes a processor 8111, a parallel-processing processor 8112, and a RAM 8113, for example. The processor 8111, the parallel-processing processor 8112, and the RAM 8113 have configurations and functions similar to those of the processor 4831, the parallel-processing processor 4833, and the RAM 4834 described above, respectively. Image data captured by the imaging part 760 is analyzed by the processor 8111 and the parallel-processing processor 8112. The parallel-processing processor 8112 executes, for example, through the processes shown as an example in FIG. 16, FIG. 17, and FIG. 18, arithmetic processes of matrix data regarding the image data in parallel by means of a plurality of arithmetic units.

The processing unit 800 need not necessarily have the functions (the processor 8111, the parallel-processing processor 8112, and the RAM 8113) of the processing part 811, and the measurement unit 700 may have the functions.

<Generation of training data>

Hereinafter, an example of generating training data in the present embodiment will be described.

Preferably, training images to be used for training the deep learning algorithm are captured in RGB colors, CMY colors, or the like. Preferably, as for a color image, the darkness/paleness or brightness of each of primary colors, such as red, green, and blue, or cyan, magenta, and yellow, is expressed by a 24 bit value (8 bits×3 colors). It is sufficient that each training image includes at least one hue, and the darkness/paleness or brightness of the hue, but more preferably, includes at least two hues and the darkness/paleness or brightness of each hue. Information including hue and the darkness/paleness or brightness of the hue is also referred to as tone.

Information of tone of each pixel in the training image is converted from, for example, RGB colors into a format that includes information of brightness and information of hue. Examples of the format that includes information of brightness and information of hue include YUV (YCbCr, YPbPr, YIQ, etc.). Here, an example of conversion to a YCbCr format will be described. A training image captured in RGB colors is converted into image data based on brightness, image data based on a first hue (e.g., bluish color), and image data based on a second hue (e.g., reddish color). Conversion from RGB to YCbCr can be performed by a known method. For example, conversion from RGB to YCbCr can be performed according to the international standard ITU-R BT.601. The image data based on the brightness, the image data based on the first hue, and the image data based on the second hue can be expressed as matrix data of gradation values as shown in FIG. 50 (hereinafter, also referred to as tone matrix data 72y, 72cb, 72cr). The image data based on the brightness, the image data based on the first hue, and the image data based on the second hue can be expressed in 256 gradations consisting of 0 to 255 gradations, for example. Here, instead of the brightness, the first hue, and the second hue, the training image may be converted into the three primary colors of red R, green G, and blue B, or the three primary colors of pigment of cyan C, magenta M, and yellow Y.

Next, on the basis of the tone matrix data 72y, 72cb, 72cr, for each pixel, tone vector data 74 is generated by combining three gradation values of the brightness 72y, the first hue 72cb, and the second hue 72cr.

Next, for example, assuming that an image of a segmented neutrophil has been captured in the training image, each piece of tone vector data 74 generated from the training image is provided with "1" as a label value 77 indicating segmented neutrophil, whereby training data 75 is obtained. In FIG. 50, for convenience, the training data 75 is expressed by 3 pixels ×3 pixels. However, in actuality, tone vector data exists by the number of pixels with which the training image has been captured.

FIG. 51 shows an example of the label value 77. As for the label value, a label value 77 that is different according to the cell type and the presence or absence of a feature of each cell is provided.

<Outline of deep learning>

With reference FIG. 50 used as an example, an outline of training of the neural network will be described. The neural network 50 is preferably a convolutional neural network. The number of nodes of the input layer 50a in the neural network 50 corresponds to the product of the number of pixels in the training data 75 to be inputted, and the number (e.g., in the above example, three, i.e., the brightness 72y, the first hue 72cb, and the second hue 72cr) of brightnesses and hues included in the image. The pieces of tone vector data 74 are inputted, as a set 76 thereof, to the input layer 50a of the neural network 50. The neural network 50 is trained by using, for the output layer 50b of the neural network, the label value 77 of each pixel of the training data 75.

On the basis of the training data 75, the neural network 50 extracts feature quantities with respect to morphological cell types and features of the cell. The output layer 50b of the neural network outputs a result reflecting these feature quantities.

The reference character 50c in FIG. 50 represents the middle layer.

The deep learning algorithm 60 having the thus trained neural network 60 is used as a discriminator for identifying which of a plurality of cell types that belong to a predetermined cell group and that are morphologically classified the analysis target cell corresponds to.

<Image analysis method>

FIG. 52 shows an example of an image analysis method. In the image analysis method, analysis data 81 is generated from an analysis image obtained by capturing an image of an analysis target cell. The analysis image is an image obtained by capturing an image of the analysis target cell.

For example, preferably, in the present embodiment, image capturing by an imaging device is performed in RGB colors, CMY colors, and the like. Preferably, as for a color image, the darkness/paleness or brightness of each of primary colors, such as red, green, and blue, or cyan, magenta, and yellow, is expressed by a 24 bit value (8 bits×3 colors). It is sufficient that the analysis image includes at least one hue, and the darkness/paleness or brightness of the hue, but more preferably, includes at least two hues and the darkness/paleness or brightness of each hue. Information including hue and the darkness/paleness or brightness of the hue is also referred to as tone.

For example, conversion from RGB colors into a format that includes information of brightness and information of hue is performed. Examples of the format that includes information of brightness and information of hue include YUV (YCbCr, YPbPr, YIQ, etc.). Here, an example of conversion to a YCbCr format will be described. A training image in RGB colors is converted into image data based on brightness, image data based on a first hue (e.g., bluish color), and image data based on a second hue (e.g., reddish color). Conversion from RGB to YCbCr can be performed by a known method. For example, conversion from RGB to YCbCr can be performed according to the international standard ITU-R BT.601. The pieces of image data respectively corresponding to the brightness, the first hue, and the second hue can be expressed as matrix data of gradation values as shown in FIG. 52 (hereinafter, also referred to as tone matrix data 79y, 79cb, 79cr). The brightness, the first hue, and the second hue 72Cr can be expressed in 256 gradations consisting of 0 to 255 gradations. Here, instead of the brightness, the first hue, and the second hue, the training image may be converted into the three primary colors of red R, green G, and blue B, or the three primary colors of pigment of cyan C, magenta M, and yellow Y

Next, on the basis of the tone matrix 79y, 79cb, 79cr, for each pixel, tone vector data 80 is generated by combining three gradation values of the brightness 79y, the first hue 79cb, and the second hue 79cr. A set of tone vector data 80 generated from a single analysis image is generated as the analysis data 81.

Preferably, the generation of the analysis data 81 and the generation of the training data 75 are performed at least under the same image capturing condition and the same generation condition of the vector data that is inputted from each image to the neural network.

The analysis data 81 is inputted to the input layer 60a of the neural network 60 forming the trained deep learning algorithm 60. The deep learning algorithm extracts feature quantities from the analysis data 81, and outputs the result from the output layer 60b of the neural network 60. The value outputted from the output layer 60b is the probability at which the analysis target cell included in the analysis image belongs to each of morphological cell classifications and features inputted as training data.

The analysis target cell included in the analysis image is determined to belong to the morphological classification that has the highest value among the probabilities, and a label value associated with the morphological cell type or the feature of the cell is outputted. The label value itself or data obtained by replacing the label value with information (e.g., a term) that indicates the morphological cell type or the presence or absence of the feature of the cell, is outputted as the analysis result 83 regarding the morphology of the cell. In FIG. 52, on the basis of the analysis data 81, a label value "1" is outputted, by the discriminator, as a label value 82 that has the highest possibility, and character data of "segmented neutrophil" corresponding to this label value is outputted as the analysis result 83 regarding the morphology of the cell.

The reference character 60c in FIG. 52 represents the middle layer.

[6. Other embodiments]

Although the outlines and specific embodiments of the present invention have been described, the present invention is not limited to the outlines and embodiments described above.

In the above embodiments, the function blocks of the training data generation part 101, the training data input part 102, the algorithm update part 103, an analysis data generation part 201, an analysis data input part 202, and an analysis part 203 are executed in a single processor 4831 and a single parallel-processing processor 4833. However, these function blocks need not necessarily be executed in a single processor and a single parallel-processing processor, and may be executed in a distributed manner by a plurality of processors and a plurality of parallel-processing processors.

In the above embodiments, a program for performing the process of steps described with reference to FIG. 43 is stored in advance in the storage 4835. Instead of this, the program may be installed to the measurement unit controller 480 from the computer-readable non-transitory tangible storage medium 98 such as a DVD-ROM or a USB memory, for example. Alternatively, the measurement unit controller 480 may be connected to the communication network 99, and the program may be downloaded and installed via the communication network 99 from, for example, an external server (not shown).

FIG. 53 shows an embodiment of the analysis result. FIG. 53 shows: cell types of cells, contained in a biological sample measured by flow cytometry, that are provided with the label values shown in FIG. 4; and the number of cells of each cell type. Instead of the display of the number of cells, or together with the display of the number of cells, the proportion (e.g., %) of each cell type with respect to the total number of cells that have been counted, may be outputted. The count of the number of cells can be obtained by counting the number of label values (the number of the same label values) that correspond to each cell type that has been outputted. In the output result, a warning indicating that abnormal cells are contained in the biological sample may be outputted. FIG. 53 shows an example in which an exclamation mark is provided as a warning in the column of the abnormal cell, but such a warning is not limited thereto. Further, the distribution of each cell type may be plotted as a scattergram, and the scattergram may be outputted. When the scattergram is outputted, for example, the highest values at the time of obtainment of signal intensities may be plotted, with the vertical axis representing the side fluorescence intensity and the horizontal axis representing the side scattered light intensity, for example.

DESCRIPTION OF the REFERENCE CHARACTERS

50 deep learning algorithm before being trained
60 trained deep learning algorithm
400, 400a, 500, 500a, 700 measurement unit
410 FCM detector
450 specimen suction part
482, 507, 3008, 6009 A/D converter
3001, 4831, 6001, 8111 processor (host processor)
3002, 4833, 6002, 8112 parallel-processing processor
4000, 4000', 4000" cell analyzer
3200, 6200, 4836 arithmetic unit

Further embodiments are defined as Embodiment 1 to Embodiment 34, as follows:

[Embodiment 1] A cell analysis method using a cell analyzer that comprises a host processor and a parallel-processing processor, the method comprising:

obtaining, on the basis of control by the host processor, data regarding each of a plurality of cells in a specimen;
executing, by the parallel-processing processor, parallel processing regarding the data; and
generating, on the basis of a result of the parallel processing, information regarding a cell type with respect to each of the plurality of cells.

[Embodiment 2] The cell analysis method according to embodiment 1, wherein
the parallel processing is at least a part of processing executed in accordance with an artificial intelligence algorithm.

[Embodiment 3] The cell analysis method according to embodiment 1 or 2, comprising
transmitting the data to the parallel-processing processor via a transmission line included in the cell analyzer.

[Embodiment 4] The cell analysis method according to embodiment 1 or 2, comprising
transmitting the data to the parallel-processing processor via a transmission line different from an Internet or an intranet.

[Embodiment 5] The cell analysis method according to embodiment 3, wherein
the transmission line has a communication band of not less than 1 gigabit/second.

[Embodiment 6] The cell analysis method according to embodiment 3, wherein
the transmission line is a bus, and the data is transmitted to the parallel-processing processor via the bus.

[Embodiment 7] The cell analysis method according to any one of embodiments 3 to 6, wherein
data obtained through conversion of an analog signal regarding each of the plurality of cells measured by a flow cytometer is transmitted to the parallel-processing processor via the transmission line.

[Embodiment 8] The cell analysis method according to any one of embodiments 1 to 7, wherein
the information includes an identifier for identifying the cell type.

[Embodiment 9] The cell analysis method according to any one of embodiments 1 to 8, wherein
the information includes a probability at which the cell belongs to each of a plurality of the cell types.

[Embodiment 10] The cell analysis method according to any one of embodiments 1 to 9, comprising
transmitting an analysis result including an identifier for identifying the cell type, to a processing unit which performs analysis of the analysis result.

[Embodiment 11] The cell analysis method according to any one of embodiments 1 to 10, comprising
transmitting an analysis result including a probability at which the cell belongs to each of a plurality of the cell types, to a processing unit which performs analysis of the analysis result.

[Embodiment 12] The cell analysis method according to any one of embodiments 1 to 11, wherein
the parallel-processing processor executes, in parallel as the parallel processing, a plurality of arithmetic processes regarding analysis of the data.

[Embodiment 13] The cell analysis method according to any one of embodiments 1 to 12, wherein
the parallel-processing processor

has a plurality of arithmetic units each capable of executing an arithmetic process regarding analysis of the data, and
executes, in parallel as the parallel processing, the arithmetic processes by the respective arithmetic units.

[Embodiment 14] The cell analysis method according to embodiment 2 or any one of embodiments 3 to 13 reciting embodiment 2, wherein
the artificial intelligence algorithm is a deep learning algorithm.

[Embodiment 15] The cell analysis method according to any one of embodiments 1 to 14, wherein
the parallel-processing processor executes, in parallel as the parallel processing, a filtering process for extracting a feature of the data.

[Embodiment 16] The cell analysis method according to embodiment 2 or any one of embodiments 3 to 15 reciting embodiment 2, wherein

the artificial intelligence algorithm is a deep learning algorithm, and
the parallel-processing processor executes, in parallel as the parallel processing, a plurality of arithmetic processes in a convolution layer in the deep learning algorithm.

[Embodiment 17] The cell analysis method according to any one of embodiments 1 to 16, wherein
the parallel-processing processor executes the parallel processing in accordance with a single order.

[Embodiment 18] The cell analysis method according to any one of embodiments 1 to 17, wherein

the data is data based on a signal detected through application of light to the cell.

[Embodiment 19] The cell analysis method according to any one of embodiments 1 to 18, comprising

inputting, as the data, sampling data obtained by sampling at a predetermined rate a signal obtained by measuring the cell, to the parallel-processing processor, wherein
the parallel-processing processor analyzes the sampling data by executing the parallel processing.

[Embodiment 20] The cell analysis method according to any one of embodiments 1 to 18, comprising

inputting, as the data, image data obtained through application of light to the cell, to the parallel-processing processor, wherein
the parallel-processing processor analyzes the image data by executing the parallel processing.

[Embodiment 21] The cell analysis method according to any one of embodiments 1 to 20, wherein
the parallel-processing processor executes a plurality of matrix operations for each piece of the data.

[Embodiment 22] The cell analysis method according to any one of embodiments 1 to 21, wherein
the parallel-processing processor executes at least 100 matrix operations for each piece of the data.

[Embodiment 23] The cell analysis method according to any one of embodiments 1 to 22, wherein
the parallel-processing processor executes at least 1000 matrix operations for each piece of the data.

[Embodiment 24] The cell analysis method according to any one of embodiments 1 to 23, wherein
the parallel-processing processor analyzes the data that corresponds to each of at least 100 said cells.

[Embodiment 25] The cell analysis method according to any one of embodiments 1 to 24, wherein
the parallel-processing processor analyzes the data that corresponds to each of at least 1000 said cells.

[Embodiment 26] The cell analysis method according to any one of embodiments 1 to 25, wherein
the parallel-processing processor analyzes pieces of the data each having a volume of at least 1 gigabyte.

[Embodiment 27] The cell analysis method according to any one of embodiments 1 to 26, wherein
the parallel-processing processor

has at least 10 arithmetic units each capable of executing an arithmetic process regarding analysis of the data, and
executes, in parallel as the parallel processing, the arithmetic processes by the respective arithmetic units.

[Embodiment 28] The cell analysis method according to any one of embodiments 1 to 27, wherein
the parallel-processing processor

has at least 100 arithmetic units each capable of executing an arithmetic process regarding analysis of the data, and
executes, in parallel as the parallel processing, the arithmetic processes by the respective arithmetic units.

[Embodiment 29] The cell analysis method according to any one of embodiments 1 to 28, wherein
the parallel-processing processor

has at least 1000 arithmetic units each capable of executing an arithmetic process regarding analysis of the data, and
executes, in parallel as the parallel processing, the arithmetic processes by the respective arithmetic units.

[Embodiment 30] The cell analysis method according to any one of embodiments 1 to 29, wherein
the parallel-processing processor executes the parallel processing using, as an input, the data read out from a memory having a capacity of at least 1 gigabyte.

[Embodiment 31] The cell analysis method according to any one of embodiments 1 to 30, wherein

data regarding a cell in a specimen is a combination of pieces of data based on a plurality of kinds of signals obtained from the cell.

[Embodiment 32] A cell analysis method comprising:

performing measurement of a cell by a measurement unit;
executing, by a parallel-processing processor connected to the measurement unit not via an Internet or an intranet, parallel processing of data regarding the cell based on the measurement; and
generating, on the basis of a result of the parallel processing, information regarding a cell type of the cell.

[Embodiment 33] A cell analysis method comprising:

suctioning a specimen by a measurement unit installed in a cell analyzer;
generating data regarding a cell in the specimen having been suctioned;
executing, by a parallel-processing processor installed in the cell analyzer, parallel processing regarding the data; and
generating, on the basis of a result of the parallel processing executed by the parallel-processing processor, information regarding a cell type of the cell.

[Embodiment 34] A cell analyzer comprising:

a measurement unit configured to measure a plurality of cells contained in a specimen;
a processor configured to perform information processing regarding analysis of the plurality of cells; and
a parallel-processing processor, wherein
the measurement unit obtains data regarding each of the plurality of cells,
the parallel-processing processor executes parallel processing regarding the data, and
the processor processes information, regarding a cell type of each of the plurality of cells, which has been generated on the basis of a result of the parallel processing.

## Claims

**1.** A particle analyzer comprising:

a measurement unit including a flow cytometer, wherein the measurement unit is configured to cause a sample containing a plurality of particles to flow through a flow cell of the flow cytometer, and to acquire an analog signal corresponding to a change in an intensity of light emitted from the particles irradiated with light for each of the plurality of particles;
an analysis unit configured to classify a type of each of the plurality of particles by processing matrix data of each of the plurality of particles obtained based on the analog signal by utilizing an artificial intelligence algorithm;
wherein the matrix data has, as elements of the matrix data, values digitally indicating the analog signal level at a plurality of time points;
wherein the artificial intelligence algorithm includes matrix operations for processing the matrix data;
wherein the analysis unit has a plurality of arithmetic units;
wherein the analysis unit assigns each of the plurality of arithmetic processes constituting the matrix operations to one of the plurality of arithmetic units;
wherein each of the plurality of arithmetic units executes the assigned arithmetic process in parallel.

**2.** The particle analyzer according to claim 1, wherein
the matrix operations included in the artificial intelligence algorithm are dividable into the plurality of arithmetic processes that are not dependent on a sequential order with each other.

**3.** The particle analyzer according to claim 1 or 2, wherein
the analysis unit comprises a first arithmetic unit and a second arithmetic unit, and the first arithmetic unit and the second arithmetic unit are configured to execute the divided arithmetic processes simultaneously.

**4.** The particle analyzer according to any one of claims 1 to 3, wherein
the analysis unit comprises a storage for storing a program for analysis, a first RAM used for deploying the program, and a second RAM used for the matrix operations, and the arithmetic unit acquires at least part of the matrix data from the second RAM and executes the arithmetic process.

5. The particle analyzer according to claim 4, wherein the analysis unit is configured to temporarily store the matrix data in the first RAM and to transmit the temporarily stored matrix data to the second RAM via a transmission line.

6. The particle analyzer according to claim 4 or 5, wherein the analysis unit is configured to temporarily store the matrix data in the first RAM and to transmit the temporarily stored matrix data to the second RAM via a transmission line different from the internet or intranet.

7. The particle analyzer according to claim 5, wherein the transmission line has a data transfer rate of not less than 1 GB/s .

8. The particle analyzer according to any one of claims 1 to 7, wherein the artificial intelligence algorithm is a deep learning algorithm.

9. The particle analyzer according to claim 8, wherein the analysis unit is configured to assign each of the plurality of arithmetic processes included in the matrix operations related to convolutional layers in the deep learning algorithm to one of the plurality of arithmetic units.

10. The particle analyzer according to any one of claims 1 to 9, wherein

the analysis unit has at least 100 arithmetic units capable of executing the arithmetic processes and is configured to execute the arithmetic processes in parallel by each of the arithmetic units, or
the analysis unit has at least 1000 arithmetic units capable of executing the arithmetic processes and is configured to execute the arithmetic processes in parallel by each of the arithmetic units.

11. The particle analyzer according to any one of claims 1 to 10, wherein the analysis unit is configured to output information classifying the type of each of the plurality of particles.

12. The particle analyzer according to claim 11, wherein

the information includes identifiers representing the types, and/or
the information includes probabilities that the particles belong to each of the plurality of types.

13. The particle analyzer according to claim 11, wherein the analysis unit is configured to generate a test result counted for each type of the plurality of particles based on the information.

14. The particle analyzer according to claim 13, wherein the analysis unit is configured to output a warning indicating that an abnormal particle is contained in the test result.

15. A particle analysis method using a measurement unit and an analysis unit, comprising:

flowing a sample containing a plurality of particles through a flow cell of a flow cytometer included in the measurement unit;
acquiring an analog signal corresponding to a change in an intensity of light emitted from the particles irradiated with light for each of the plurality of particles;
converting the analog signal into matrix data related to each of the plurality of particles in the sample, wherein the matrix data has, as elements, values digitally indicating the analog signal level at a plurality of time points for each of the plurality of particles;
classifying, by the analysis unit, a type of each of the plurality of particles by processing the matrix data obtained based on the analog signal by utilizing an artificial intelligence algorithm, wherein the artificial intelligence algorithm includes matrix operations for processing the matrix data;

wherein the processing of the matrix data by the analysis unit includes:

assigning each of the plurality of arithmetic processes constituting the matrix operations to one of the plurality of arithmetic units possessed by the analysis unit;
executing the assigned arithmetic processes in parallel by each of the plurality of arithmetic units.

FIG. 1

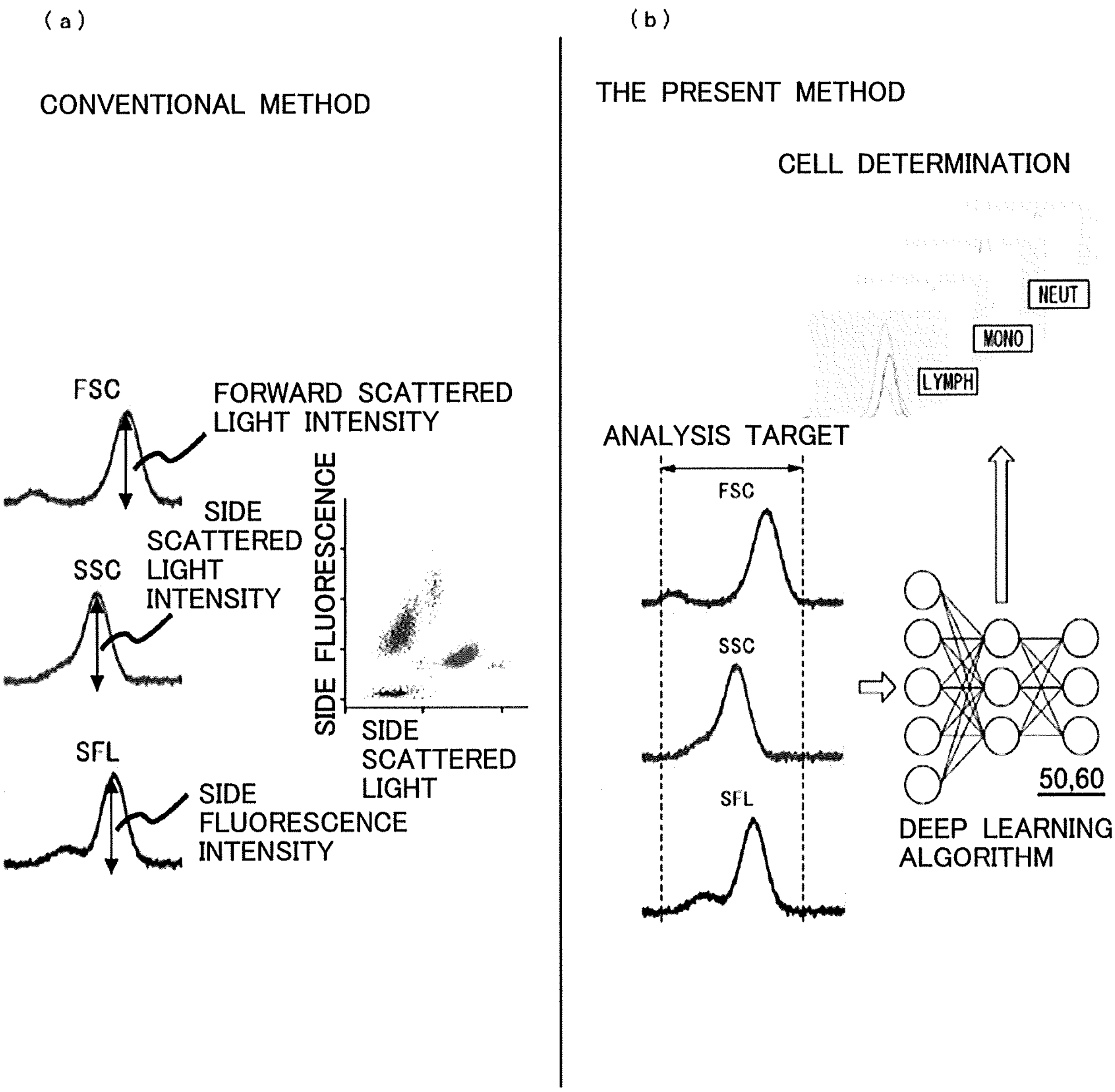
(a)
CONVENTIONAL METHOD
FSC
FORWARD SCATTERED LIGHT INTENSITY
SIDE SCATTERED LIGHT INTENSITY
SSC
SFL
SIDE FLUORESCENCE INTENSITY
SIDE FLUORESCENCE
SIDE SCATTERED LIGHT
(b)
THE PRESENT METHOD
CELL DETERMINATION
NEUT
MONO
LYMPH
ANALYSIS TARGET
FSC
SSC
SFL
50,60
DEEP LEARNING ALGORITHM

FIG. 2

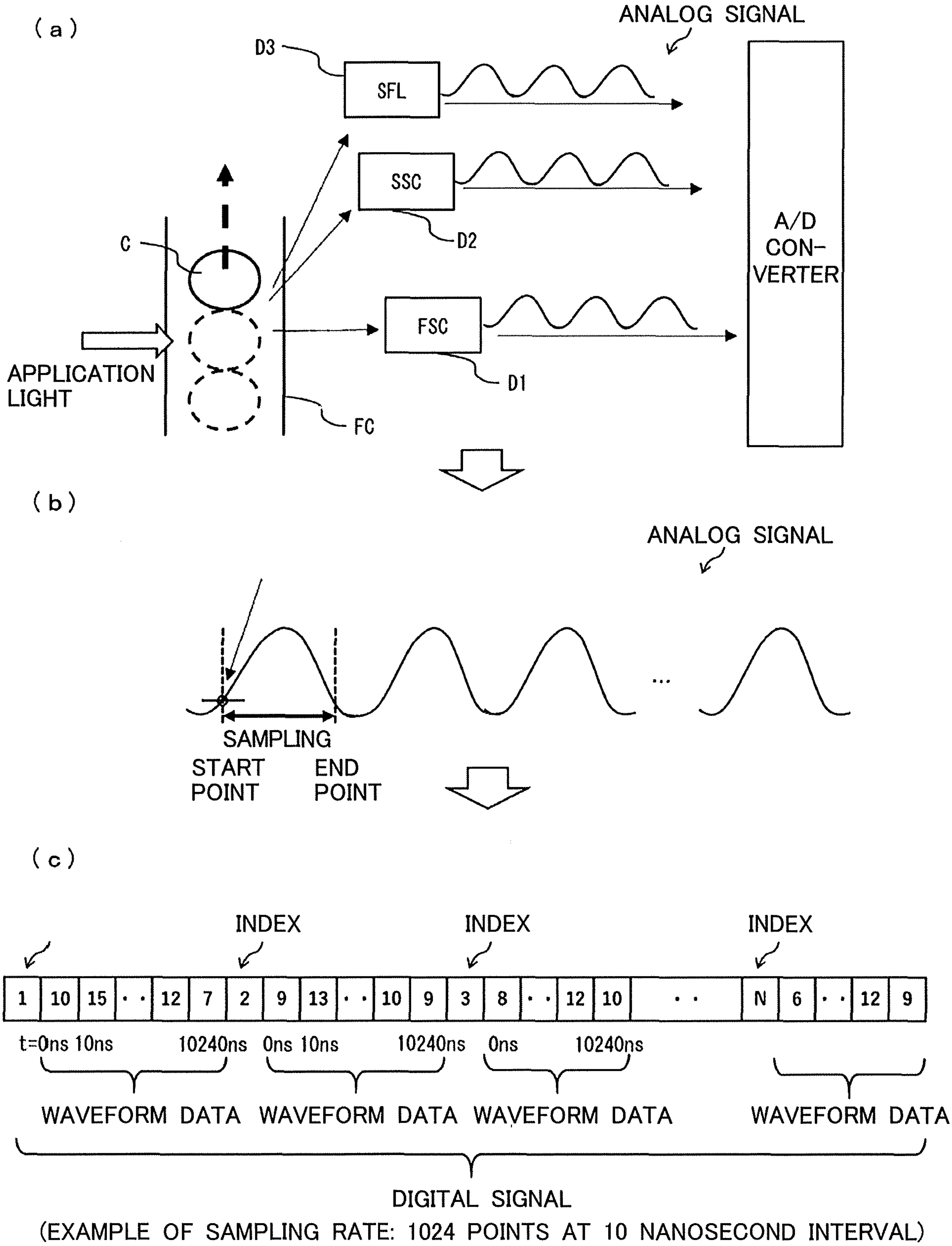
(a)
ANALOG SIGNAL
D3
SFL
SSC
D2
A/D CONVERTER
C
FSC
D1
APPLICATION LIGHT
FC
(b)
ANALOG SIGNAL
...
SAMPLING
START POINT
END POINT
(c)
INDEX
INDEX
INDEX
1 | 10 | 15 | · · | 12 | 7 | 2 | 9 | 13 | · · | 10 | 9 | 3 | 8 | · · | 12 | 10 | · · | N | 6 | · · | 12 | 9
t=0ns 10ns
10240ns
0ns 10ns
10240ns
0ns
10240ns
WAVEFORM DATA
WAVEFORM DATA
WAVEFORM DATA
WAVEFORM DATA
DIGITAL SIGNAL
(EXAMPLE OF SAMPLING RATE: 1024 POINTS AT 10 NANOSECOND INTERVAL)

FIG. 3

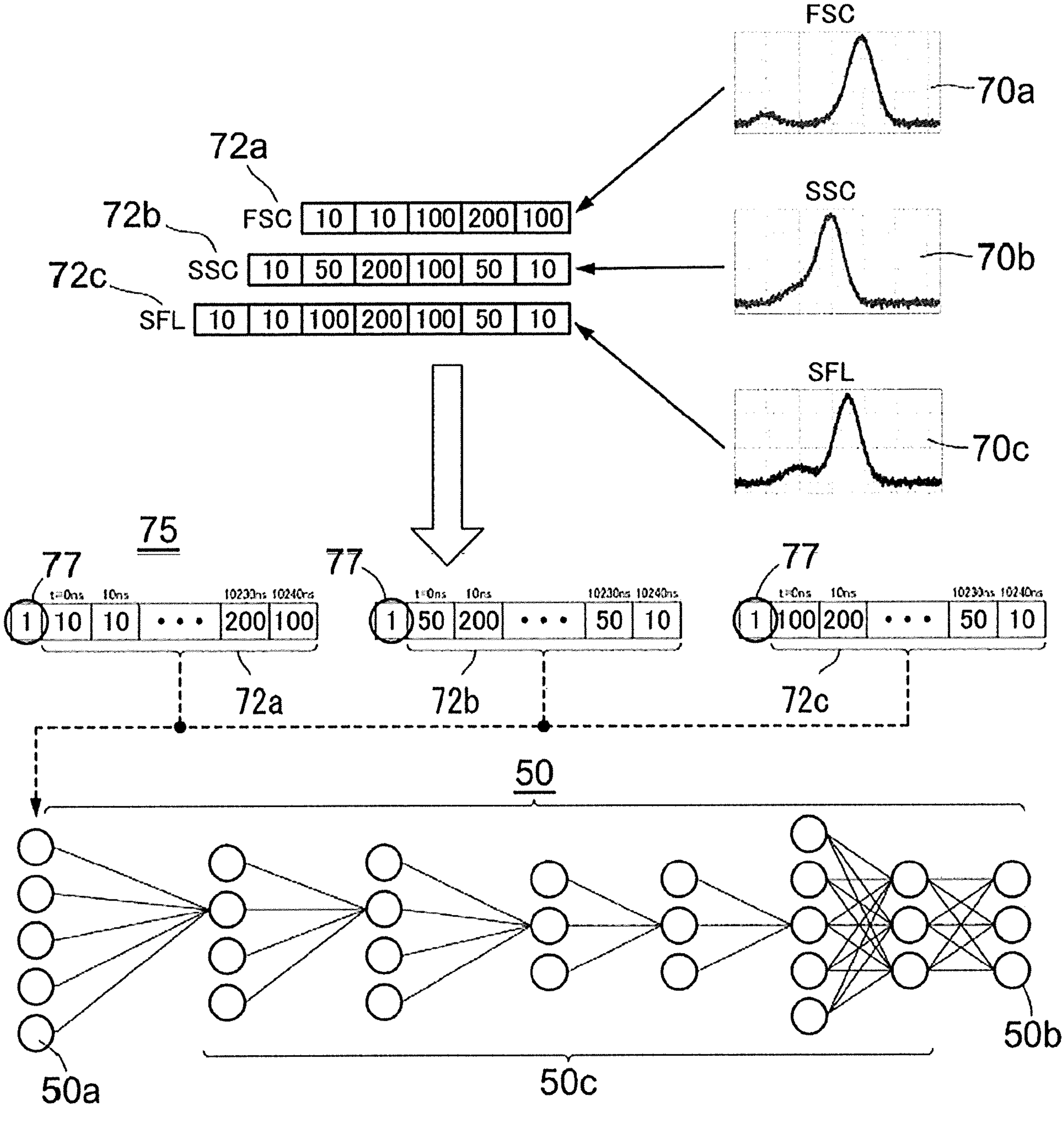
FSC
70a
SSC
70b
SFL
70c
72a
FSC 10 10 100 200 100
72b
SSC 10 50 200 100 50 10
72c
SFL 10 10 100 200 100 50 10
75
77
1 10 10 • • • 200 100
t=0ns 10ns 10230ns 10240ns
1 50 200 • • • 50 10
1 100 200 • • • 50 10
72a
72b
72c
50
50a
50b
50c

FIG. 4

| CELL TYPE | LABEL VALUE |
|---|---|
| NOT APPLICABLE | 0 |
| NEUTROPHIL (NEUT) | 1 |
| LYMPHOCYTE (LYMPH) | 2 |
| MONOCYTE (MONO) | 3 |
| EOSINOPHIL (EO) | 4 |
| BASOPHIL (BASO) | 5 |
| IMMATURE GRANULOCYTE (IG) | 6 |
| ABNORMAL CELL (BLOOD-DERIVED CELL OTHER THAN FIVE CLASSIFICATIONS) | 7 |

FIG. 5

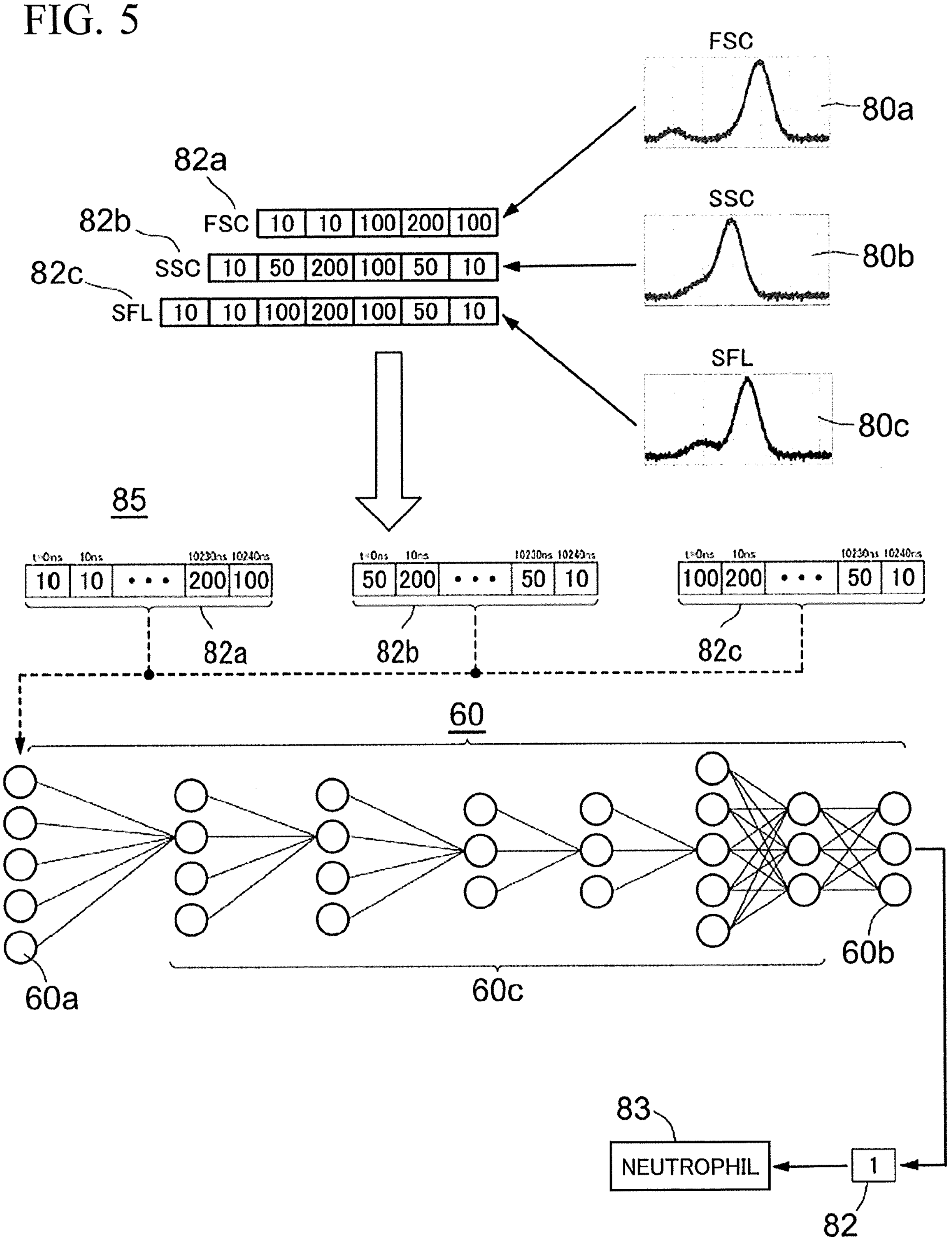
FSC
80a
SSC
80b
SFL
80c
82a
82b
82c
FSC 10 10 100 200 100
SSC 10 50 200 100 50 10
SFL 10 10 100 200 100 50 10
85
10 10 • • • 200 100
50 200 • • • 50 10
100 200 • • • 50 10
60
60a
60b
60c
83
NEUTROPHIL
1
82

FIG. 6

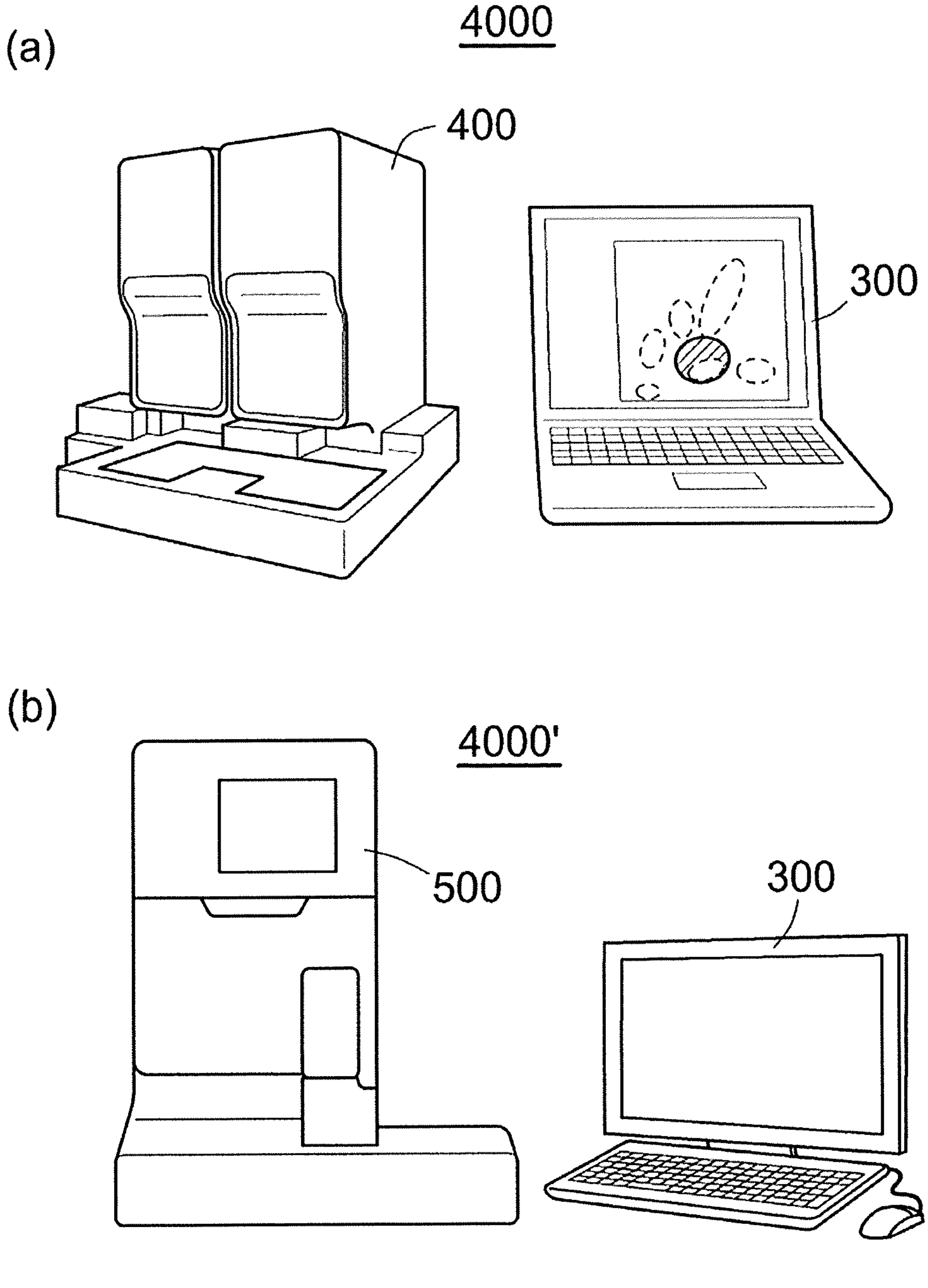

FIG. 7

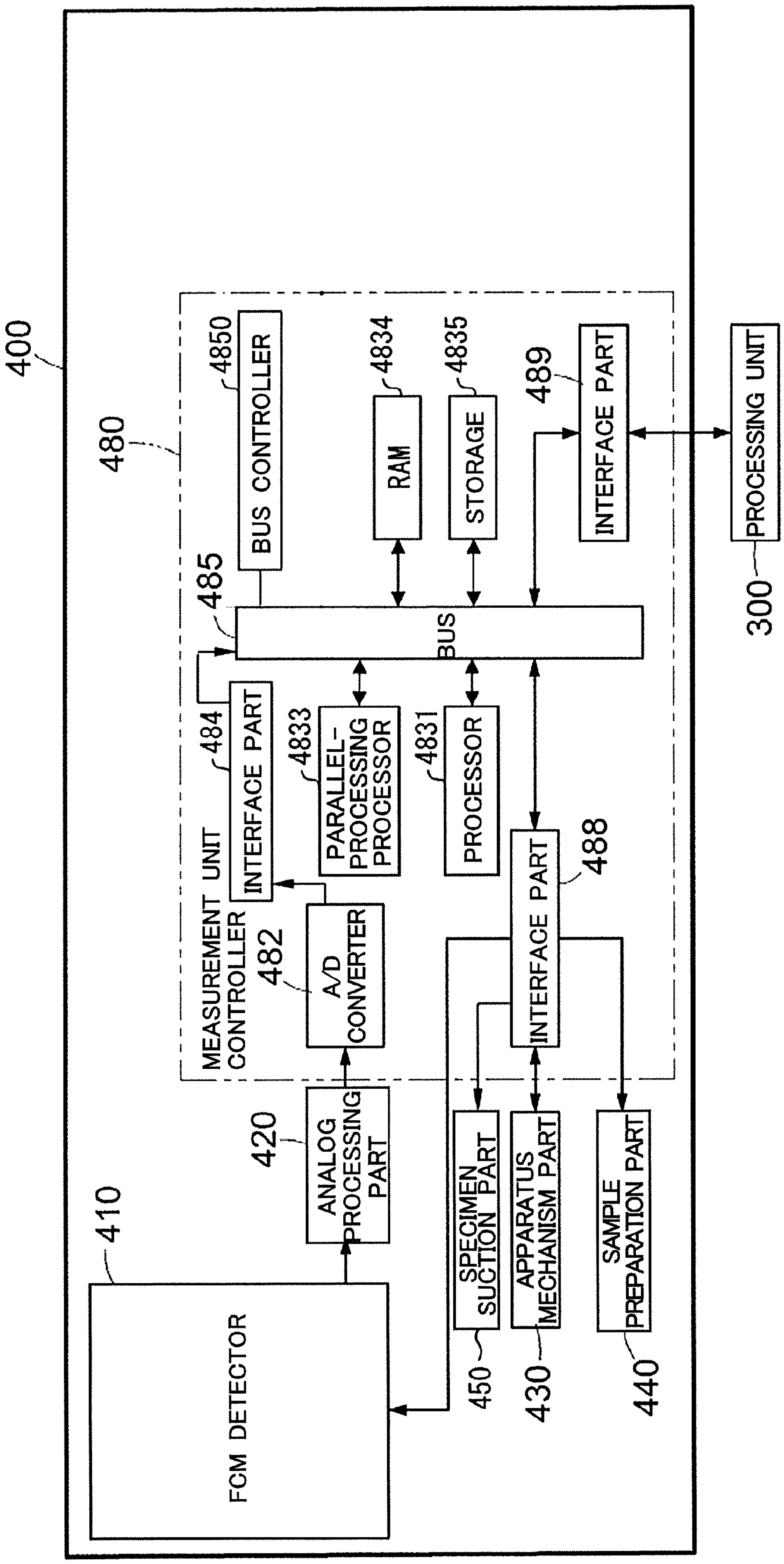
400
480
4850
BUS CONTROLLER
4834
RAM
4835
STORAGE
489
INTERFACE PART
PROCESSING UNIT
300
485
BUS
484
INTERFACE PART
4833
PARALLEL-PROCESSING PROCESSOR
4831
PROCESSOR
488
INTERFACE PART
MEASUREMENT UNIT CONTROLLER
482
A/D CONVERTER
420
ANALOG PROCESSING PART
410
FCM DETECTOR
SPECIMEN SUCTION PART
450
APPARATUS MECHANISM PART
430
SAMPLE PREPARATION PART
440

FIG. 8

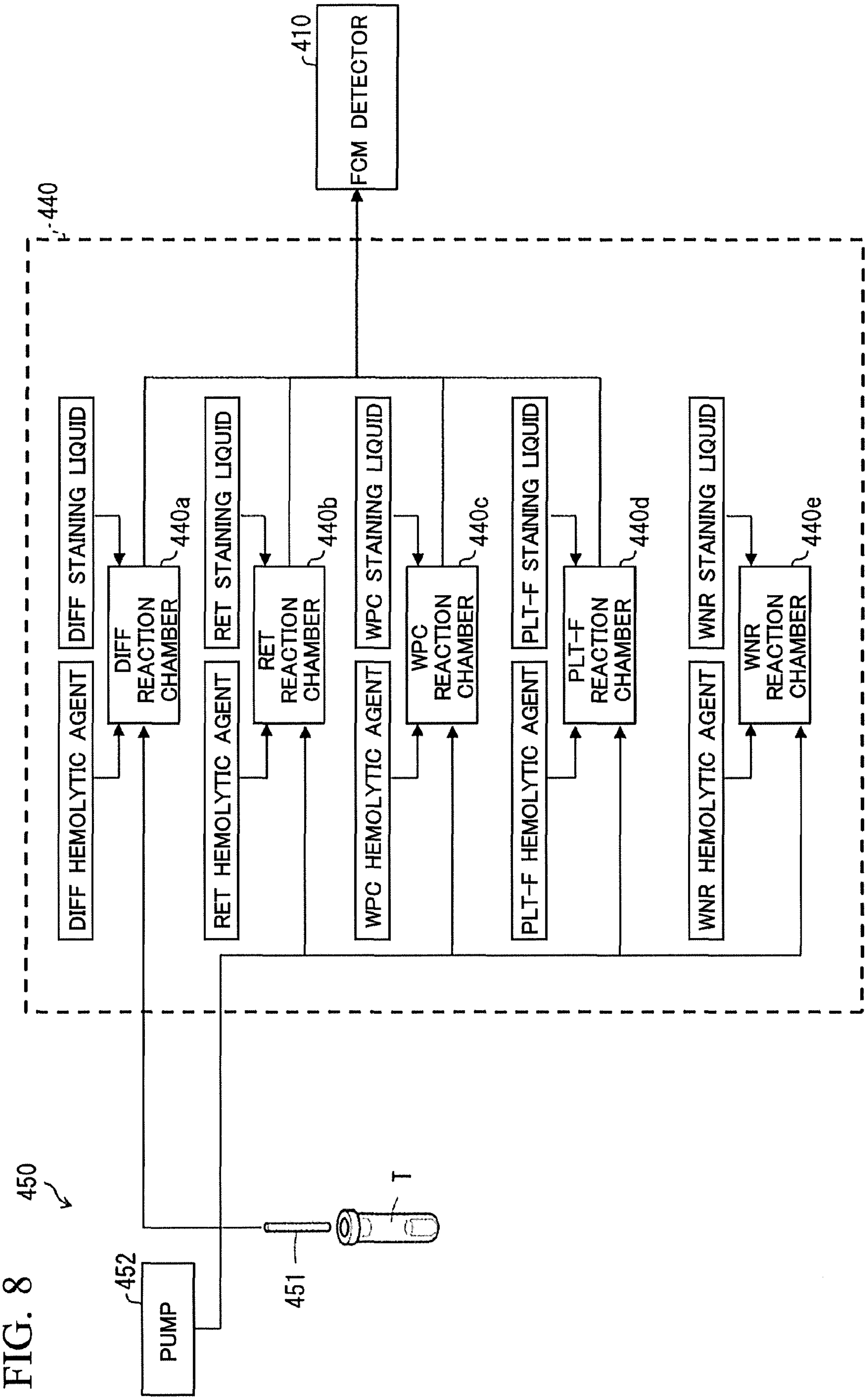
FCM DETECTOR
410
440
DIFF HEMOLYTIC AGENT
DIFF STAINING LIQUID
DIFF REACTION CHAMBER
440a
RET HEMOLYTIC AGENT
RET STAINING LIQUID
RET REACTION CHAMBER
440b
WPC HEMOLYTIC AGENT
WPC STAINING LIQUID
WPC REACTION CHAMBER
440c
PLT-F HEMOLYTIC AGENT
PLT-F STAINING LIQUID
PLT-F REACTION CHAMBER
440d
WNR HEMOLYTIC AGENT
WNR STAINING LIQUID
WNR REACTION CHAMBER
440e
450
PUMP
452
451
T

FIG. 9

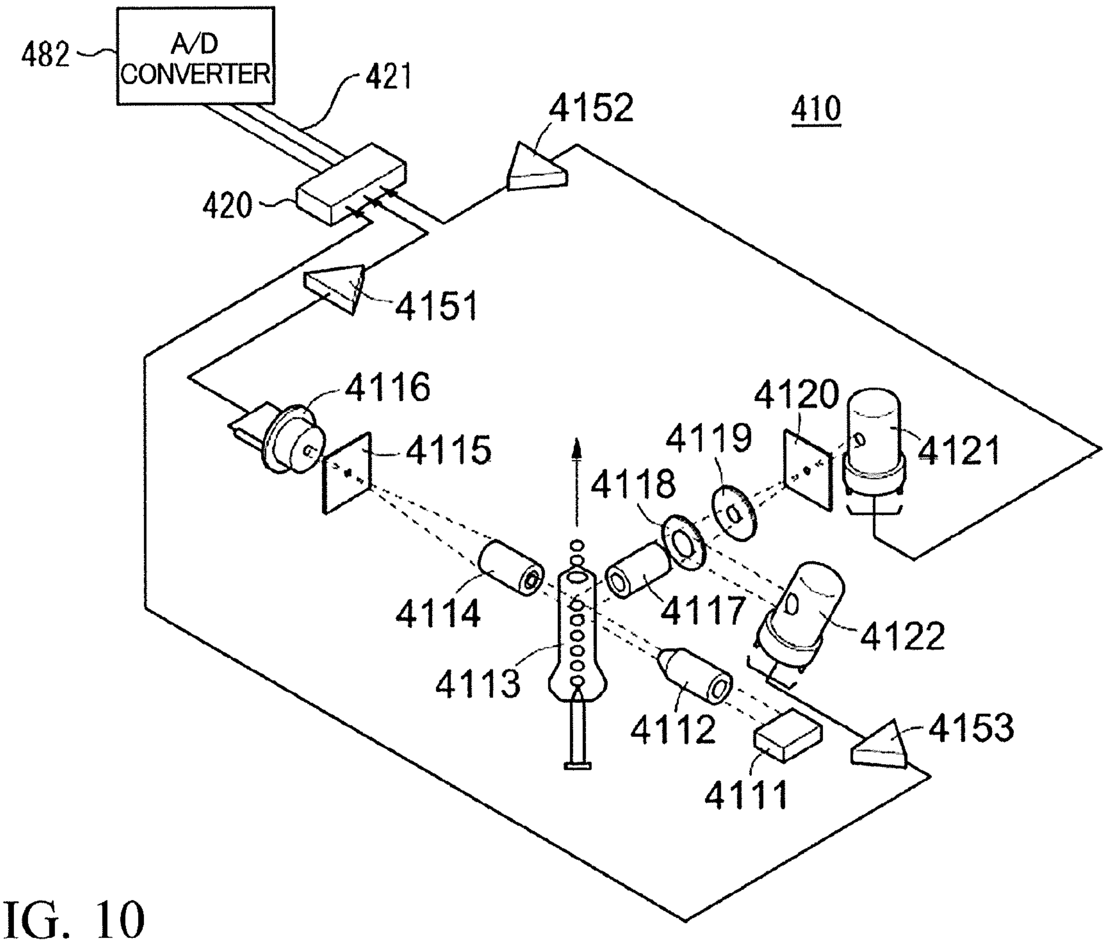
482
A/D CONVERTER
421
4152
410
420
4151
4116
4120
4115
4119
4121
4118
4114
4117
4122
4113
4112
4153
4111

FIG. 10

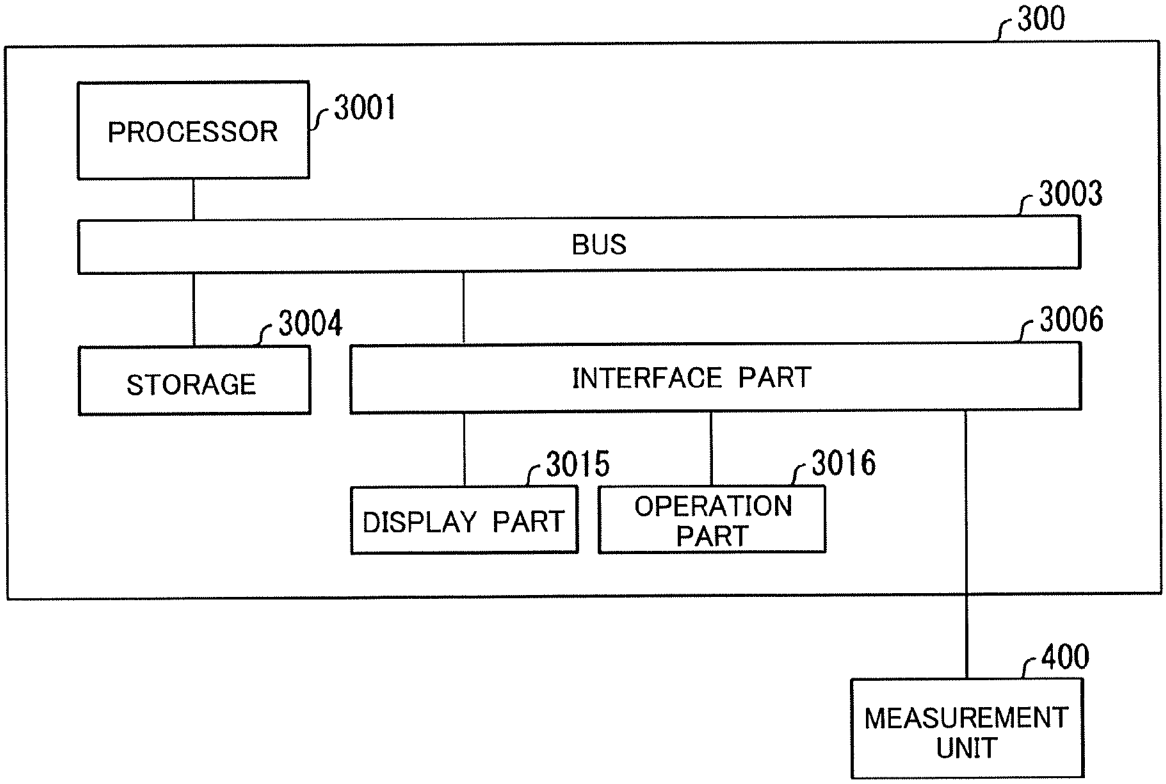
300
PROCESSOR
3001
3003
BUS
3004
STORAGE
3006
INTERFACE PART
3015
3016
DISPLAY PART
OPERATION PART
400
MEASUREMENT UNIT

FIG. 11

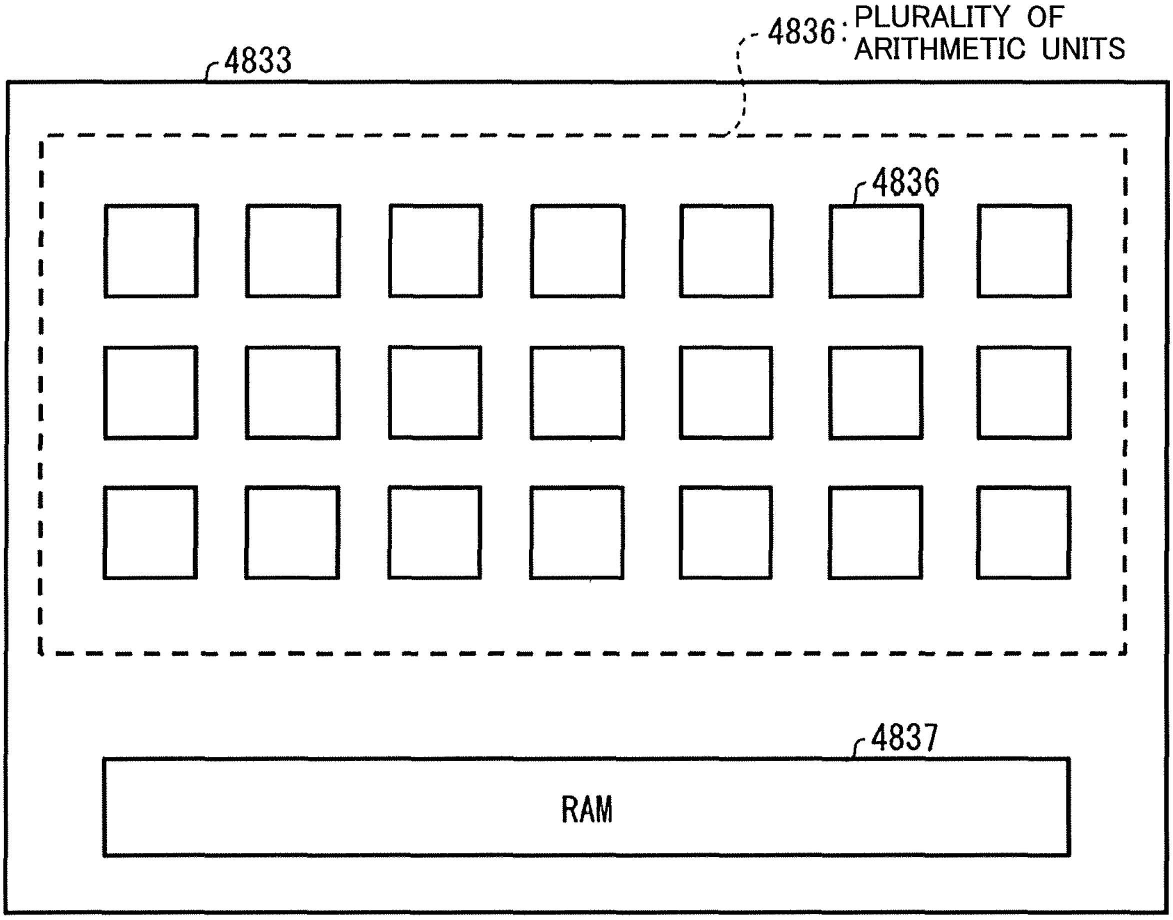
4836: PLURALITY OF ARITHMETIC UNITS
4833
4836
4837
RAM

FIG. 12

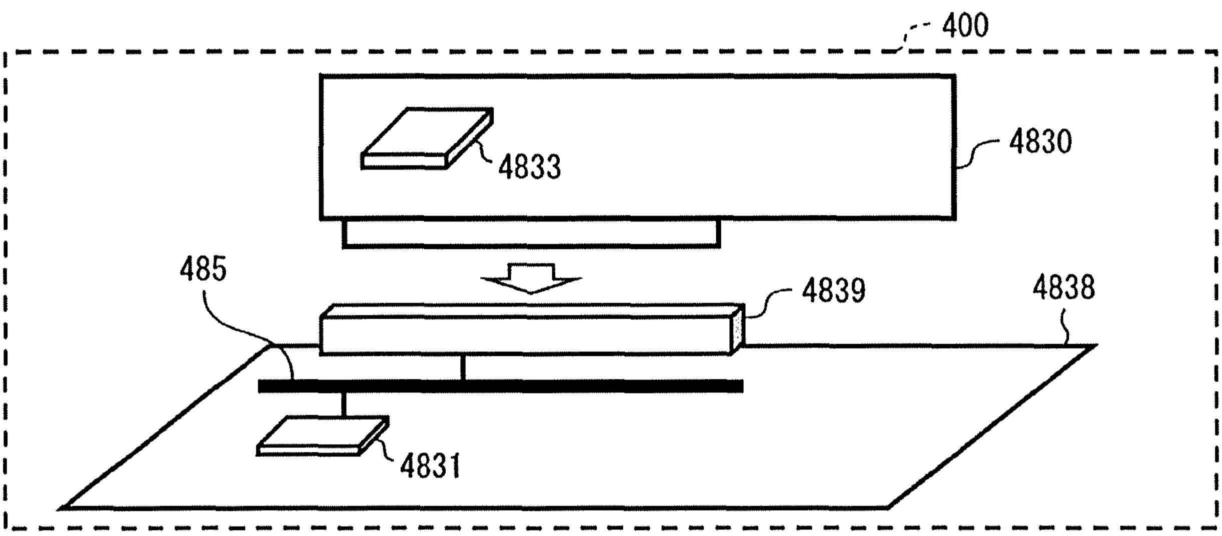
400
4833
4830
485
4839
4838
4831

FIG. 13

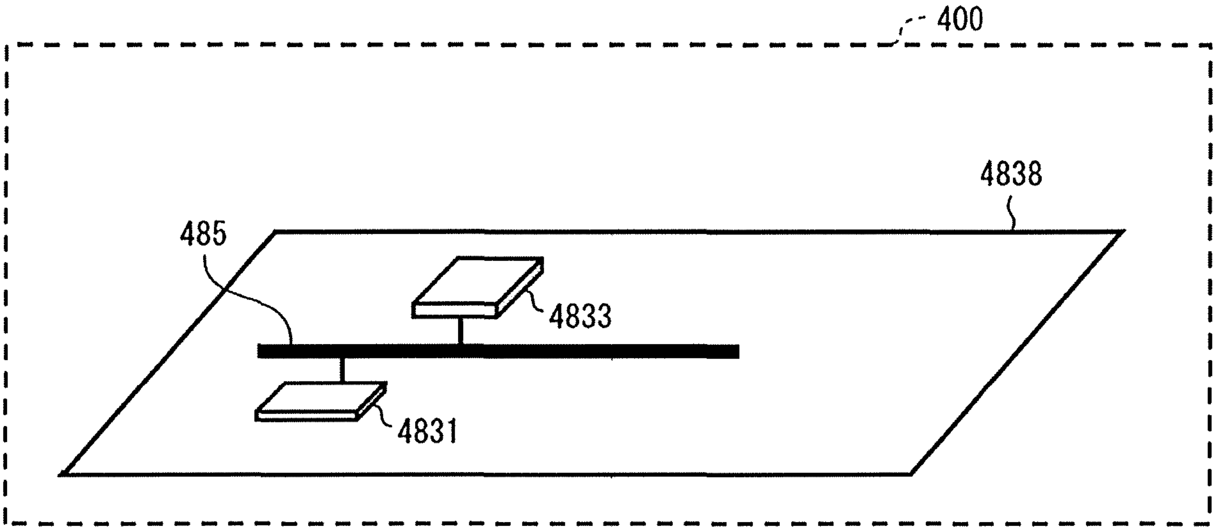


FIG. 14

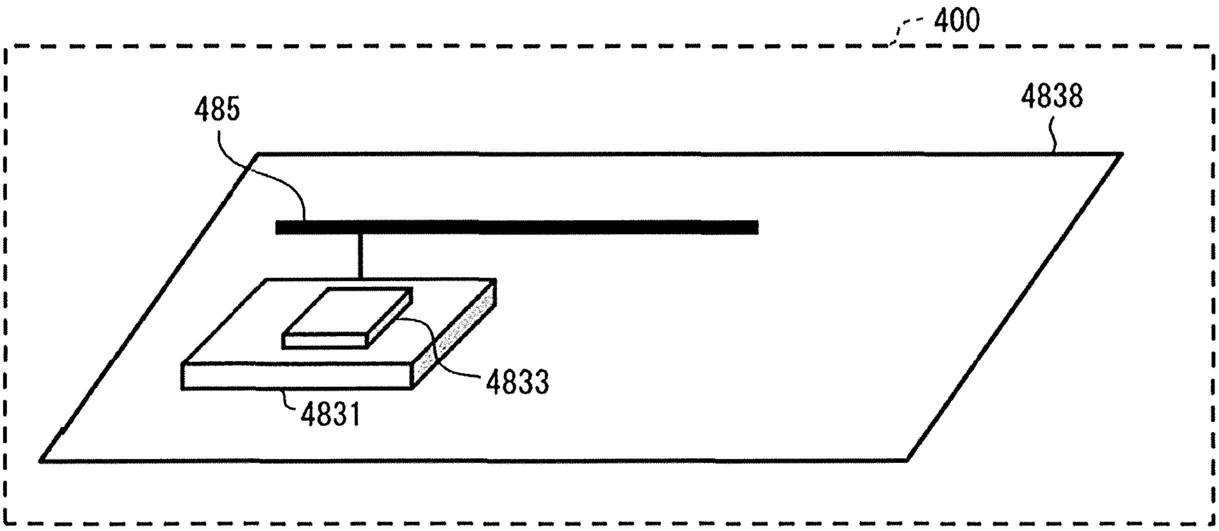

FIG. 15

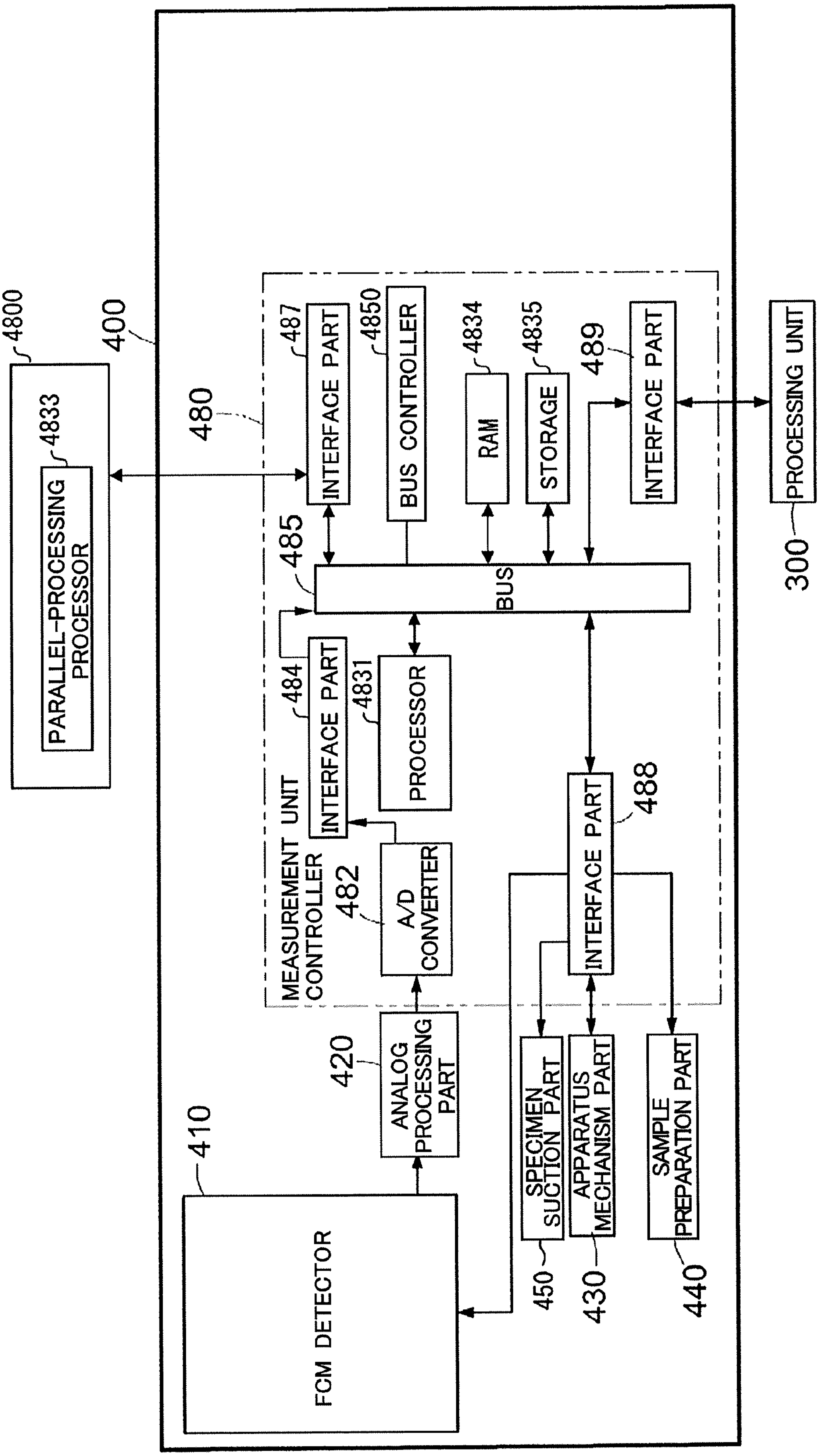
4800
PARALLEL-PROCESSING PROCESSOR
4833
400
480
INTERFACE PART
487
BUS CONTROLLER
4850
RAM
4834
STORAGE
4835
INTERFACE PART
489
PROCESSING UNIT
300
BUS
485
INTERFACE PART
484
PROCESSOR
4831
MEASUREMENT UNIT CONTROLLER
A/D CONVERTER
482
INTERFACE PART
488
ANALOG PROCESSING PART
420
FCM DETECTOR
410
SPECIMEN SUCTION PART
450
APPARATUS MECHANISM PART
430
SAMPLE PREPARATION PART
440

FIG. 16

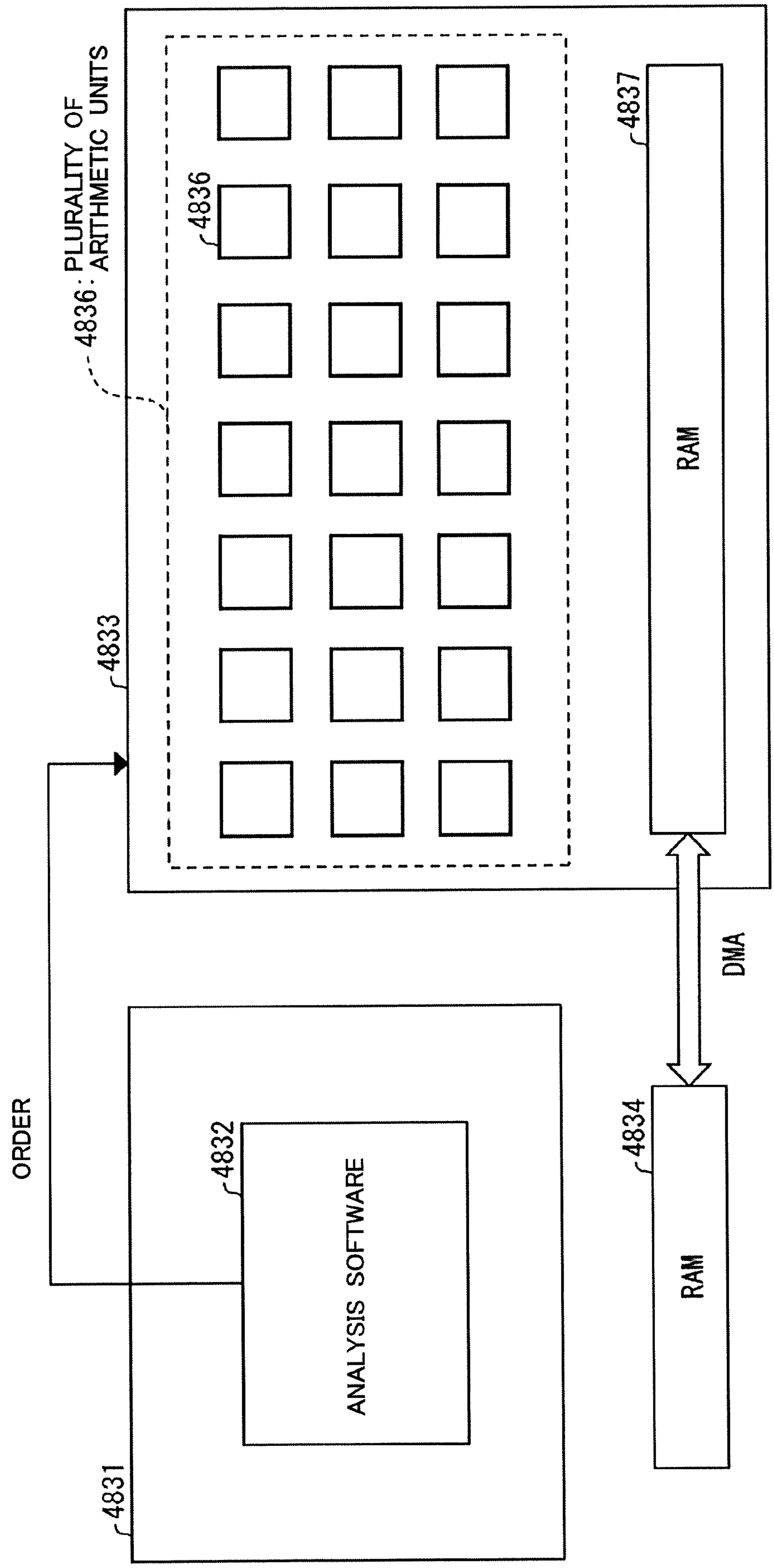
4836 : PLURALITY OF ARITHMETIC UNITS
4836
4833
4837
RAM
DMA
ORDER
4832
ANALYSIS SOFTWARE
4834
RAM
4831

# FIG. 17

(a) CALCULATION OF PRODUCT OF MATRIX

$$c_{ij} = \sum_{k=1}^{n} a_{ik} \times b_{kj}$$

```
for(int i=0; i<n; i++){
    for(int j=0; j<n; j++){
        for(int k=0; k<n; k++){
         c[ i ][ j ] += a[ i ][ k ]*b[ k ][ j ]
} } }
```

(b) CALCULATION PROCESS BY PARALLEL-PROCESSING PROCESSOR

| | | |
|---|---|---|
| for(int k=0; k<n; k++){<br>c[0][0] += a[0][k]*b[k][0] } | for(int k=0; k<n; k++){<br>c[0][1] += a[0][k]*b[k][1] } | . . . |
| for(int k=0; k<n; k++){<br>c[1][0] += a[1][k]*b[k][0] } | for(int k=0; k<n; k++){<br>c[1][1] += a[1][k]*b[k][1] } | . . . |
| . . . | . . . | |

FIG. 18

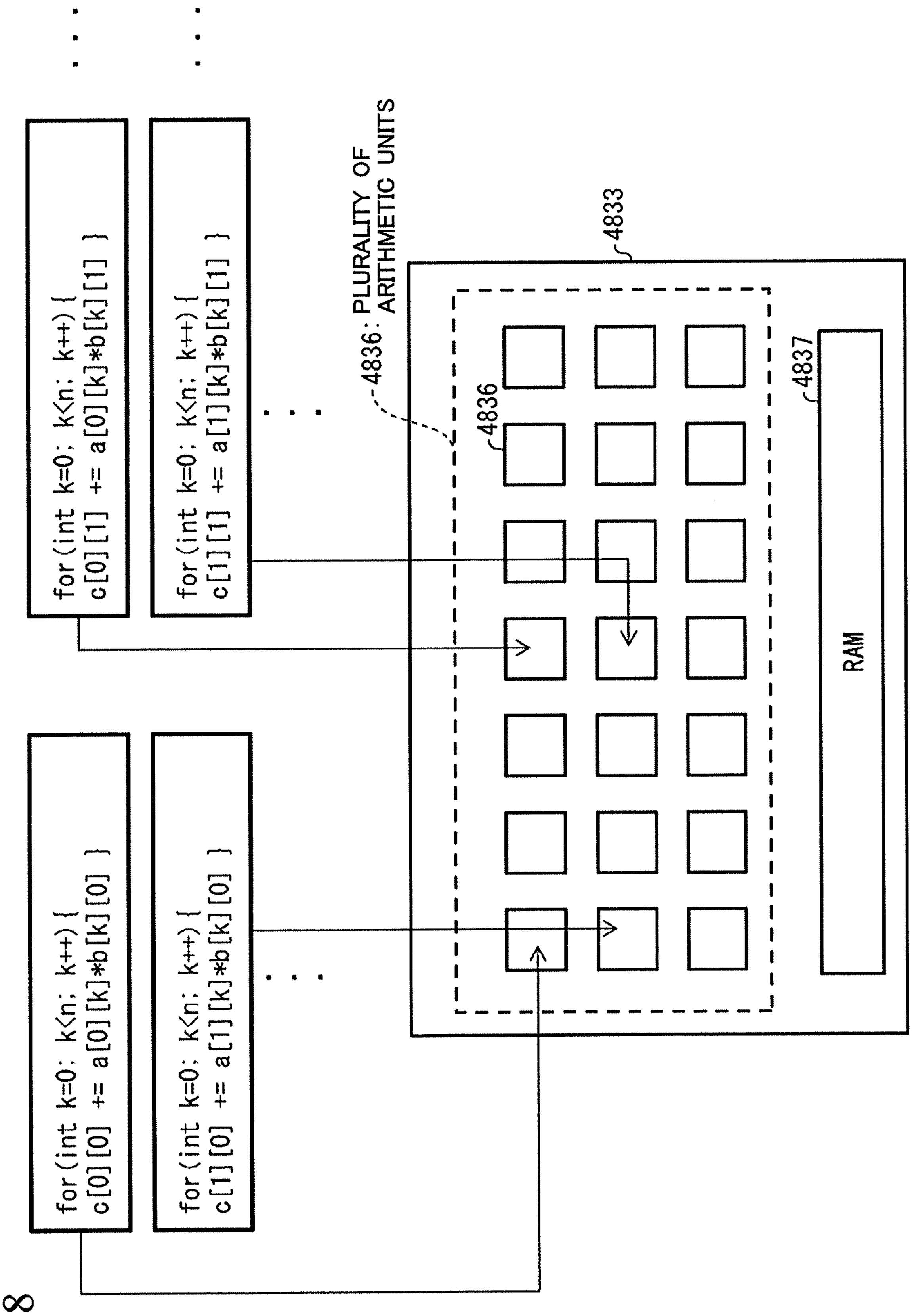
for(int k=0; k<n; k++){
c[0][0] += a[0][k]*b[k][0] }
for(int k=0; k<n; k++){
c[1][0] += a[1][k]*b[k][0] }
for(int k=0; k<n; k++){
c[0][1] += a[0][k]*b[k][1] }
for(int k=0; k<n; k++){
c[1][1] += a[1][k]*b[k][1] }
4836: PLURALITY OF ARITHMETIC UNITS
4833
4836
4837
RAM

FIG. 19

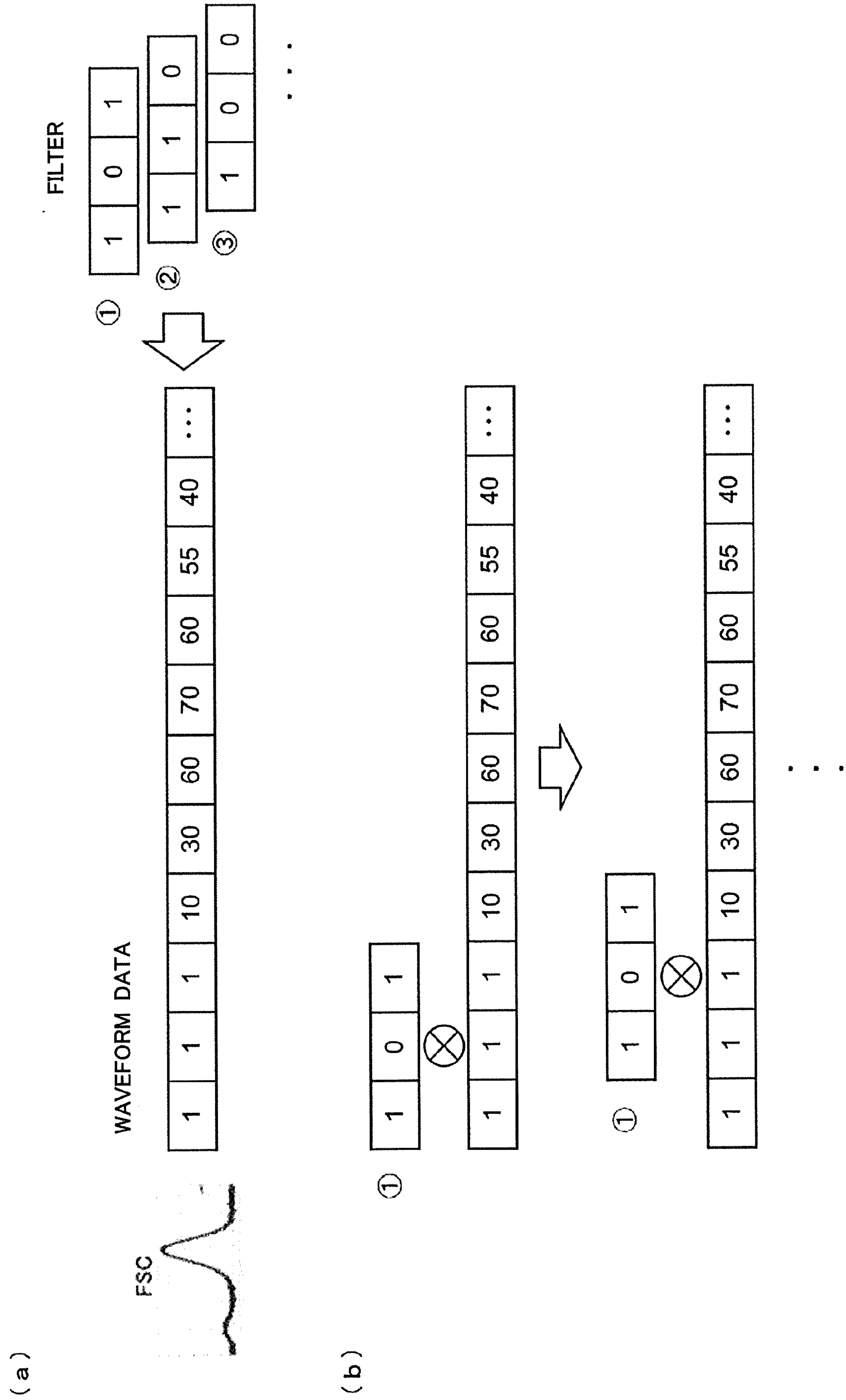
(a)
FSC
WAVEFORM DATA
1 1 1 10 30 60 70 60 55 40 ...
FILTER
① 1 0 1
② 1 1 0
③ 1 0 0
(b)
① 1 0 1
1 1 1 10 30 60 70 60 55 40 ...
① 1 0 1
1 1 1 10 30 60 70 60 55 40 ...

FIG. 20

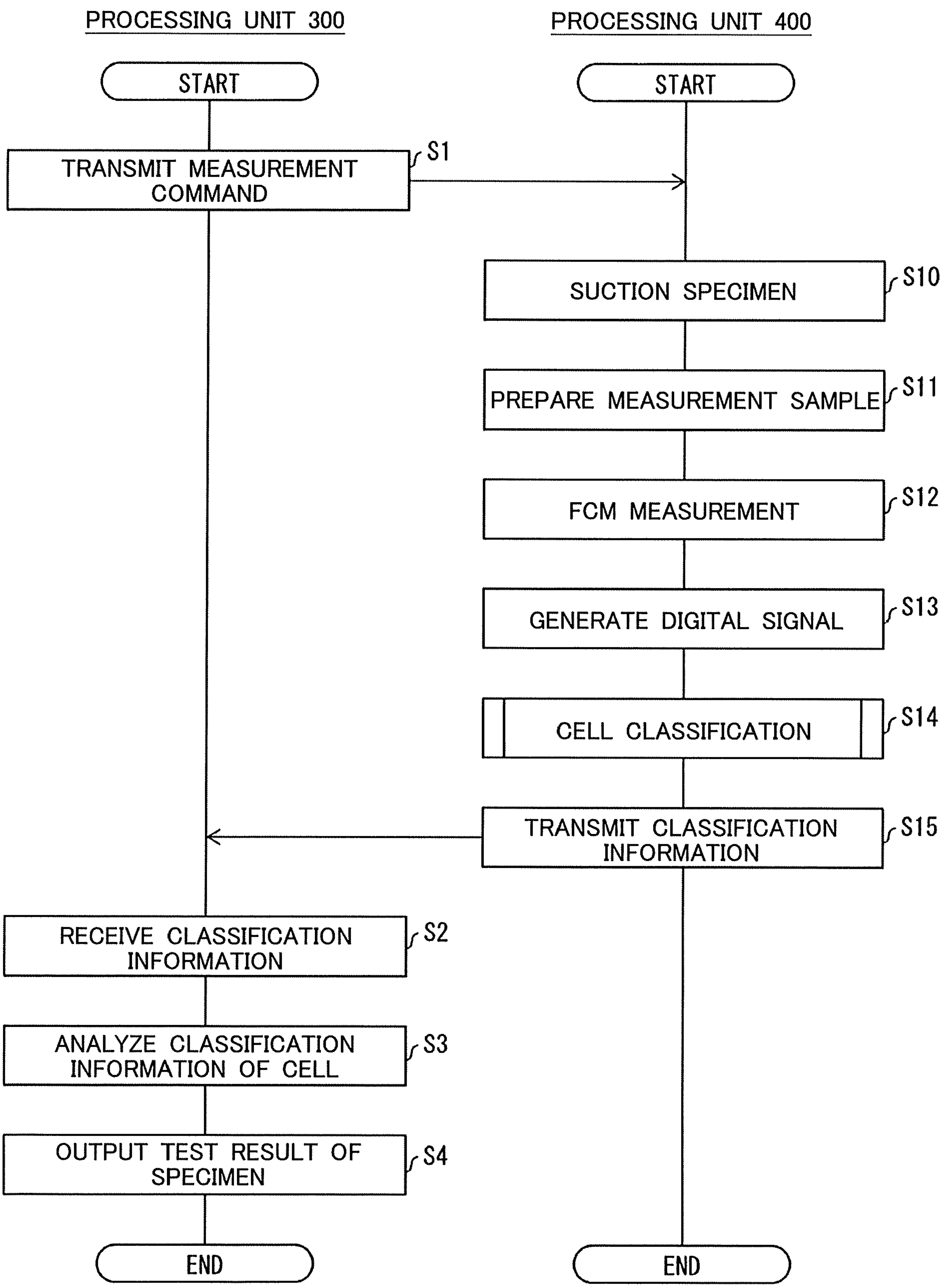
PROCESSING UNIT 300
PROCESSING UNIT 400
START
START
TRANSMIT MEASUREMENT COMMAND
S1
SUCTION SPECIMEN
S10
PREPARE MEASUREMENT SAMPLE
S11
FCM MEASUREMENT
S12
GENERATE DIGITAL SIGNAL
S13
CELL CLASSIFICATION
S14
TRANSMIT CLASSIFICATION INFORMATION
S15
RECEIVE CLASSIFICATION INFORMATION
S2
ANALYZE CLASSIFICATION INFORMATION OF CELL
S3
OUTPUT TEST RESULT OF SPECIMEN
S4
END
END

FIG. 21

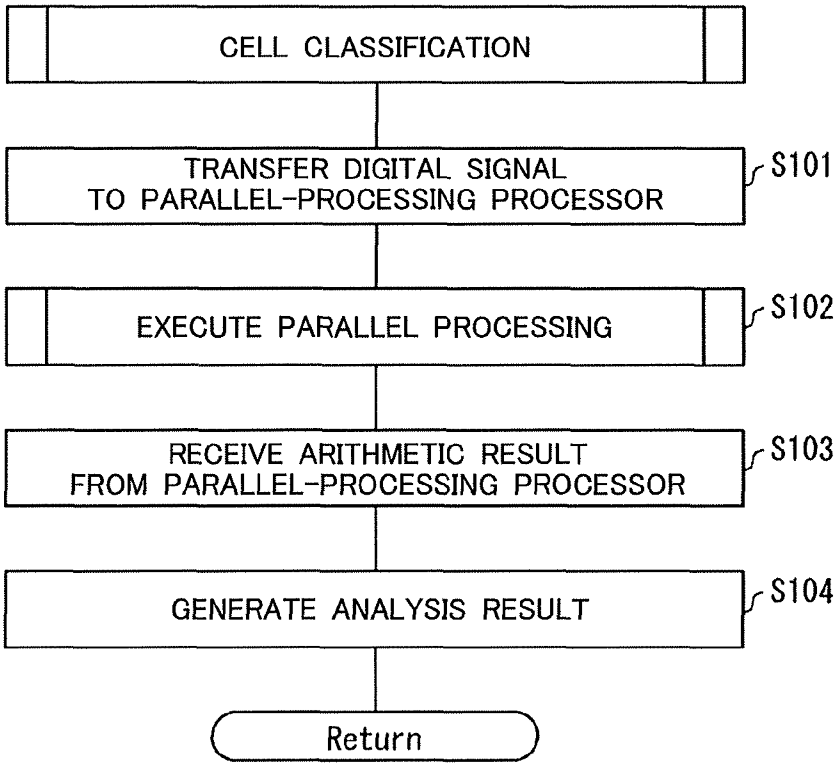
CELL CLASSIFICATION
TRANSFER DIGITAL SIGNAL TO PARALLEL-PROCESSING PROCESSOR
S101
EXECUTE PARALLEL PROCESSING
S102
RECEIVE ARITHMETIC RESULT FROM PARALLEL-PROCESSING PROCESSOR
S103
GENERATE ANALYSIS RESULT
S104
Return

FIG. 22

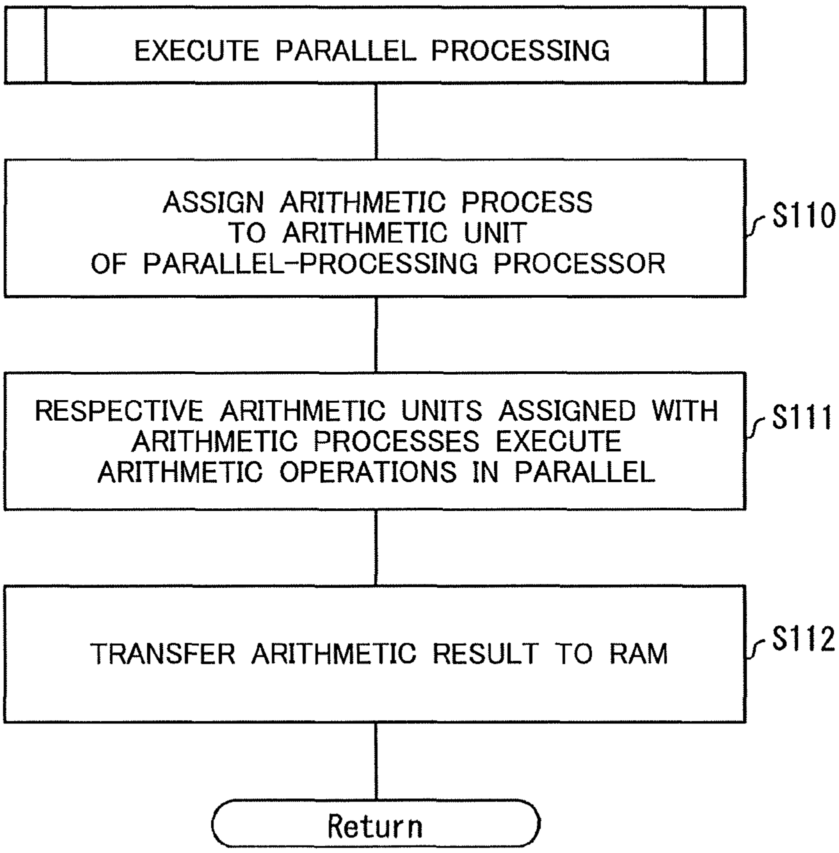
EXECUTE PARALLEL PROCESSING
ASSIGN ARITHMETIC PROCESS TO ARITHMETIC UNIT OF PARALLEL-PROCESSING PROCESSOR
S110
RESPECTIVE ARITHMETIC UNITS ASSIGNED WITH ARITHMETIC PROCESSES EXECUTE ARITHMETIC OPERATIONS IN PARALLEL
S111
TRANSFER ARITHMETIC RESULT TO RAM
S112
Return

FIG. 23

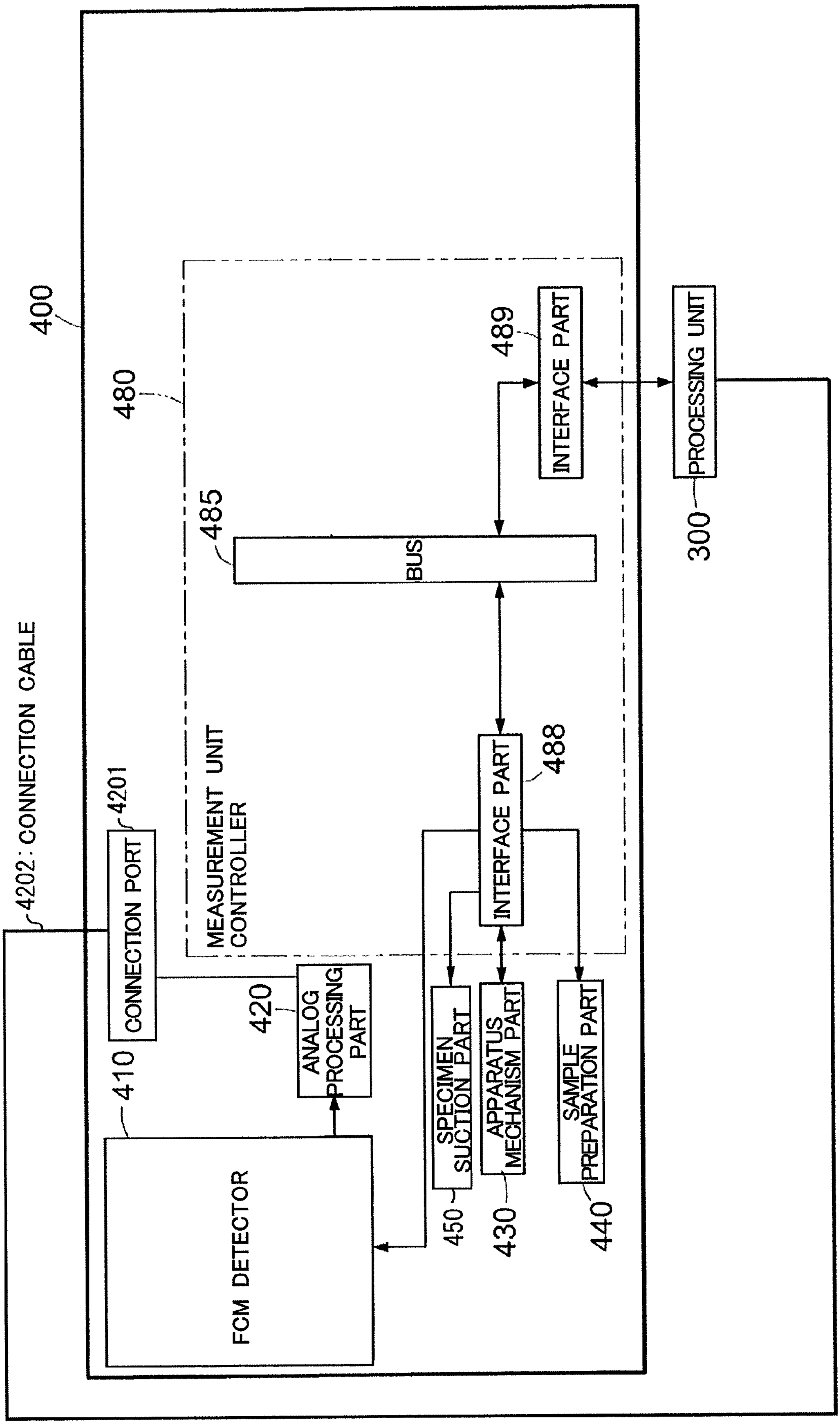
400
480
485
489
INTERFACE PART
PROCESSING UNIT
300
BUS
4202: CONNECTION CABLE
4201
CONNECTION PORT
MEASUREMENT UNIT CONTROLLER
INTERFACE PART
488
410
420
ANALOG PROCESSING PART
SPECIMEN SUCTION PART
APPARATUS MECHANISM PART
SAMPLE PREPARATION PART
FCM DETECTOR
450
430
440

FIG. 24

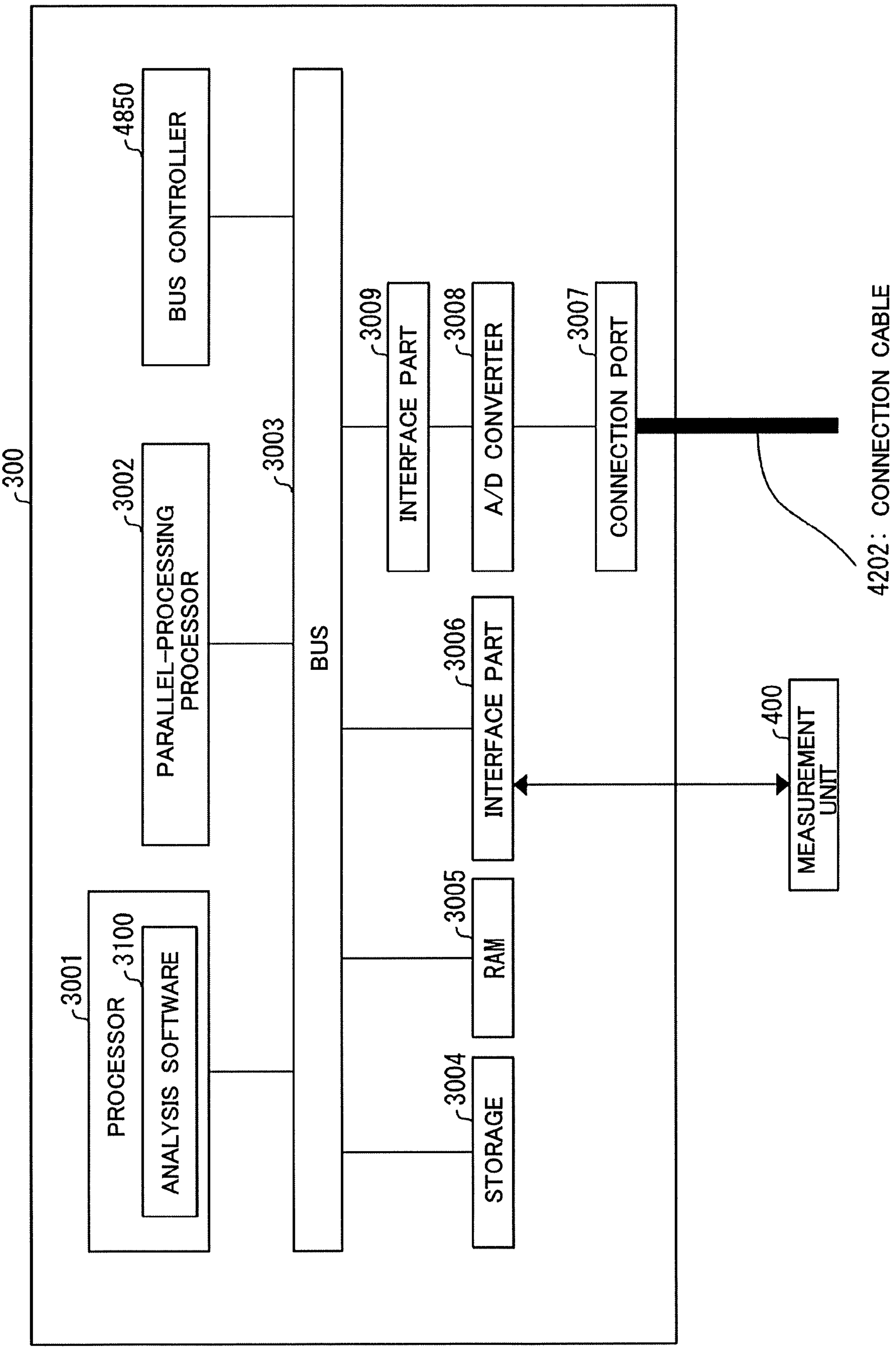
300
4850
BUS CONTROLLER
3002
PARALLEL-PROCESSING PROCESSOR
3003
BUS
3009
INTERFACE PART
3008
A/D CONVERTER
3007
CONNECTION PORT
4202: CONNECTION CABLE
3006
INTERFACE PART
400
MEASUREMENT UNIT
3001
PROCESSOR
3100
ANALYSIS SOFTWARE
3005
RAM
3004
STORAGE

FIG. 25

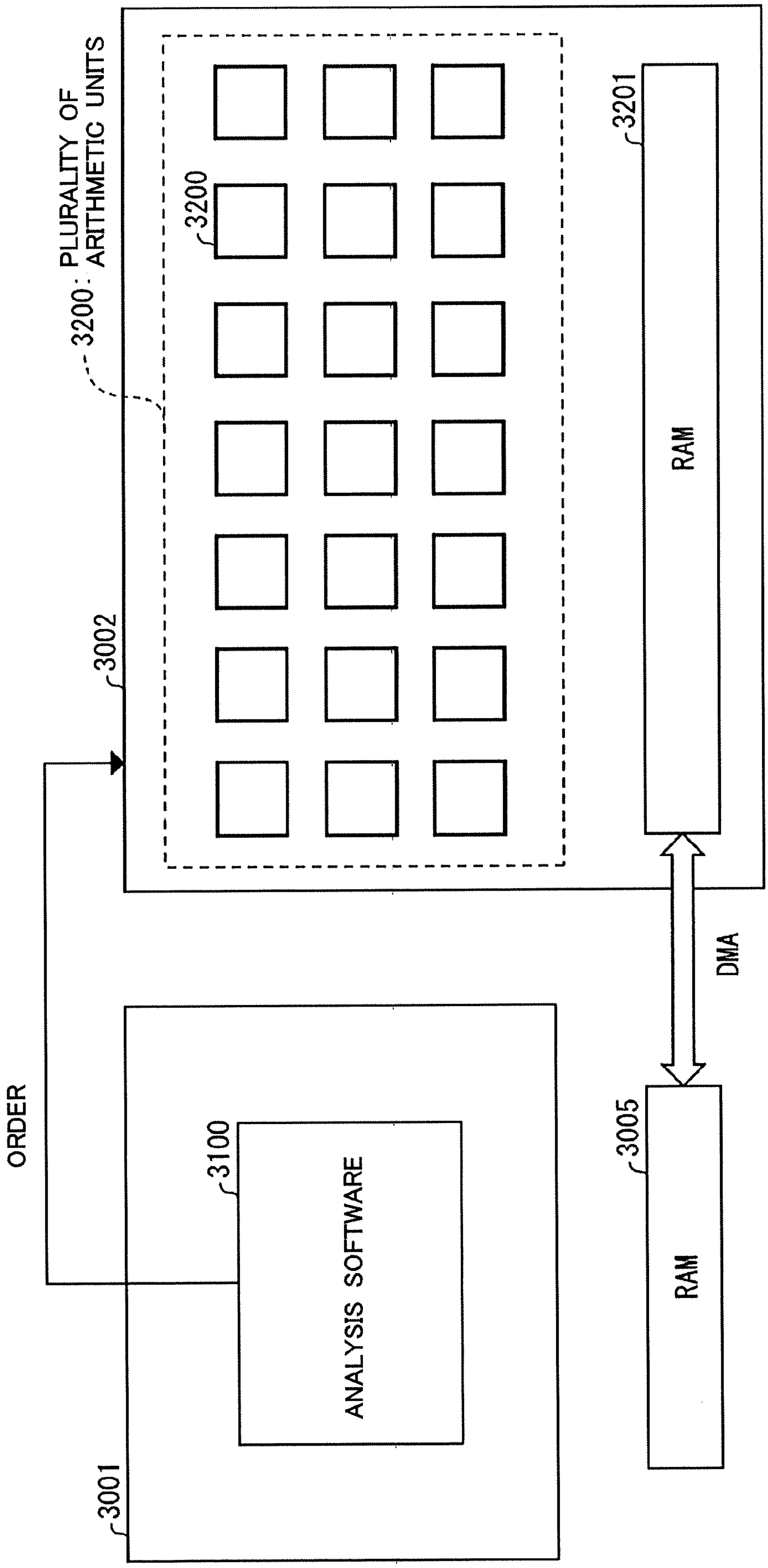
3200 : PLURALITY OF ARITHMETIC UNITS
3200
3201
3002
RAM
DMA
ORDER
3100
ANALYSIS SOFTWARE
3005
RAM
3001

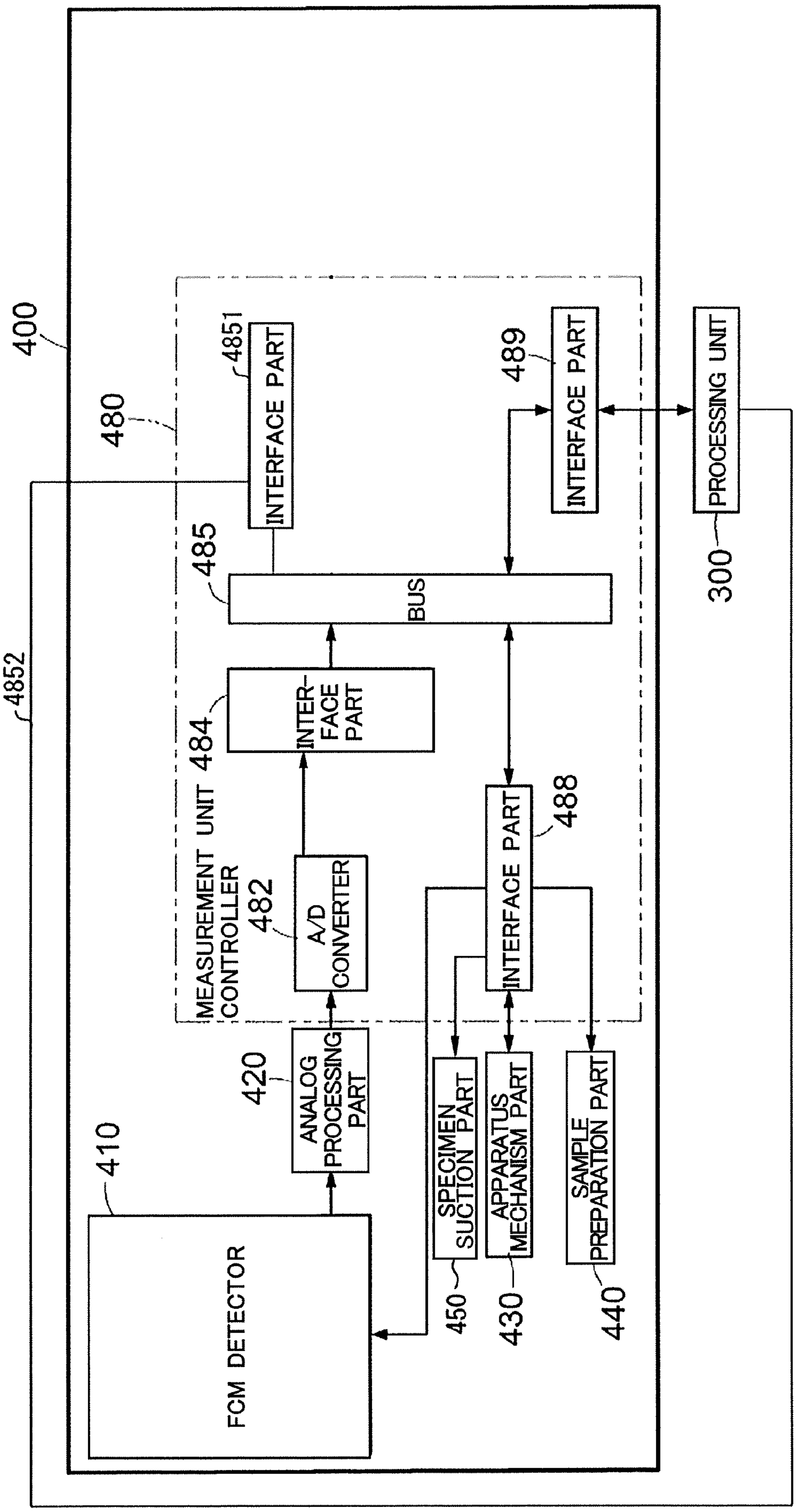
400
480
MEASUREMENT UNIT CONTROLLER
4851
INTERFACE PART
489
INTERFACE PART
PROCESSING UNIT
300
485
BUS
4852
484
INTER-FACE PART
482
A/D CONVERTER
488
INTERFACE PART
420
ANALOG PROCESSING PART
410
FCM DETECTOR
SPECIMEN SUCTION PART
450
APPARATUS MECHANISM PART
430
SAMPLE PREPARATION PART
440

FIG. 26

FIG. 27

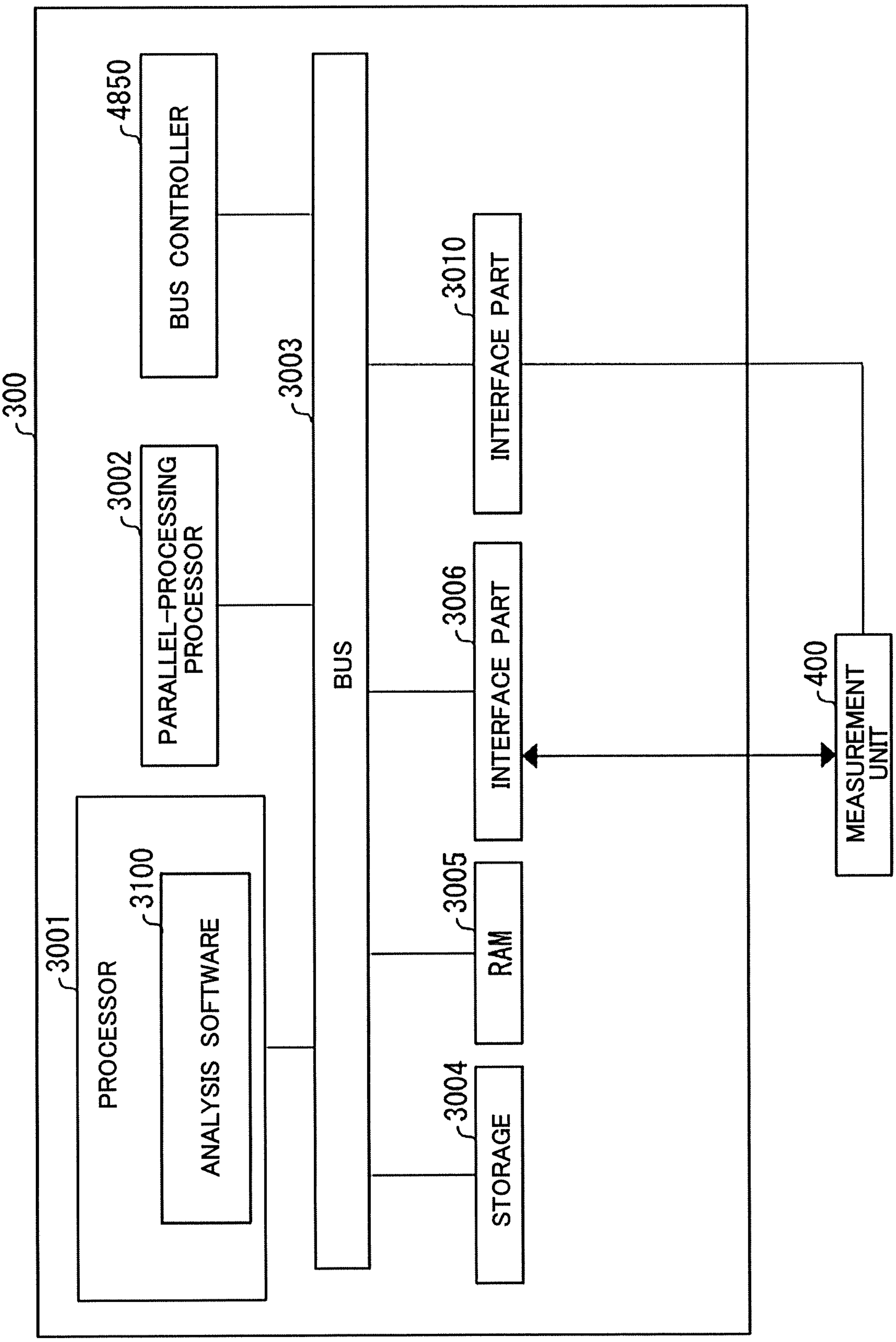
300
4850
BUS CONTROLLER
3002
PARALLEL-PROCESSING PROCESSOR
3003
BUS
3010
INTERFACE PART
3006
INTERFACE PART
400
MEASUREMENT UNIT
3001
PROCESSOR
3100
ANALYSIS SOFTWARE
3005
RAM
3004
STORAGE

FIG. 28

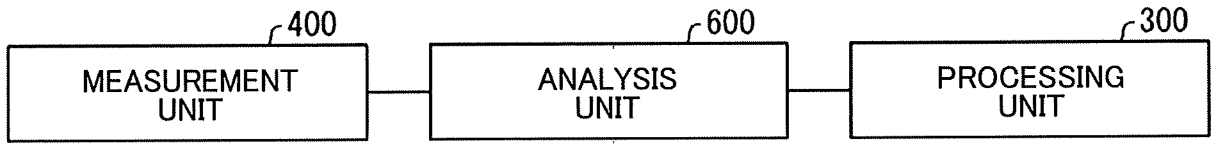
400
MEASUREMENT UNIT
600
ANALYSIS UNIT
300
PROCESSING UNIT

FIG. 29

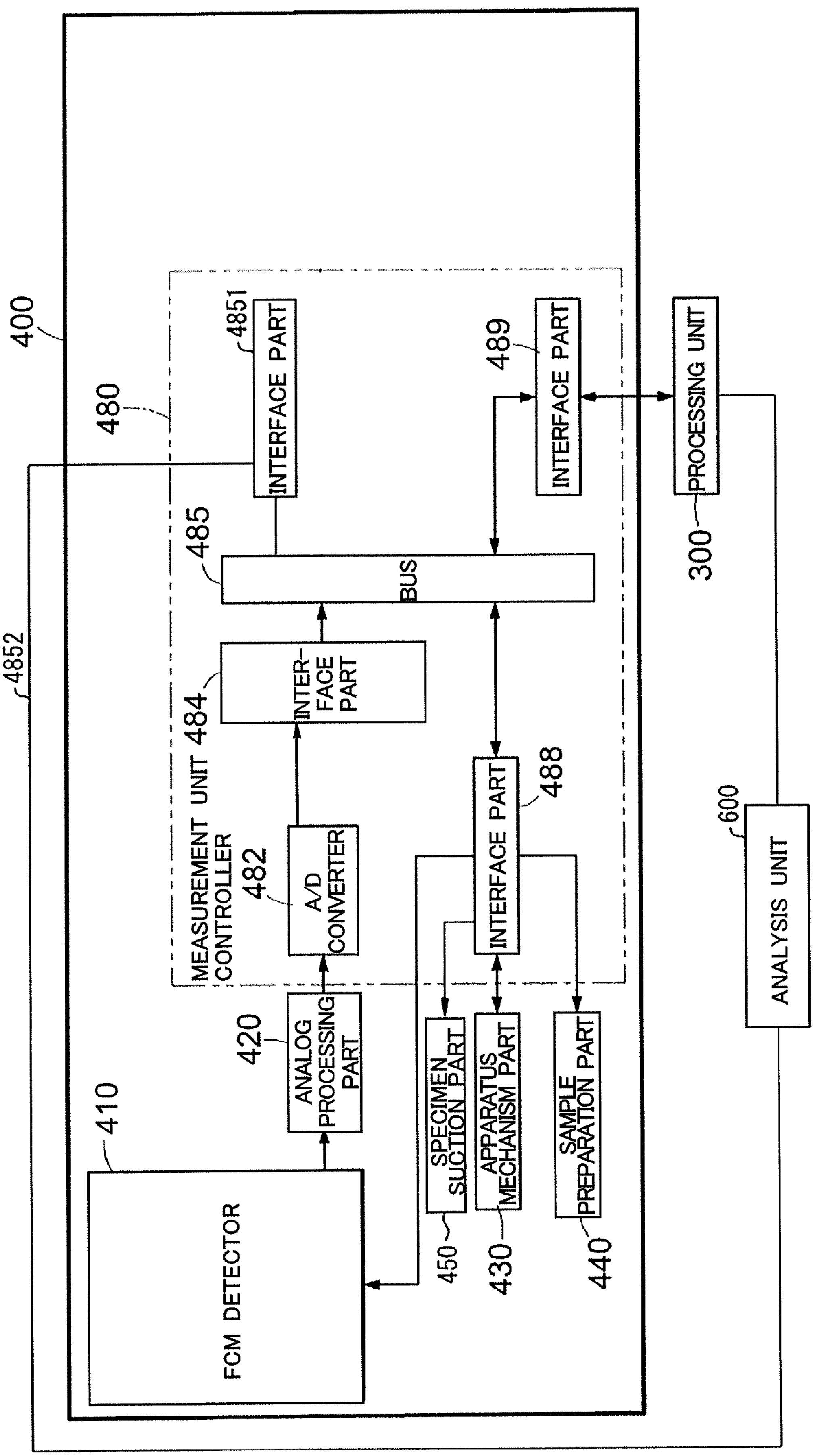
400
480
4851
INTERFACE PART
489
INTERFACE PART
PROCESSING UNIT
300
485
BUS
4852
484
INTER-FACE PART
MEASUREMENT UNIT CONTROLLER
482
A/D CONVERTER
488
INTERFACE PART
600
ANALYSIS UNIT
420
ANALOG PROCESSING PART
410
FCM DETECTOR
SPECIMEN SUCTION PART
APPARATUS MECHANISM PART
SAMPLE PREPARATION PART
450
430
440

FIG. 30

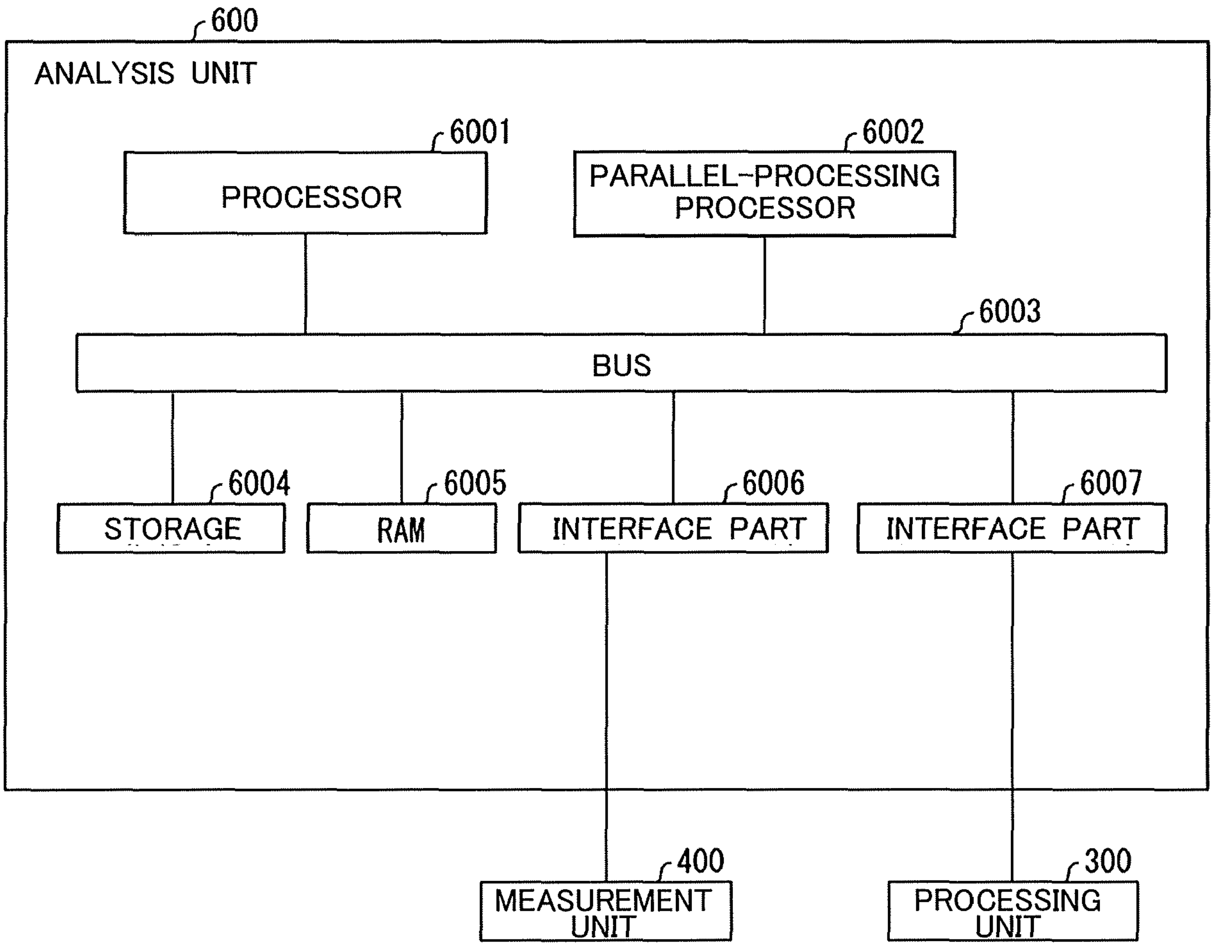
600
ANALYSIS UNIT
6001
PROCESSOR
6002
PARALLEL-PROCESSING PROCESSOR
6003
BUS
6004
STORAGE
6005
RAM
6006
INTERFACE PART
6007
INTERFACE PART
400
MEASUREMENT UNIT
300
PROCESSING UNIT

FIG. 31

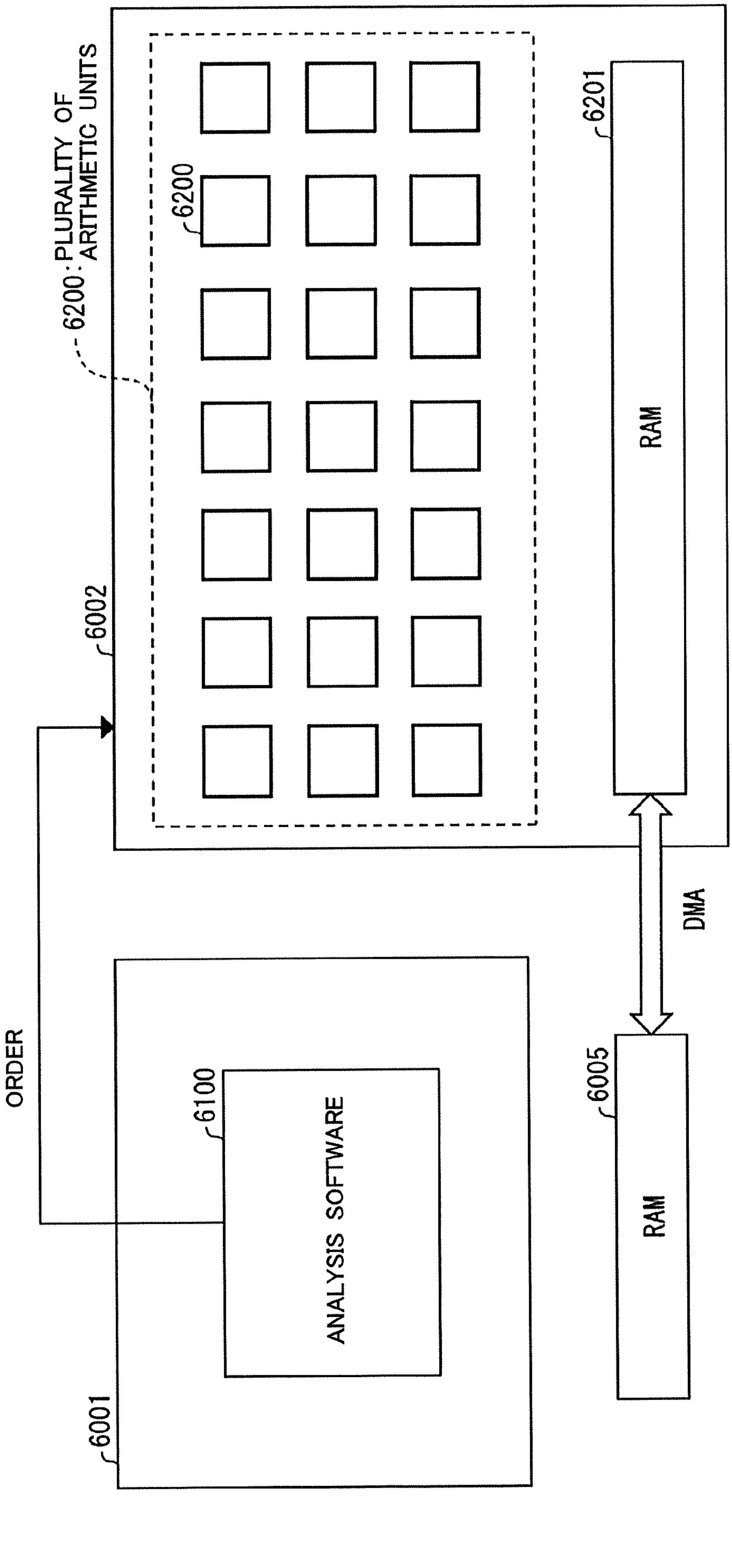
6200: PLURALITY OF ARITHMETIC UNITS
6200
6201
6002
RAM
ORDER
DMA
6100
ANALYSIS SOFTWARE
6005
RAM
6001

FIG. 32

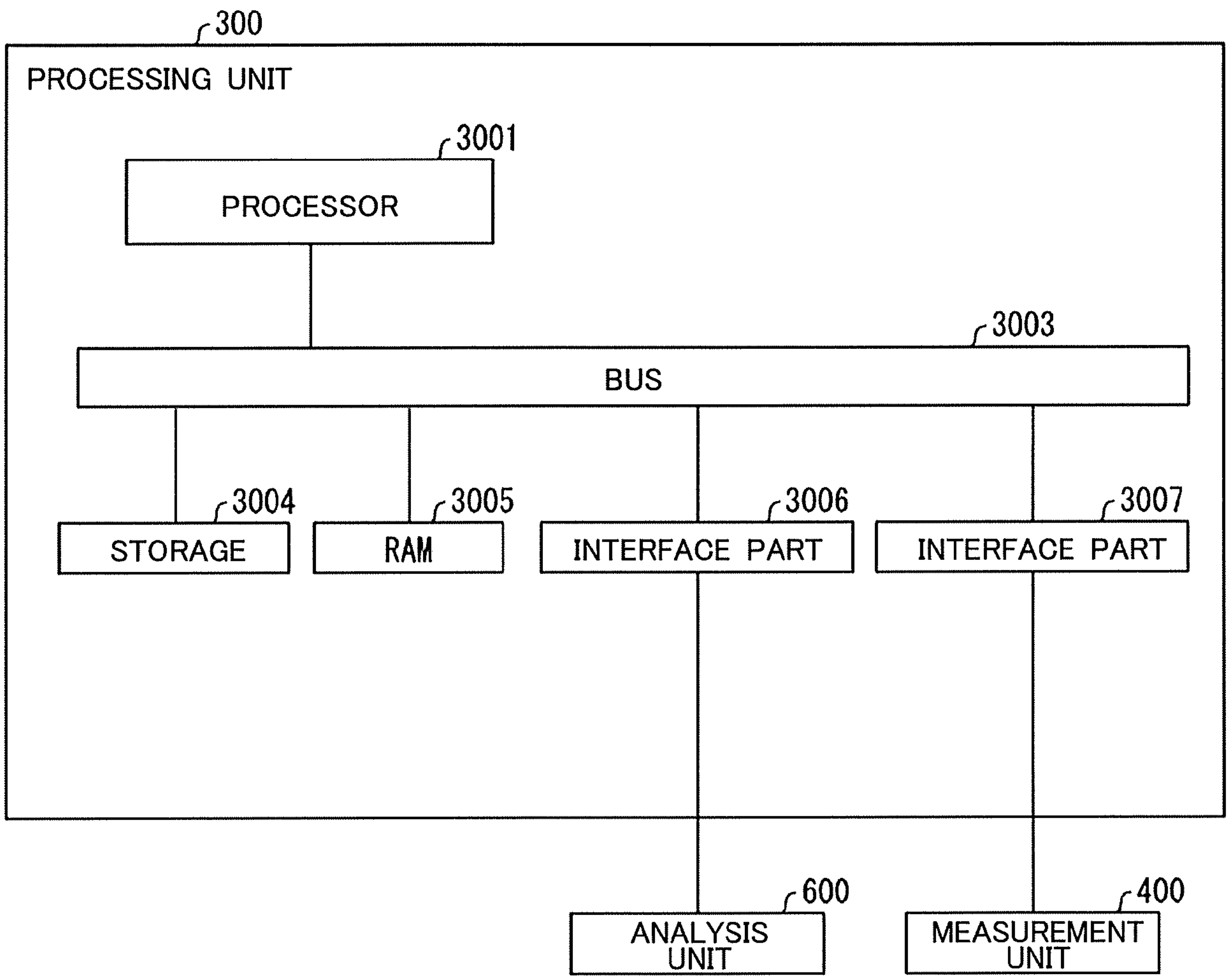
300
PROCESSING UNIT
3001
PROCESSOR
3003
BUS
3004
STORAGE
3005
RAM
3006
INTERFACE PART
3007
INTERFACE PART
600
ANALYSIS UNIT
400
MEASUREMENT UNIT

FIG. 33

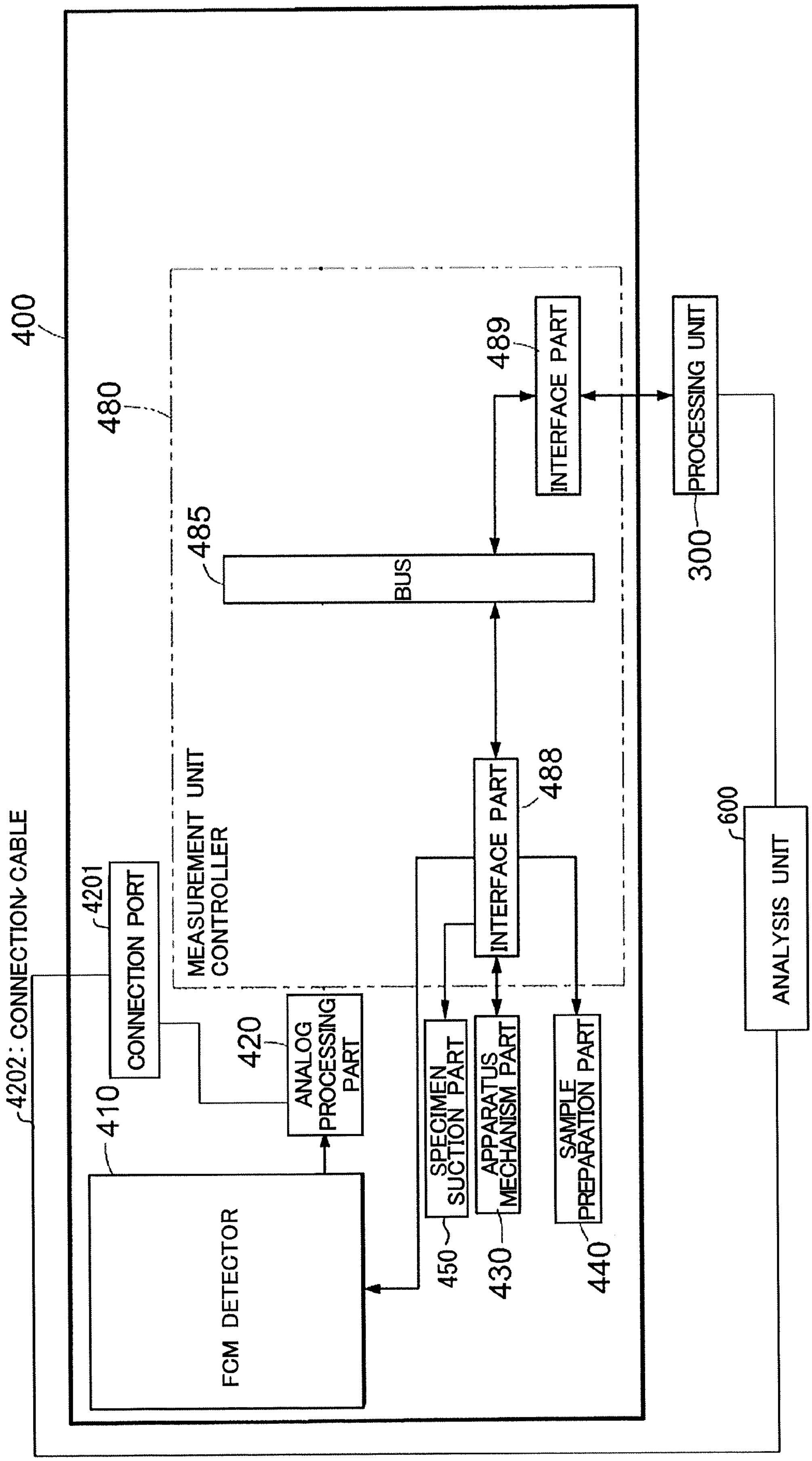

4202: CONNECTION CABLE
400
4201
CONNECTION PORT
410
FCM DETECTOR
420
ANALOG PROCESSING PART
480
MEASUREMENT UNIT CONTROLLER
485
BUS
488
INTERFACE PART
489
INTERFACE PART
450
SPECIMEN SUCTION PART
430
APPARATUS MECHANISM PART
440
SAMPLE PREPARATION PART
300
PROCESSING UNIT
600
ANALYSIS UNIT

FIG. 34

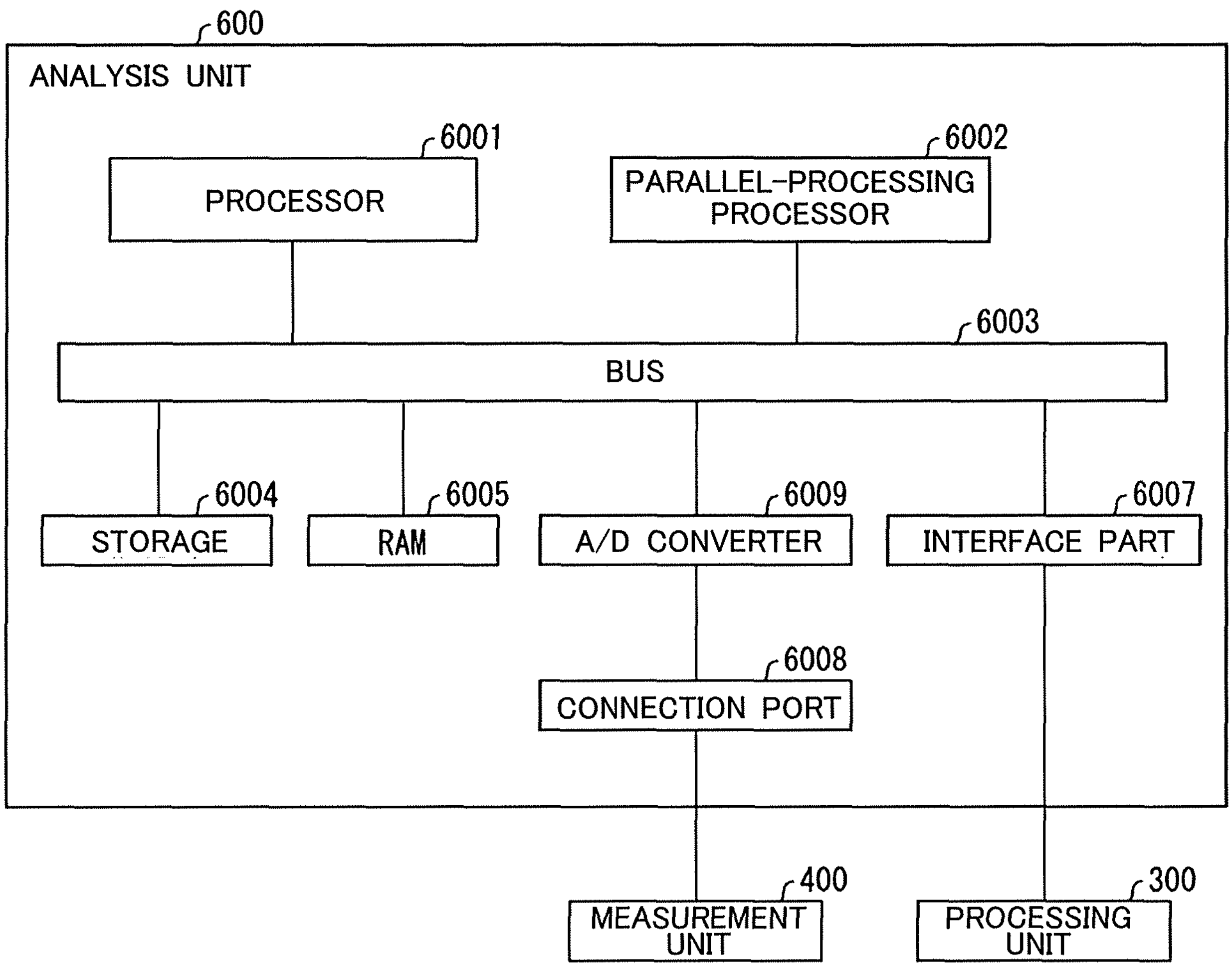
600
ANALYSIS UNIT
6001
PROCESSOR
6002
PARALLEL-PROCESSING PROCESSOR
6003
BUS
6004
STORAGE
6005
RAM
6009
A/D CONVERTER
6007
INTERFACE PART
6008
CONNECTION PORT
400
MEASUREMENT UNIT
300
PROCESSING UNIT

FIG. 35

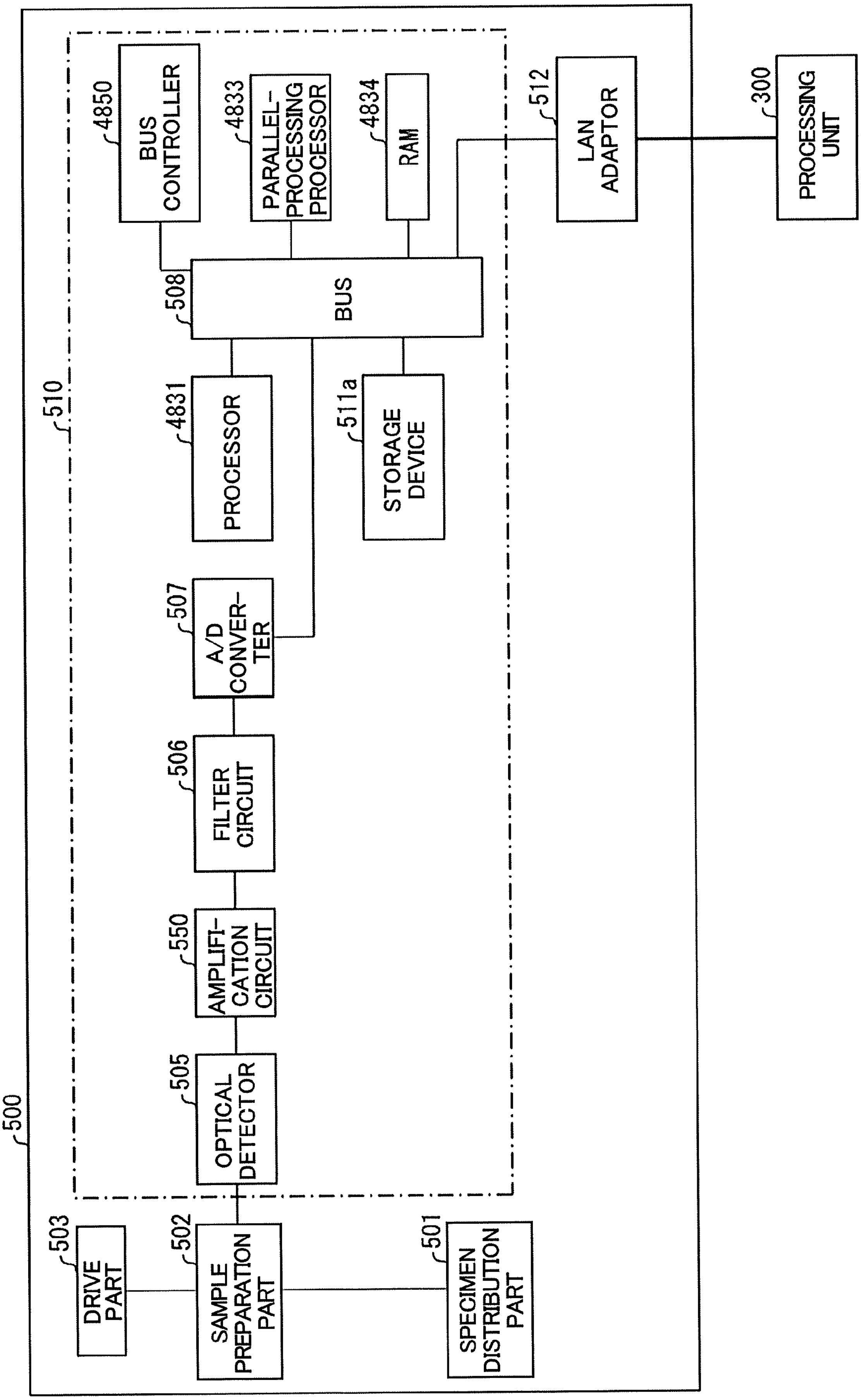
500
510
503
DRIVE PART
502
SAMPLE PREPARATION PART
501
SPECIMEN DISTRIBUTION PART
505
OPTICAL DETECTOR
550
AMPLIFI-CATION CIRCUIT
506
FILTER CIRCUIT
507
A/D CONVER-TER
4831
PROCESSOR
511a
STORAGE DEVICE
508
BUS
4850
BUS CONTROLLER
4833
PARALLEL-PROCESSING PROCESSOR
4834
RAM
512
LAN ADAPTOR
300
PROCESSING UNIT

FIG. 36

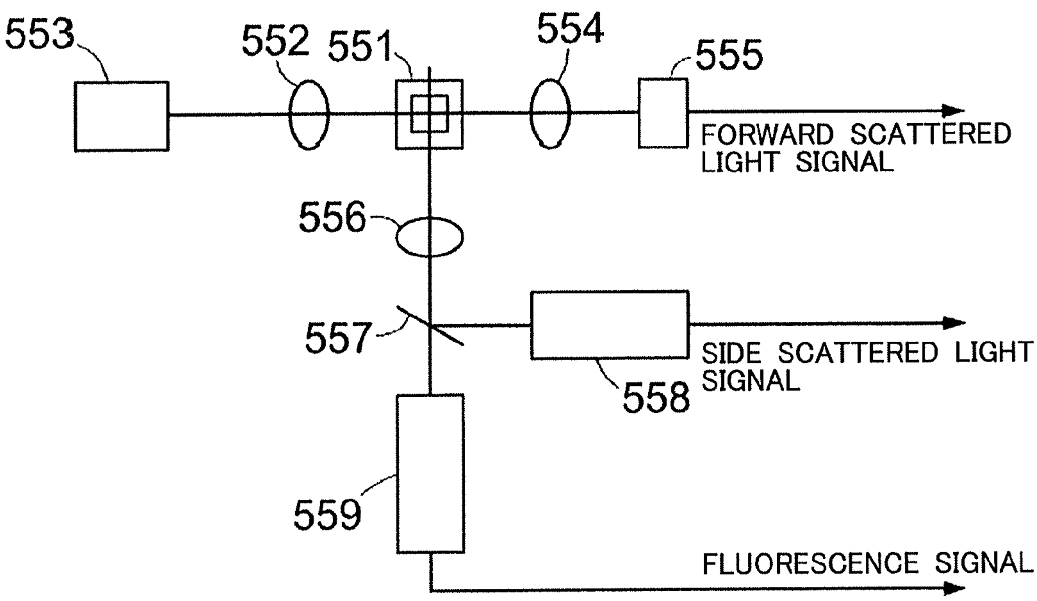
553
552
551
554
555
FORWARD SCATTERED LIGHT SIGNAL
556
557
SIDE SCATTERED LIGHT SIGNAL
558
559
FLUORESCENCE SIGNAL

FIG. 37

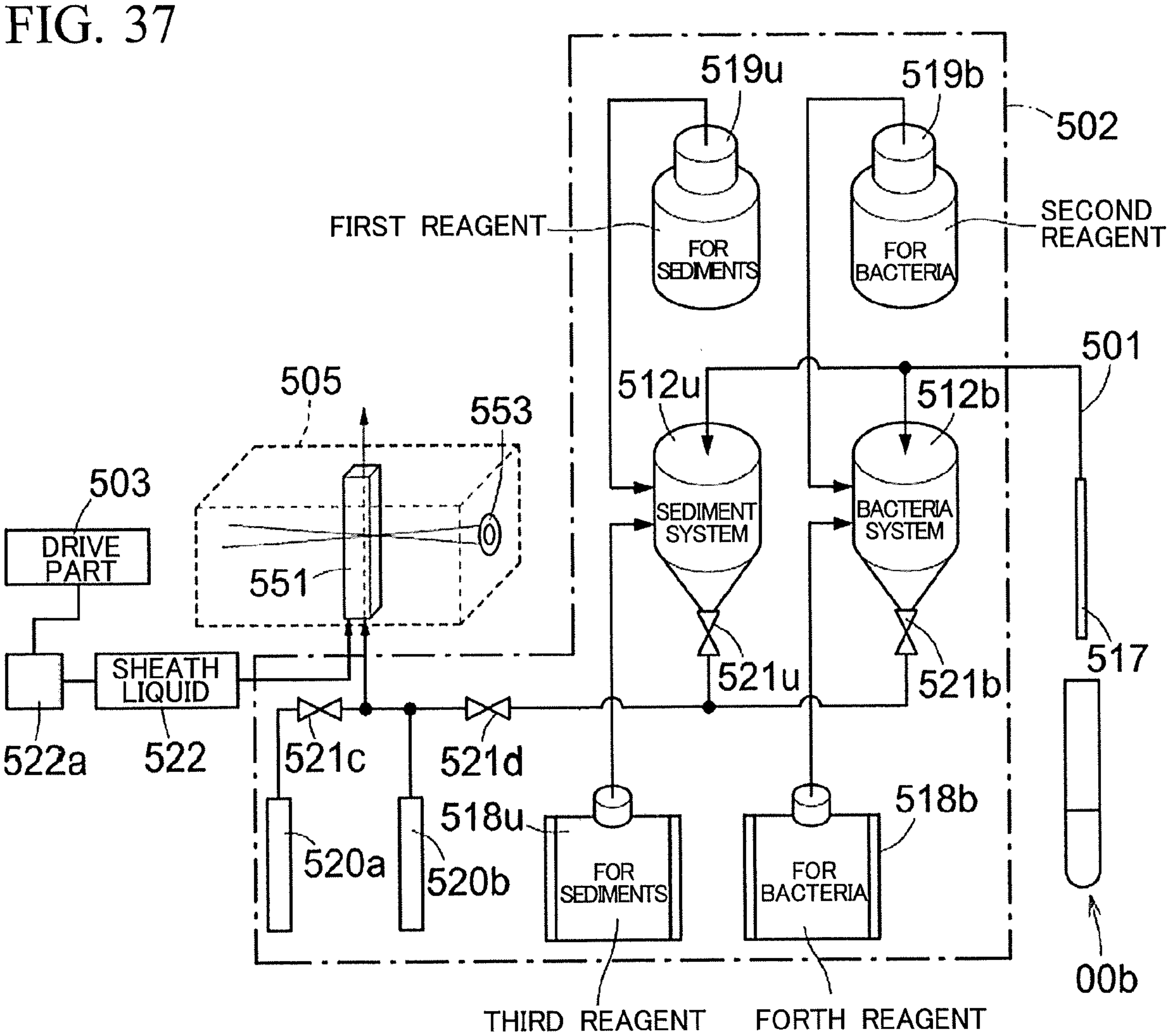
519u
519b
502
FIRST REAGENT
FOR SEDIMENTS
FOR BACTERIA
SECOND REAGENT
501
505
553
512u
512b
503
DRIVE PART
SEDIMENT SYSTEM
BACTERIA SYSTEM
551
SHEATH LIQUID
521u
521b
517
522a
522
521c
521d
518u
518b
520a
520b
FOR SEDIMENTS
FOR BACTERIA
00b
THIRD REAGENT
FORTH REAGENT

FIG. 38

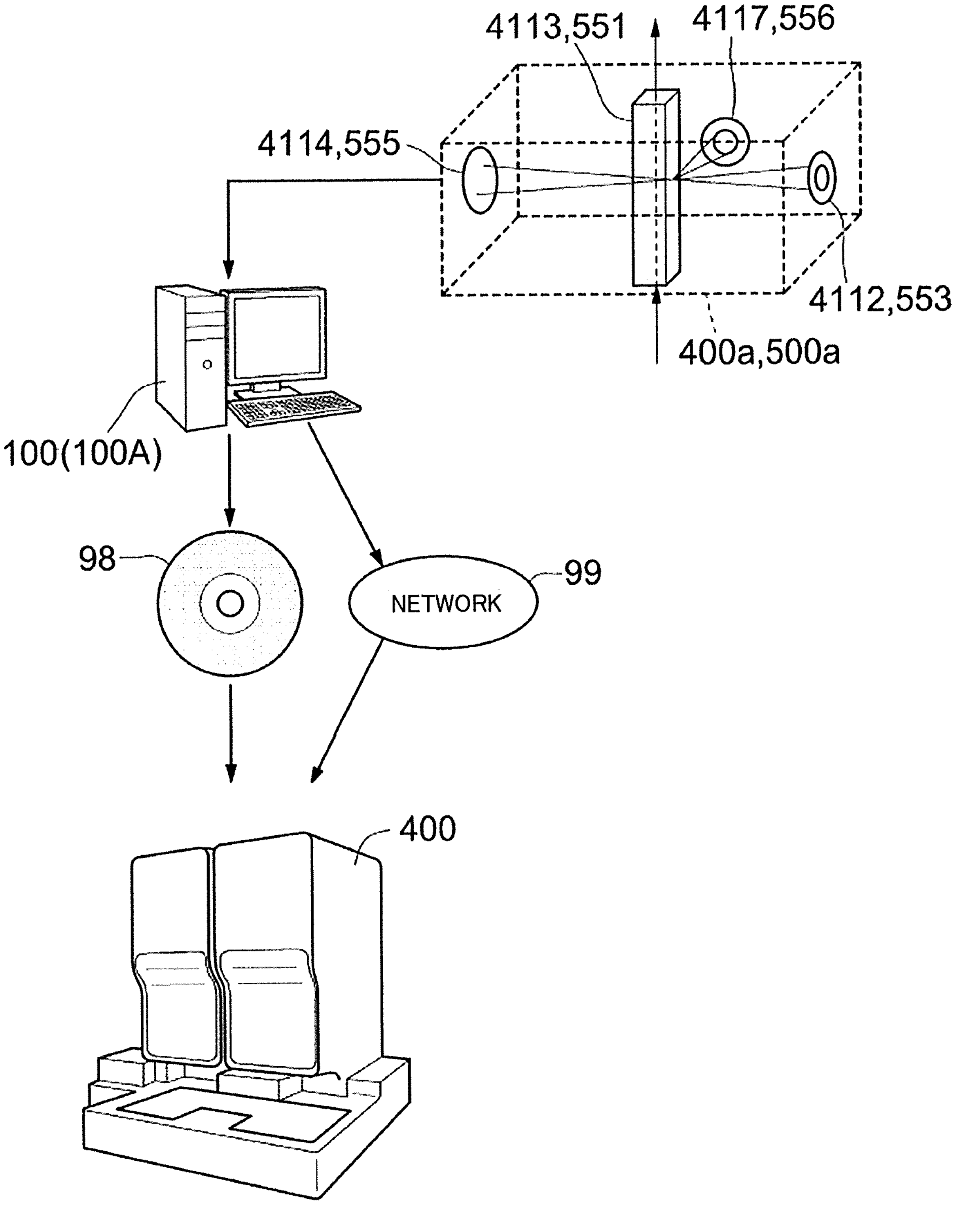
4113,551
4117,556
4114,555
4112,553
400a,500a
100(100A)
98
NETWORK
99
400

FIG. 39

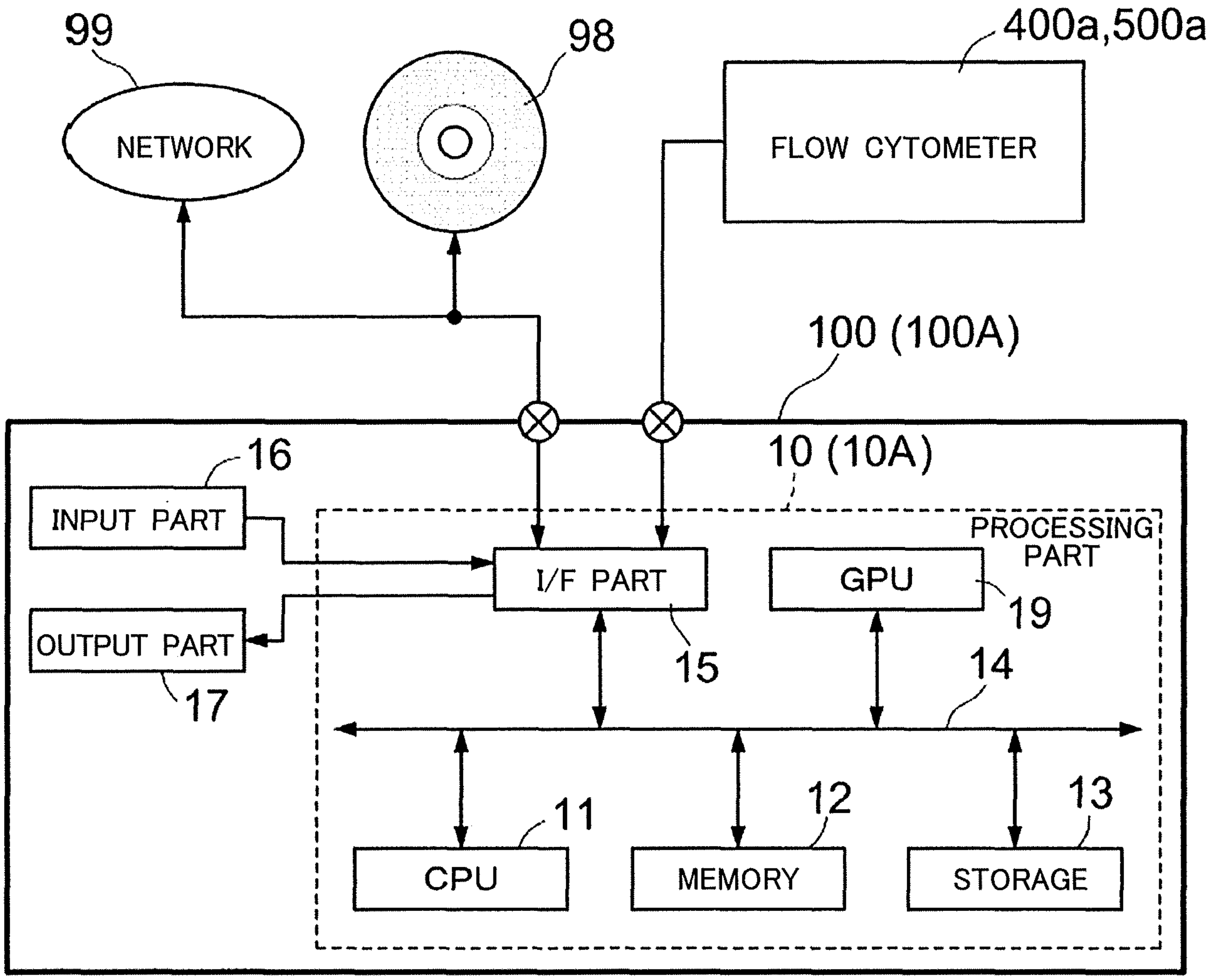
99
NETWORK
98
400a,500a
FLOW CYTOMETER
100 (100A)
16
INPUT PART
10 (10A)
PROCESSING PART
I/F PART
GPU
19
OUTPUT PART
17
15
14
11
12
13
CPU
MEMORY
STORAGE

FIG. 40

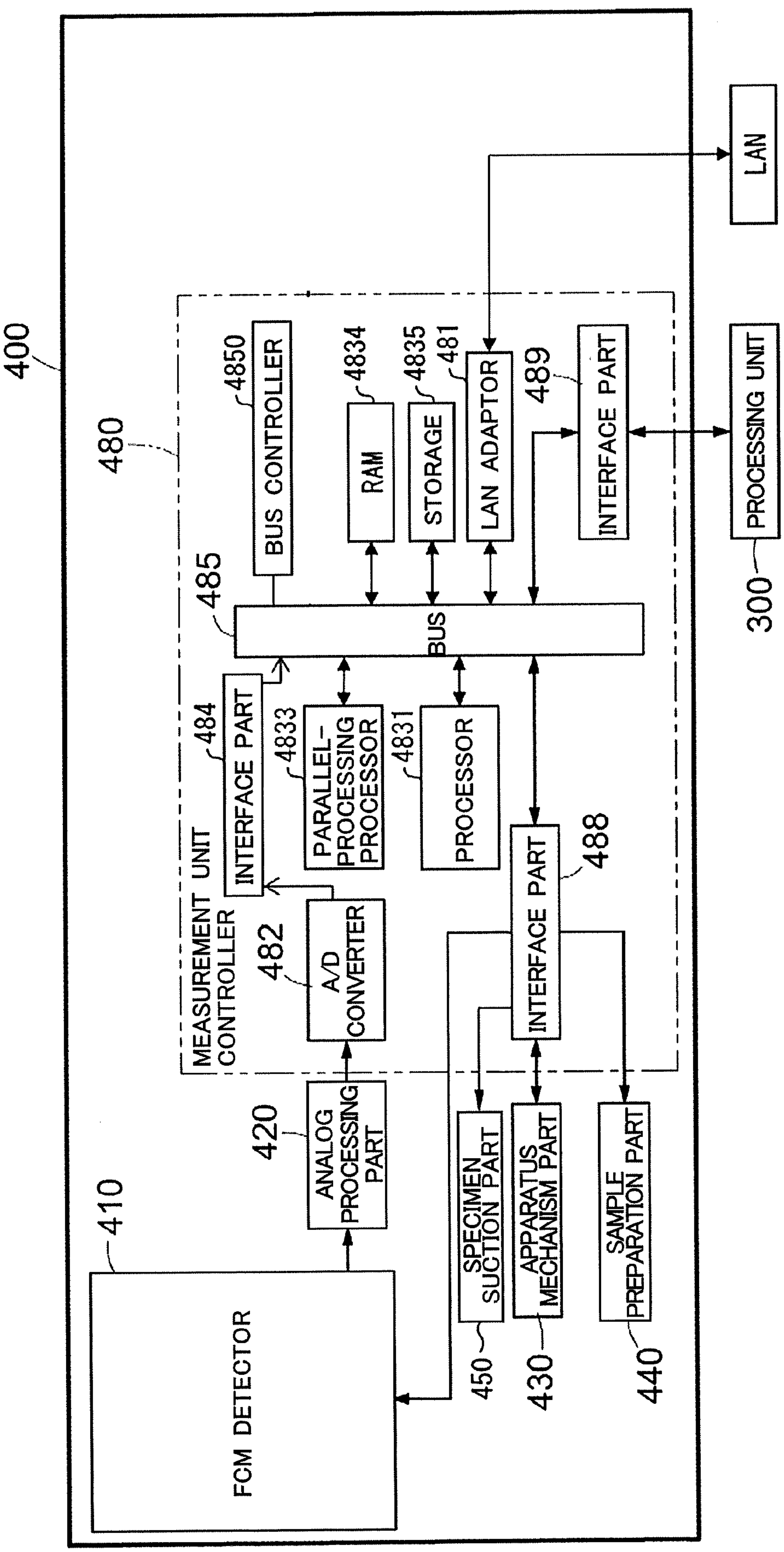
400
480
MEASUREMENT UNIT CONTROLLER
BUS CONTROLLER
4850
RAM
4834
STORAGE
4835
LAN ADAPTOR
481
INTERFACE PART
489
LAN
PROCESSING UNIT
300
485
BUS
INTERFACE PART
484
PARALLEL-PROCESSING PROCESSOR
4833
PROCESSOR
4831
INTERFACE PART
488
A/D CONVERTER
482
ANALOG PROCESSING PART
420
410
FCM DETECTOR
SPECIMEN SUCTION PART
450
APPARATUS MECHANISM PART
430
SAMPLE PREPARATION PART
440

FIG. 41

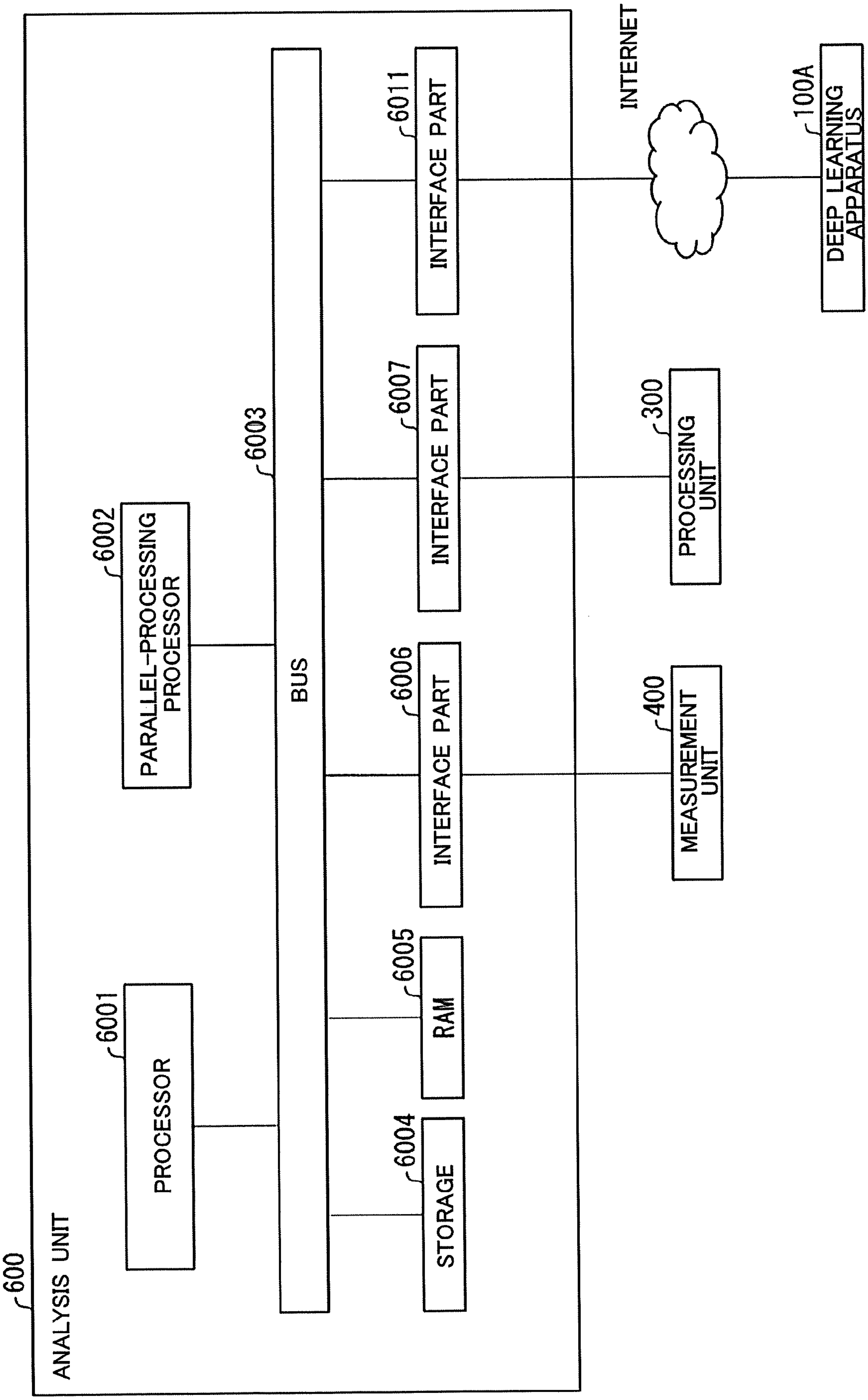
600
ANALYSIS UNIT
6001
PROCESSOR
6002
PARALLEL-PROCESSING PROCESSOR
6003
BUS
6004
STORAGE
6005
RAM
6006
INTERFACE PART
6007
INTERFACE PART
6011
INTERFACE PART
400
MEASUREMENT UNIT
300
PROCESSING UNIT
INTERNET
100A
DEEP LEARNING APPARATUS

FIG. 42

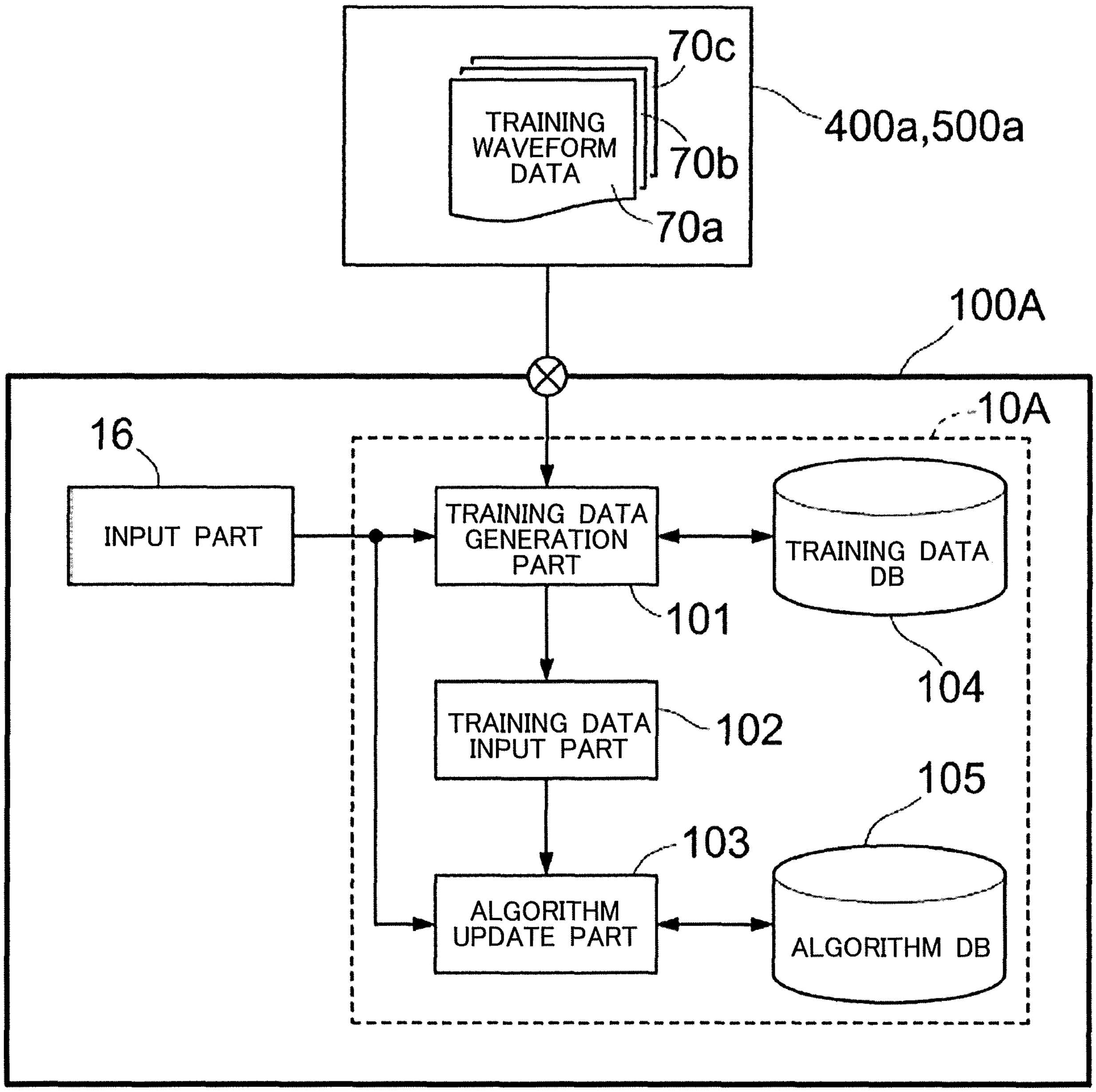
70c
TRAINING
WAVEFORM
DATA
70b
70a
400a,500a
100A
10A
16
INPUT PART
TRAINING DATA
GENERATION
PART
TRAINING DATA
DB
101
104
TRAINING DATA
INPUT PART
102
105
103
ALGORITHM
UPDATE PART
ALGORITHM DB

FIG. 43

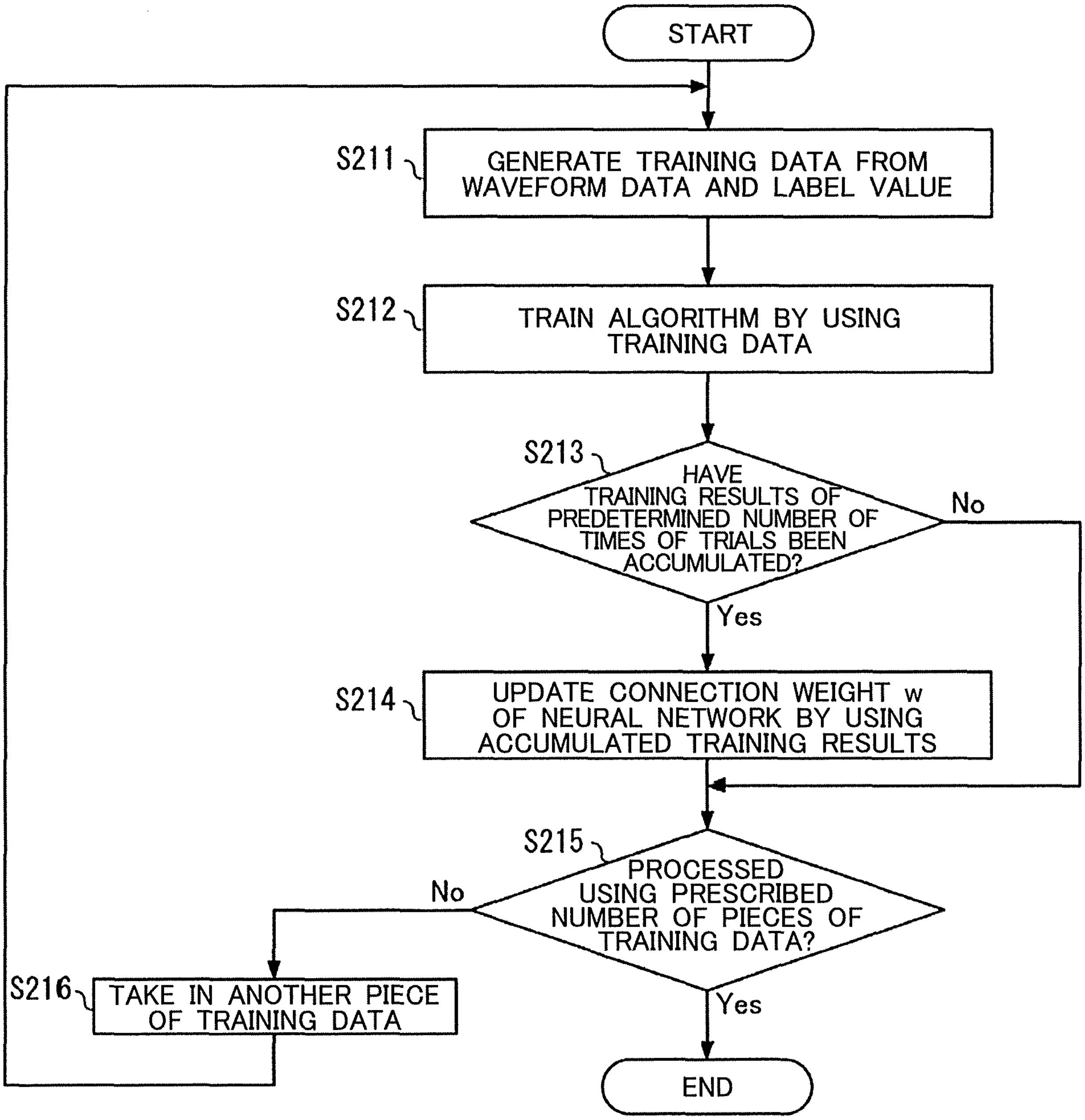
START
S211
GENERATE TRAINING DATA FROM WAVEFORM DATA AND LABEL VALUE
S212
TRAIN ALGORITHM BY USING TRAINING DATA
S213
HAVE TRAINING RESULTS OF PREDETERMINED NUMBER OF TIMES OF TRIALS BEEN ACCUMULATED?
No
Yes
S214
UPDATE CONNECTION WEIGHT w OF NEURAL NETWORK BY USING ACCUMULATED TRAINING RESULTS
S215
PROCESSED USING PRESCRIBED NUMBER OF PIECES OF TRAINING DATA?
No
Yes
S216
TAKE IN ANOTHER PIECE OF TRAINING DATA
END

FIG. 44

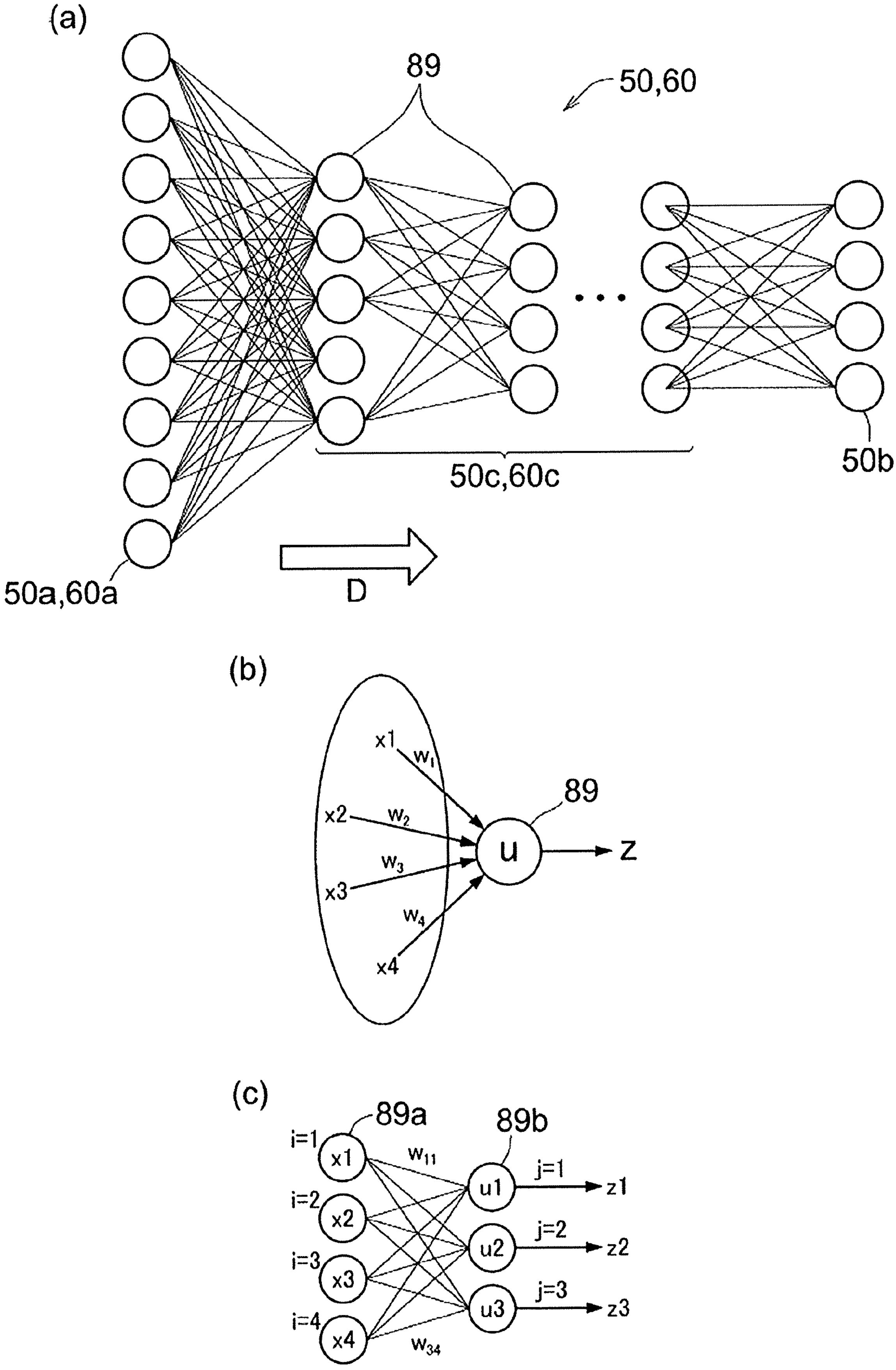
(a)
89
50,60
50c,60c
50b
50a,60a
D
(b)
x1
w1
x2
w2
w3
x3
w4
x4
u
89
z
(c)
89a
89b
i=1
i=2
i=3
i=4
x1
x2
x3
x4
w11
w34
u1
u2
u3
j=1
j=2
j=3
z1
z2
z3

FIG. 45

| REFERENCE METHOD (*) \ DEEP LEARNING | NEUT | LYMPH | MONO | EO | BASO | IG | CONT | GST |
|---|---|---|---|---|---|---|---|---|
| NEUT | 3450 | 0 | 1 | 0 | 0 | 2 | 0 | 0 |
| LYMPH | 1 | 1852 | 1 | 0 | 0 | 0 | 1 | 0 |
| MONO | 1 | 1 | 195 | 0 | 0 | 1 | 0 | 0 |
| EO | 6 | 0 | 0 | 41 | 0 | 0 | 0 | 0 |
| BASO | 0 | 27 | 0 | 0 | 4 | 0 | 0 | 0 |
| IG | 1 | 0 | 0 | 0 | 0 | 13 | 0 | 0 |
| CONT | 0 | 4 | 2 | 0 | 0 | 0 | 649 | 0 |
| GST | 7 | 1 | 0 | 0 | 19 | 0 | 0 | 0 |

| TARGET CELL | NUMBER OF CELLS |
|---|---|
| NEUT | 3453 |
| LYMPH | 1855 |
| MONO | 198 |
| EO | 47 |
| BASO | 31 |
| IG | 14 |
| CONTROL BLOOD CELL | 655 |

FIG. 46

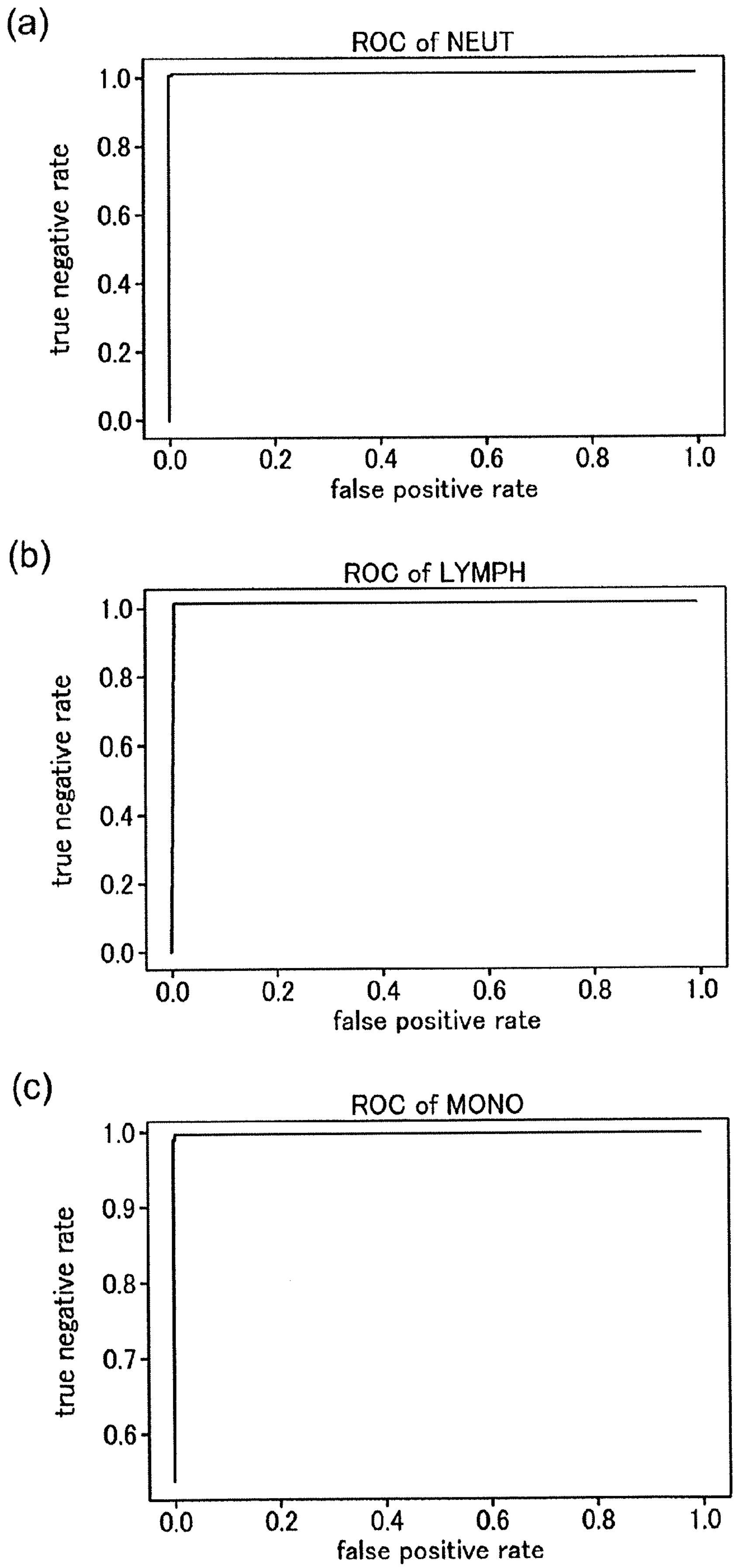
(a)
ROC of NEUT
true negative rate
1.0
0.8
0.6
0.4
0.2
0.0
0.0
0.2
0.4
0.6
0.8
1.0
false positive rate
(b)
ROC of LYMPH
true negative rate
1.0
0.8
0.6
0.4
0.2
0.0
0.0
0.2
0.4
0.6
0.8
1.0
false positive rate
(c)
ROC of MONO
true negative rate
1.0
0.9
0.8
0.7
0.6
0.0
0.2
0.4
0.6
0.8
1.0
false positive rate

FIG. 47

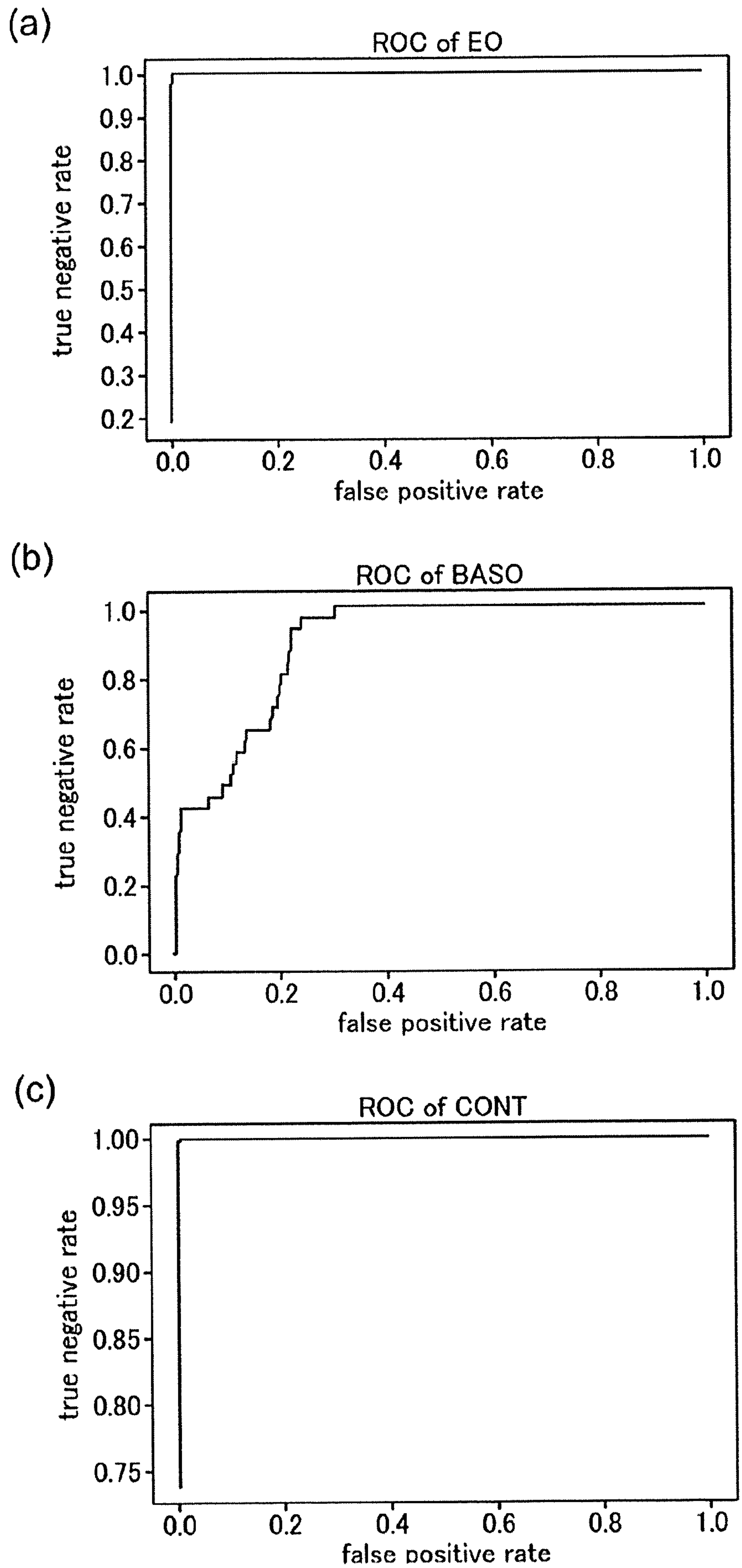
(a)
ROC of EO
true negative rate
1.0
0.9
0.8
0.7
0.6
0.5
0.4
0.3
0.2
0.0
0.2
0.4
0.6
0.8
1.0
false positive rate
(b)
ROC of BASO
true negative rate
1.0
0.8
0.6
0.4
0.2
0.0
0.0
0.2
0.4
0.6
0.8
1.0
false positive rate
(c)
ROC of CONT
true negative rate
1.00
0.95
0.90
0.85
0.80
0.75
0.0
0.2
0.4
0.6
0.8
1.0
false positive rate

FIG. 48

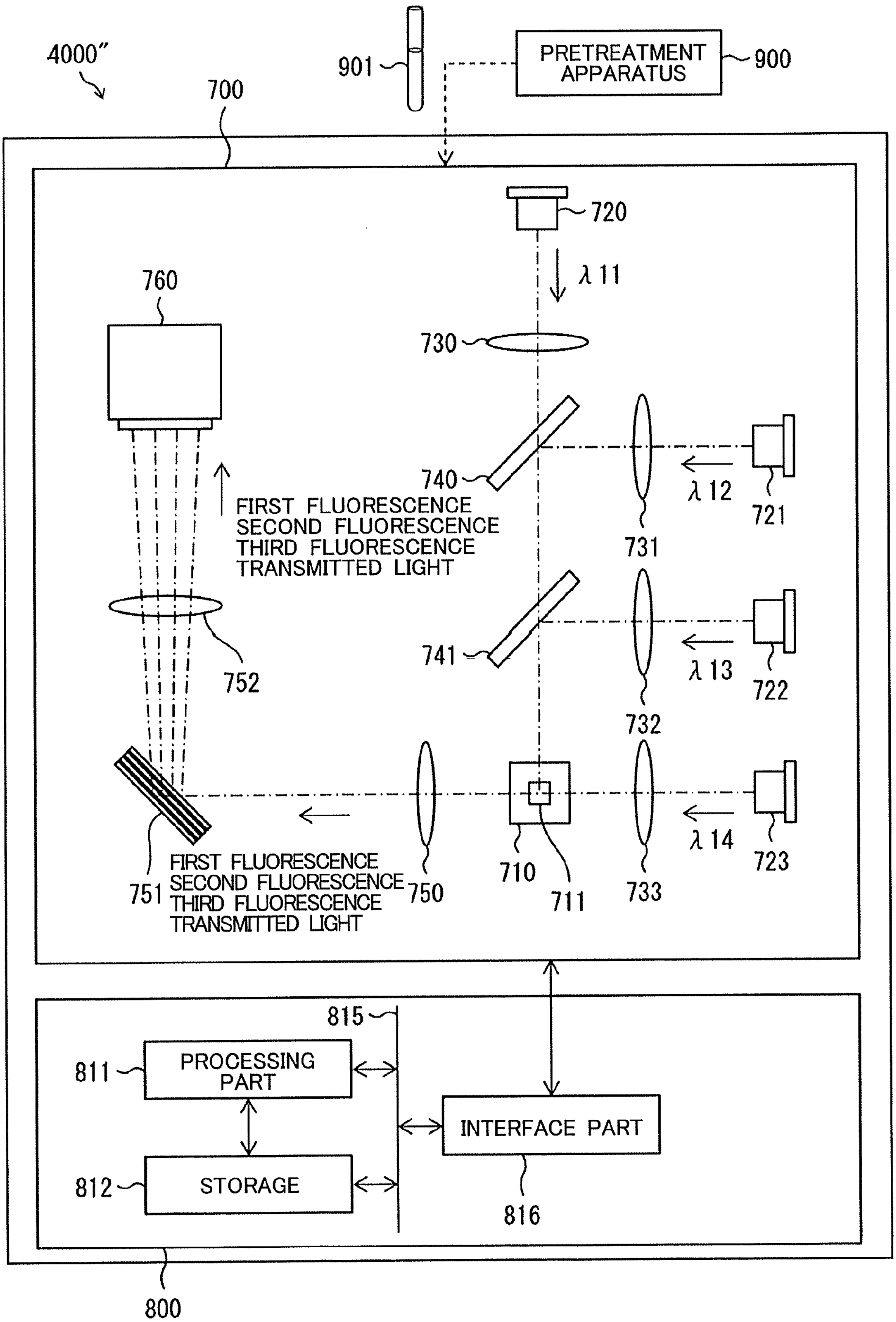
4000"
700
901
PRETREATMENT APPARATUS
900
720
λ11
760
730
740
λ12
721
731
FIRST FLUORESCENCE
SECOND FLUORESCENCE
THIRD FLUORESCENCE
TRANSMITTED LIGHT
741
λ13
722
732
752
751
FIRST FLUORESCENCE
SECOND FLUORESCENCE
THIRD FLUORESCENCE
TRANSMITTED LIGHT
750
710
711
733
λ14
723
815
PROCESSING PART
811
STORAGE
812
INTERFACE PART
816
800

FIG. 49

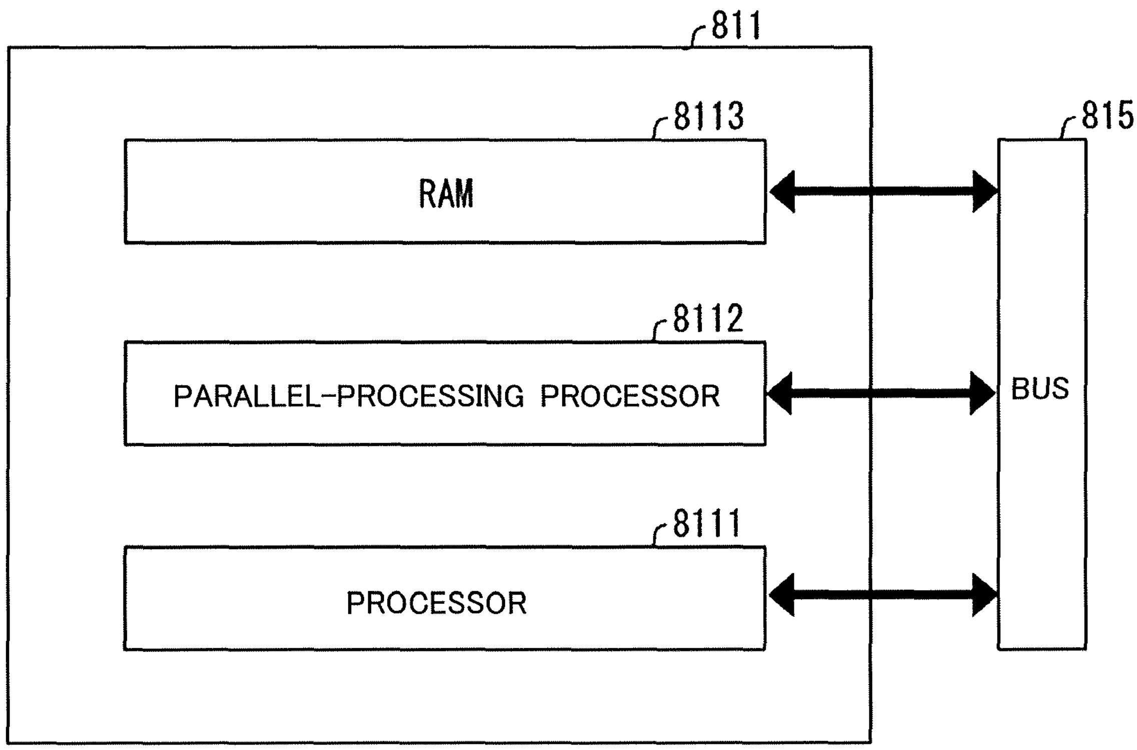
811
8113
RAM
8112
PARALLEL-PROCESSING PROCESSOR
8111
PROCESSOR
815
BUS

FIG. 50

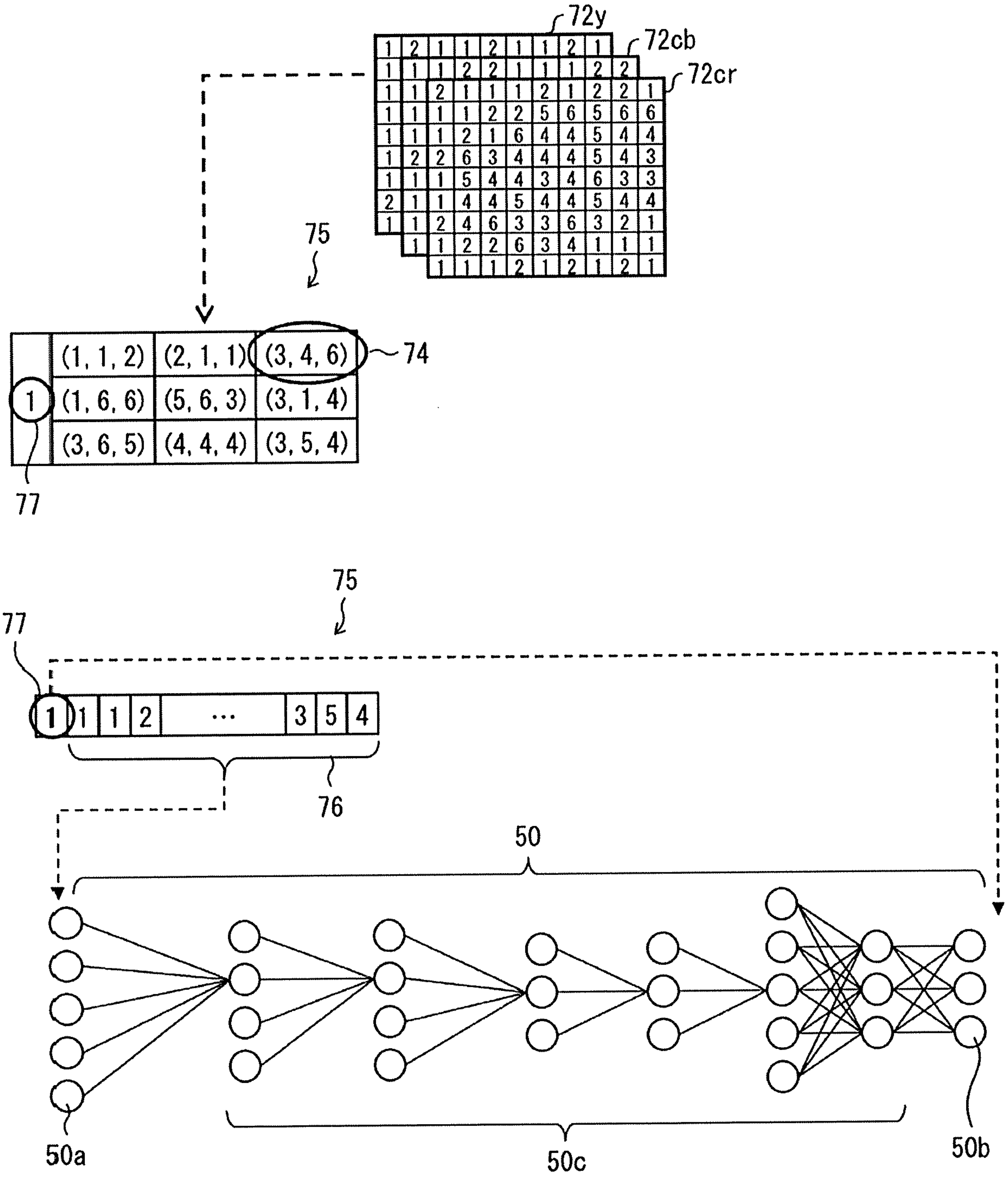
72y
72cb
72cr
75
(1, 1, 2)
(2, 1, 1)
(3, 4, 6)
74
1
(1, 6, 6)
(5, 6, 3)
(3, 1, 4)
(3, 6, 5)
(4, 4, 4)
(3, 5, 4)
77
75
77
1
1 1 2 ··· 3 5 4
76
50
50a
50c
50b

# FIG. 51

| CELL TYPE | LABEL VALUE |
|---|---|
| NOT APPLICABLE | 0 |
| SEGMENTED NEUTROPHIL | 1 |
| BAND NEUTROPHIL | 2 |
| METAMYELOCYTE | 3 |
| MYELOCYTE | 4 |
| PROMYELOCYTE | 5 |
| BLAST | 6 |
| LYMPHOCYTE | 7 |
| PLASMA CELL | 8 |
| ATYPICAL LYMPHOCYTE | 9 |
| MONOCYTE | 10 |
| EOSINOPHIL | 11 |
| BASOPHIL | 12 |
| ERYTHROBLAST | 13 |
| MEGA-THROMBOCYTE | 14 |
| PLATELET AGGREGATE | 15 |
| MEGAKARYOCYTE | 16 |
| smudge | 17 |
| Artifact | 18 |

| CELL TYPE | FEATURE | | LABEL VALUE |
|---|---|---|---|
| BLAST | MORPHOLOGICAL DYSKARYOSIS | PRESENT | 21 |
| | | ABSENT | 22 |
| | VACUOLATION | PRESENT | 23 |
| | | ABSENT | 24 |
| | GRANULAR | PRESENT | 25 |
| | | ABSENT | 26 |
| BAND NEUTROPHIL | GIANT | PRESENT | 27 |
| | | ABSENT | 28 |
| | NUCLEUS MORPHOLOGY ABNORMALITY (INCLUDING SPHERICAL/ELLIPTICAL NUCLEUS) | PRESENT | 29 |
| | | ABSENT | 30 |
| | GRANULE DISTRIBUTION ABNORMALITY (INCLUDING "AGRANULAR") | PRESENT | 31 |
| | | ABSENT | 32 |
| SEGMENTED NEUTROPHIL | GIANT | PRESENT | 33 |
| | | ABSENT | 34 |
| | NUCLEUS MORPHOLOGY ABNORMALITY (INCLUDING RING-SHAPED NUCLEUS) | PRESENT | 35 |
| | | ABSENT | 36 |
| | PSEUDO-PELGER (HYPOSEGMENTED NUCLEUS) | PRESENT | 37 |
| | | ABSENT | 38 |
| | HYPERSEGMENTATION | PRESENT | 39 |
| | | ABSENT | 40 |
| | GRANULE DISTRIBUTION ABNORMALITY (INCLUDING "AGRANULAR") | PRESENT | 41 |
| | | ABSENT | 42 |
| ERYTHRO-BLAST | GIANT | PRESENT | 43 |
| | | ABSENT | 44 |
| MEGA-KARYOCYTE | BARE NUCLEUS | PRESENT | 45 |
| | | ABSENT | 46 |

FIG. 52

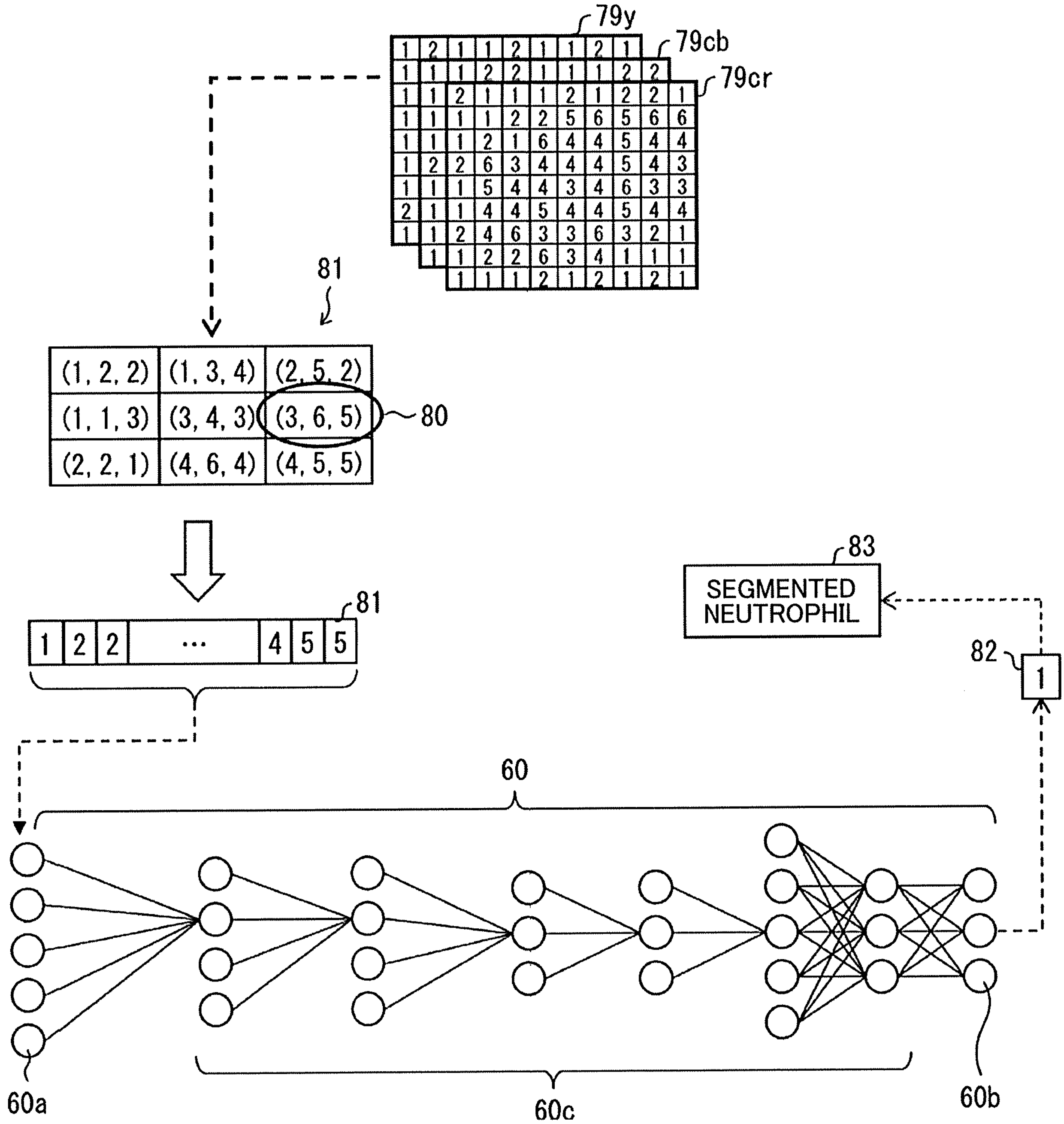
79y
79cb
79cr
81
(1, 2, 2)
(1, 3, 4)
(2, 5, 2)
(1, 1, 3)
(3, 4, 3)
(3, 6, 5)
80
(2, 2, 1)
(4, 6, 4)
(4, 5, 5)
81
1 2 2 ... 4 5 5
83
SEGMENTED NEUTROPHIL
82
1
60
60a
60c
60b

FIG. 53

| CELL TYPE | NUMBER OF CELLS |
|---|---|
| NEUTROPHIL (NEUT) | 3500 |
| LYMPHOCYTE (LYMPH) | 1800 |
| MONOCYTE (MONO) | 200 |
| EOSINOPHIL (EO) | 50 |
| BASOPHIL (BASO) | 30 |
| IMMATURE GRANULOCYTE (IG) | 15 |
| ABNORMAL CELL | 10 ! |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012519848 A
- US 5891733 A
- EP 1136563 A
- US 7309581 B